# EUROPEAN PATENT APPLICATION

(11) **EP 1 213 352 A1**
(43) Date of publication of application: **12.06.2002**
(21) Application number: 00403440.1
(22) Date of filing: 07.12.2000
(51) Int. Cl.: C12N 15/12, C07K 14/705, C07K 14/47, C12Q 1/68, C12N 5/10, C07K 16/28, A61K 48/00, A61K 38/17, G01N 33/68

(54) **Nucleic acids of the human abca5, abca6, abca9, and abca10 genes, vectors containing such nucleic acids and uses thereof**

(71) Applicant: Aventis Pharma S.A., 92160 Antony (FR)
(72) Inventor: Denefle, Patrice, 94100 Saint Maur (FR); Rosier-Montus, Marie-Françoise, 92160 Antony (FR); Prades, Catherine, 94320 Thiais (FR); Arnoud-Reguigne, Isabelle, 94430 Chennevieres sur Marne (FR); Duverger, Nicolas, 75005 Paris (FR); Allikmets, Rando, Monroe, NY 10950 (US); Dean, Michael, Frederick, MD 21703 (US)
(74) Representative: Lecca, Patricia

(57) **Abstract**

The present invention relates to nucleic acids corresponding to various exons of ABCA5, ABCA6, ABCA9, and ABCA10 genes as well as cDNAs encoding the novel full length of ABCA5, ABCA6, ABCA9, and ABCA10 proteins. The invention also relates to means for the detection of polymorphisms in general, and of mutations in particular, in the ABCA5, ABCA6, ABCA9, and ABCA10 genes or in the corresponding protein produced by the allelic form of the ABCA5, ABCA6, ABCA9, and ABCA10 genes.

## Description

The present invention relates to novel nucleic acids corresponding to ABCA5, ABCA6, ABCA9, and ABCA10 genes, and cDNAs encoding novel ABCA5, ABCA6, ABCA9, and ABCA10 proteins. The invention also relates to means for the detection of polymorphisms in general, and mutations in particular in the ABCA5, ABCA6, ABCA9, and ABCA10 genes or corresponding proteins produced by the allelic forms of the ABCA5, ABCA6, ABCA9, and ABCA10 genes.

The ABC (ATP-binding cassette transporter) gene superfamily encode active transporter proteins and constitute a family of proteins that are extremely well conserved during evolution, from bacteria to humans (Ames and Lecar, FASEB J., 1992, 6, 2660-2666). The ABC proteins are involved in extra- and intracellular membrane transport of various substrates, for example ions, amino acids, peptides, sugars, vitamins or steroid hormones. Among the 40 characterized humans members, 11 members have been described as associated with human disease, such as *inter alia* ABCA1, ABCA4 (ABCR) and ABCC7 (CFTR) which are thought to be involved in Tangier disease (Bodzioch M et al., *Nat. Genet.*, 1999, 22(4); 347-351; Brooks-Wilson et al., *Nat Genet*,1999, 22(4), 336-345 ; Rust S et al., *Nat. Genet.*, 1999, 22, 352-355; Remaley A T et al., ), the Stargardt disease (Lewis R A et al., *Am. J. Hum. Genet.*, 1999, 64, 422-434), and the Cystic Fibrosis (Riordan JM et al., *Science*, 1989, 245, 1066-1073), respectively. These implications reveal the importance of the functional role of the ABC gene family and the discovery of new family gene members should provide new insights into the physiopathology of human diseases.

The prototype ABC protein binds ATP and uses the energy from ATP hydrolysis to drive the transport of various molecules across cell membranes. The functional protein contains two ATP-binding domains (nucleotide binding fold, NBF) and two transmembrane (TM) domains. The genes are typically organized as full transporters containing two of each domain, or half transporters with only one of each domain. Most full transporters are arranged in a TM-NBF-TM-NBF fashion (Dean et al., *Curr Opin Genet*, 1995, 5, 79-785).

Analysis of amino acids sequence alignments of the ATP-binding domains has allowed the ABC genes to be separated into sub-families (Allikmets et al., *Hum Mol Genet,* 1996, 5, 1649-1655). Currently, according to the recent HUGO classification, seven ABC gene sub-families named ABC (A to G) have been described in the human genome (ABC1, CFTR/MRP, MDR, ABC8, ALD, GCN20, OABP) with all except one (OABP) containing multiple members. For the most part these sub-families contain genes that also display considerable conservation in the transmembrane domain sequences and have similar gene organization. However, ABC proteins transport very various substrates, and some members of different sub-families have been shown to share more similarity in substrate recognition than do proteins within same sub-family. Five of the sub-families are also represented in the yeast genome, indicating that these groups have been and retained early in the evolution of eukaryotes (Decottignies et al., *Nat Genet*, 1997, 137-45; Michaelis et al., 1995, Cold Spring Harbor Laboratory Press). Several ABC transport proteins that have been identified in humans are associated with various diseases. For example, cystic fibrosis is caused by mutations in the CFTR (cystic fibrosis transmembrane conductance regulator) gene (Riordan JM et al., *Science*, 1989, 245, 1066-1073). Moreover, some multiple drug resistance phenotypes in tumor cells have been associated with the gene encoding the MDR (multi-drug resistance) protein, which also has an ABC transporter structure (Anticancer Drug Des. 1999 Apr; 14(2):115-31. ). Other ABC transporters have been associated with neuronal and tumor conditions (US Patent No. 5,858,719) or potentially involved in diseases caused by impairment of the homeostasis of metals (Biochim Biophys Acta. 1999 Dec 6;1461(2):18-404. ). Likewise, another transport ABC, designated PFIC2, appears to be involved in a progressive familial intrahepatic cholestasia form, this protein being potentially responsible, in humans, for the export of bile salts (Strautnieks S S et al, *Nat Genet,* 1998, 20, 233-238).

Among the ABC sub-families, the ABCA gene subfamily is probably the most evolutionary complex. The *ABCA* genes and *OABP* represent the only two sub-families of ABC genes that do not have identifiable orthologs in the yeast genome (Decottignies and Goffeau, 1997; Michaelis and Berkower, 1995). There is, however, at least one *ABCA*-related gene in *C. elegans* (ced-7) and several in *Drosophila.* Thus the ABCA genes appear to have diverged after eukaryotes became multicellular and developed more sophisticated transport requirements. To date eleven members of the human ABCA sub-family have been described, making it the largest such group.

Full sequences of four genes of the ABCA sub-family have been described revealing a complex exon-intron structure. Best characterized ABCA genes are ABCA4, and ABCA1. In mammals the ABCA1 gene is highly expressed in macrophages and monocytes and is associated with the engulfment of apoptotic cells (Luciani et al, Genomics (1994) 21, 150-9; Moynault et al., Biochem Soc Trans (1998) 26, 629-35; Wu et al., Cell (1998) 93, 951-60). The ced-7 gene, ortholog of ABCA1 in C. elegans, plays a role in the recognition and engulfment of apoptotic cells suggesting a conserved function. Recently ABCA1 was demonstrated to be the gene responsible for Tangier disease, a disorder characterized by high levels of cholesterol in peripheral tissues, and a very low level of HDLs, and familial hypoalphalipoproteinemia (FHD) (Bodzioch et al., Nat Genet (1999) 22, 347-51; Brooks-Wilson et al., Nat Genet (1999) 336-45; Rust et al., Nat Genet (1999) 22, 352-5; Marcil et al., The Lancet (1999) 354, 1341-46). The ABCA1 protein is proposed to function in the reverse transport of cholesterol from peripheral tissues via an interaction with the apolipoprotein 1 (ApoA-1) of HDL tissues (Wang et al., JBC (2000). The ABCA2 gene is highly expressed in the brain and ABCA3 in the lung but no function has been ascribed to these loci. The ABCA4 gene is exclusively expressed in the rod photoreceptors of the retina and mutations thereof are responsible for several pathologies of human eyes, such as retinal degenerative disorders retinoids (Allikmets et al., Science (1997) 277, 1805-1807; Allikmets et al., Nat Genet (1997) 15, 236-246; Sun et al., J Biol Chem (1999) 8269-81; Weng et al., Cell (1999) 98, 13-23; Cremers et al., Hum Mol Genet (1998) 7, 355-362; Martinez-Mir et al., Genomics (1997) 40, 142-146). ABCA4 is believed to transport retinal and/or retinal-phospholipid complexes from the rod photoreceptor outer segment disks to the cytoplasm, facilitating phototransduction.

Therefore characterization of new genes from the ABCA subfamily is likely to yield biologically important transporters that may have an translocase activity for membrane lipid transport and may play a major role in human pathologies.

Lipids are water-insoluble organic biomolecules, which are essential components of diverse biological functions, including the storage, transport, and metabolism of energy, and membrane structure and fluidity. Lipids are derived from two sources in humans and other animals: some lipids are ingested as dietary fats and oils and other lipids are biosynthesized by the human or animal. In mammals at least 10% of the body weight is lipid, the bulk of which is in the form of triacylglycerols.

Triacylglycerols, also known as triglycerides and triacylglycerides, are made up of three fatty acids esterified to glycerol. Dietary triacylglycerols are stored in adipose tissues as a source of energy, or hydrolyzed in the digestive tract by triacylglycerol lipases, the most important of which is pancreatic lipase. Triacylglycerols are transported between tissues in the form of lipoproteins.

Lipoproteins are micelle-like assemblies found in plasma and contain varying proportions of different types of lipids and proteins (called apoproteins). There are five main classes of plasma lipoproteins, the major function of which is lipid transport. These classes are, in order of increasing density, chylomicrons, very low density lipoproteins (VLDL), intermediate-density lipoproteins (IDL), low density lipoproteins (LDL), and high density lipoproteins (HDL). Although many types of lipids are found associated with each lipoprotein class, each class transports predominantly one type of lipid: triacylglycerols are transported in chylomicrons, VLDL, and IDL; while phospholipids and cholesterol esters are transported in HDL and LDL respectively.

Phospholipids are di-fatty acid esters of glycerol phosphate, also containing a polar group coupled to the phosphate. Phospholipids are important structural components of cellular membranes. Phospholipids are hydrolyzed by enzymes called phospholipases. Phosphatidylcholine, an exemplary phospholipid, is a major component of most eukaryotic cell membranes.

Cholesterol is the metabolic precursor of steroid hormones and bile acids as well as an essential constituent of cell membranes. In humans and other animals, cholesterol is ingested in the diet and also synthesized by the liver and other tissues. Cholesterol is transported between tissues in the form of cholesteryl esters in LDLs and other lipoproteins.

Membranes surround every living cell, and serve as a barrier between the intracellular and extracellular compartments. Membranes also enclose the eukaryotic nucleus, make up the endoplasmic reticulum, and serve specialized functions such as in the myelin sheath that surrounds axons. A typical membrane contains about 40% lipid and 60% protein, but there is considerable variation. The major lipid components are phospholipids, specifically phosphatidylcholine and phosphatidylethanolamine, and cholesterol. The physicochemical properties of membranes, such as fluidity, can be changed by modification of either the fatty acid profiles of the phospholipids or the cholesterol content. Modulating the composition and organization of membrane lipids also modulates membrane-dependent cellular functions, such as receptor activity, endocytosis, and cholesterol flux.

High-density lipoproteins (HDL) are one of the five major classes of lipoproteins circulating in blood plasma. These lipoproteins are involved in various metabolic pathways such as lipid transport, the formation of bile acids, steroidogenesis, cell proliferation and, in addition, interfere with the plasma proteinase systems.

HDLs are perfect free cholesterol acceptors and, in combination with enzymatic activities such as that of the cholesterol ester transfer protein (CETP), the lipoprotein lipase (LPL), the hepatic lipase (HL) and the lecithin:cholesterol acyltransferase (LCAT), play a major role in the reverse transport of cholesterol, that is to say the transport of excess cholesterol in the peripheral cells to the liver for its elimination from the body in the form of bile acid. It has been demonstrated that the HDLs play a central role in the transport of cholesterol from the peripheral tissues to the liver.

Various diseases linked to an HDL deficiency have been described, including Tangier, FHD disease, and LCAT deficiency. In addition, HDL-cholesterol deficiencies have been observed in patients suffering from malaria and diabetes (Kittl et al., 1992. Wein Klin Wochenschr 104 :21-4; Nilsson et al., 1990, J. Intern. Med., 227:151-5; Djoumessi, 1989, Pathol Biol., 37:909-11; Mohanty et al., 1992. Ann Trop Med Parasitol., 86 :601-6; Maurois et al., 1985, Biochimie., 67 :227-39; Grellier et al., 1997. Vox Sang. 72 :211-20; Agbedana et al., 1990, Ann Trop Med Parasitol., 84 :529-30; Erel et al., 1998, Haematologia, Budap, 29 :207-12; Cuisinier et al., 1990, Med Trop, 50 :91-5; Chander et al., 1998, Indian J EXP Biol., 36 :371-4; Efthimiou et al., 1992, Wein Klin Wochenschr., 104 :705-6; Baptista et al., 1996. Parasite, 3:335-40; Davis et al., 1993, J. Infect. 26 :279-85; Davis et al., 1995, J. Infect. 31:181-8; Pinch et al., 1993, Semin Thromb Hemost., 19:138-43; Tomlinson and Raper, 1996, Nat. Biotechnol., 14:717-21; Hager and Hajduk, 1997, Nature 385:823-6; Kwiterovich, 1995, Ann NY Acad Sci., 748 :313-30; Syvanne et al. 1995, Circulation, 92:364-70; and Syvanne et al., 1995, J.LIPID Res., 36:573-82). The deficiency involved in Tangier and/or FHD disease is linked to a cellular defect in the translocation of cellular cholesterol which causes a degradation of the HDLs and leads to a disruption in the lipoprotein metabolism.

Atherosclerosis is defined in histological terms by deposits (lipid or fibrolipid plaques) of lipids and of other blood derivatives in blood vessel walls, especially the large arteries (aorta, coronary arteries, carotid). These plaques, which are more or less calcified according to the degree of progression of the atherosclerosis process, may be coupled with lesions and are associated with the accumulation in the vessels of fatty deposits consisting essentially of cholesterol esters. These plaques are accompanied by a thickening of the vessel wall, hypertrophy of the smooth muscle, appearance of foam cells (lipid-laden cells resulting from uncontrolled uptake of cholesterol by recruited macrophages) and accumulation of fibrous tissue. The atheromatous plaque protrudes markedly from the wall, endowing it with a stenosing character responsible for vascular occlusions by atheroma, thrombosis or embolism, which occur in those patients who are most affected. These lesions can lead to serious cardiovascular pathologies such as myocardial infarction, sudden death, cardiac insufficiency, and stroke.

Mutations within genes that play a role in lipoprotein metabolism have been identified. Specifically, several mutations in the apolipoprotein apoA-I gene have been characterized. These mutations are rare and may lead to a lack of production of apoA-I. Mutations in the genes encoding LPL or its activator apoC-II are associated with severe hypertriglyceridemias and substantially reduced HDL-C levels. Mutations in the gene encoding the enzyme LCAT are also associated with a severe HDL deficiency.

In addition, dysfunctions in the reverse transport of cholesterol may be induced by physiological deficiencies affecting one or more of the steps in the transport of stored cholesterol, from the intracellular vesicles to the membrane surface where it is accepted by the HDLs.

Therefore, an increasing need exists in the state of the art to identify genes involved in any of the steps in the metabolism of cholesterol and/or lipoproteins, and in particular, genes associated with dysfunctions in the reverse transport of cholesterol from the peripheral cells to the liver.

The applicant have discovered and characterized a gene cluster containing 4 new genes belonging to the ABCA protein sub-family, which have been designated ABCA5, ABCA6, ABCA9, and ABCA10. These new genes appear to be closely related to other ABCA subfamily members such as ABCA1 and ABCA8, as the particularly in the ATP-binding domain, and more particularly in the C-terminal ATP binding domains. The newly discovered genes also show considerable conservation of the amino acid sequences, particularly within the transmembrane region (TM) and the ATP-binding regions (NBD), and have a similar gene organization.

Surprisingly, the applicants have found these genes to be organized in a single large cluster on chromosome 17q24, in a head-to-tail fashion, with a similar intron/exon organization, suggesting that they have arisen from tandem duplication and may form a distinct functional group with the ABCA subfamily.

Furthermore, each of the discovered genes is transcribed with a tissue-specific distribution, but present an heterogenous pattern of expression, suggesting a regional and probably functional specialization of the corresponding proteins.

### SUMMARY OF THE INVENTION

The present invention relates to nucleic acids corresponding to the various human ABCA5, ABCA6, ABCA9, and ABCA10 genes, which are likely to be involved in the reverse transport of cholesterol, as well as in the membrane transport of lipophilic molecules, in particular inflammation mediating substances such as prostaglandins and prostacyclins, or in any pathology whose candidate chromosomal region is situated on chromosome 17, more precisely on the 17q arm and still more precisely in the 17q24 locus.

Thus, a first subject of the invention is a nucleic acid comprising a nucleotide sequence of any one of SEQ ID NOs: 1-4, and 9-126, or a complementary nucleotide sequence thereof.

The invention also relates to a nucleic acid comprising at least 8 consecutive nucleotides of a nucleotide sequence of a) any one of SEQ ID NOs: 1-4, and 9-126 or a complementary nucleotide sequence thereof.

The invention also relates to a nucleic acid having at least 80% nucleotide identity with a nucleic acid comprising a nucleotide sequence of any one of SEQ ID NOs: 1- 4, and 9-126, or a complementary nucleotide sequence thereof.

The invention also relates to a nucleic acid having at least 85%, preferably 90%, more preferably 95% and still more preferably 98% nucleotide identity with a nucleic acid comprising a nucleotide sequence of any one of SEQ ID NOs: 1-4 and 9-126, or a complementary nucleotide sequence thereof.

The invention also relates to a nucleic acid hybridizing, under high stringency conditions, with a nucleotide sequence of any one of SEQ ID NOs: 1-4 and 9-126, or a complementary nucleotide sequence thereof.

The invention also relates to nucleic acids, particularly cDNA molecules, which encode the full length human ABCA5, ABCA6, ABCA9, and ABCA10 proteins. Thus, the invention relates to a nucleic acid comprising a nucleotide sequence of any one of SEQ ID NO: 1-4, or of a complementary nucleotide sequence.

The invention also relates to a nucleic acid comprising a nucleotide sequence as depicted in SEQ ID NO: 1-4, or a complementary nucleotide sequence.

According to the invention, a nucleic acid comprising a nucleotide sequence of SEQ ID NO: 1, which encodes a full length ABCA5 polypeptide of 1642 amino acids comprising the amino acid sequence of SEQ ID NO: 5.

According to the invention, a nucleic acid comprising a nucleotide sequence of SEQ ID NO: 2, which encodes a full length ABCA6 polypeptide of 1617 amino acids comprising the amino acid sequence of SEQ ID NO: 6.

According to the invention, a nucleic acid comprising a nucleotide sequence of SEQ ID NO: 3, which encodes a full length ABCA9 polypeptide of 1624 amino acids comprising the amino acid sequence of SEQ ID NO: 7.

According to the invention, a nucleic acid comprising a nucleotide sequence of SEQ ID NO: 4, which encodes a full length ABCA10 polypeptide of 1543 amino acids comprising the amino acid sequence of SEQ ID NO: 8.

Thus, the invention also relates to a nucleic acid encoding a polypeptide comprising an amino acid sequence of any one of SEQ ID NO: 5-8.

Thus, the invention also relates to a polypeptide comprising an amino acid sequence of any one of SEQ ID NO: 5-8.

The invention also relates to a polypeptide comprising an amino acid sequence as depicted in any one of SEQ ID NO: 5-8.

The invention also relates to a means for detecting polymorphisms in general, and mutations in particular, in the ABCA5, ABCA6, ABCA9, and ABCA10 genes or in the corresponding proteins produced by the allelic form of these genes.

According to another aspect, the invention also relates to the nucleotide sequences of ABCA5, ABCA6, ABCA9, and ABCA10 genes comprising at least one biallelic polymorphism such as for example a substitution, addition or deletion of one or more nucleotides.

Nucleotide probes and primers hybridizing with a nucleic acid sequence located in the region of any one of ABCA5, ABCA6, ABCA9, and ABCA10 nucleic acid (genomic DNA, messenger RNA, cDNA), in particular, a nucleic acid sequence comprising any one of the mutations or polymorphisms.

The nucleotide probes or primers according to the invention comprise at least 8 consecutive nucleotides of a nucleic acid comprising any one of SEQ ID NOs: 1-4 and 9-126 or a complementary nucleotide sequence thereof.

Preferably, nucleotide probes or primers according to the invention will have a length of 10, 12, 15, 18 or 20 to 25, 35, 40, 50, 70, 80, 100, 200, 500, 1000, 1500 consecutive nucleotides of a nucleic acid according to the invention, in particular of a nucleic acid comprising any one of SEQ ID NOs: 1-4 and 9-126, or a complementary nucleotide sequence thereof.

Alternatively, a nucleotide probe or primer according to the invention will consist of and/or comprise fragments having a length of 12, 15, 18, 20, 25, 35, 40, 50, 100, 200, 500, 1000, 1500 consecutive nucleotides of a nucleic acid according to the invention, more particularly of a nucleic acid comprising any one of SEQ ID NOs: 1-4 and 9-126, or a complementary nucleotide sequence thereof.

The definition of a nucleotide probe or primer according to the invention therefore covers oligonucleotides which hybridize, under the high stringency hybridization conditions defined above, with a nucleic acid comprising any one of SEQ ID NOs: 1-4 and 9-126, or a complementary nucleotide sequence thereof.

The preferred probes and primers according to the invention comprise all or part of a nucleotide sequence comprising any one of SEQ ID NOs: 127-217, or a complementary nucleotide sequence thereof.

The nucleotide primers according to the invention may be used to amplify any one of the nucleic acids according to the invention, and more particularly a nucleic acid comprising a nucleotide sequence of any one of SEQ ID NOs: 1-4 and 9-126, or a complementary nucleotide sequence thereof.

According to the invention, some nucleotide primers specific for an ABCA5 gene, may be used to amplify a nucleic acid comprising a SEQ ID NOs: 1, and comprise a nucleotide sequence of any one of SEQ ID NOs: 127-150, or a complementary nucleotide sequence thereof.

The invention also relates to nucleotide primers are specific for an ABCA6 gene, may be used to amplify a nucleic acid comprising any one of SEQ ID NOs: 2 and 9-47, and comprise a nucleotide sequence of any one of SEQ ID NOs: 151-177, or a complementary nucleotide sequence.

The invention is further directed to nucleotide primers specific for an ABCA9 gene, may be used to amplify a nucleic acid comprising any one of SEQ ID NOs: 3, and 48-86, and comprise a nucleotide sequence of any one of SEQ ID NOs: 178-209, or a complementary nucleotide sequence.

The present invention is further directed to nucleotide primers specific for an ABCA10 gene, may be used to amplify a nucleic acid comprising any one of SEQ ID NOs: 4, and 87-126, and comprise a nucleotide sequence of any one of SEQ ID NOs: 210-217, or a complementary nucleotide sequence.

Another subject of the invention relates to a method of amplifying a nucleic acid according to the invention, and more particularly a nucleic acid comprising a) any one of SEQ ID NOs: 1-4 and 9-126, a complementary nucleotide sequence thereof, or b) as depicted in any one of SEQ ID NOs: 1-4 and 9-126, or a complementary nucleotide sequence thereof, contained in a sample, said method comprising the steps of:
a) bringing the sample in which the presence of the target nucleic acid is suspected into contact with a pair of nucleotide primers whose hybridization position is located respectively on the 5' side and on the 3' side of the region of the target nucleic acid whose amplification is sought, in the presence of the reagents necessary for the amplification reaction; and
b) detecting the amplified nucleic acids.

The present invention also relates to a method of detecting the presence of a nucleic acid comprising a nucleotide sequence of any one of SEQ ID NOs: 1-4 and 9-126, or a complementary nucleotide sequence, or a nucleic acid fragment or variant of any one of SEQ ID NOs: 1-4 and 9-126, or a complementary nucleotide sequence in a sample, said method comprising the steps of:
1) bringing one or more nucleotide probes according to the invention into contact with the sample to be tested;
2) detecting the complex which may have formed between the probe(s) and the nucleic acid present in the sample.

According to a specific embodiment of the method of detection according to the invention, the oligonucleotide probes are immobilized on a support.

According to another aspect, the oligonucleotide probes comprise a detectable marker.

Another subject of the invention is a box or kit for amplifying all or part of a nucleic acid comprising a) any one of SEQ ID NOs: 1-4 and 9-126, or a complementary nucleotide sequence thereof, or b) as depicted in any one of SEQ ID NOs: 1-4 and 9-126 or of a complementary nucleotide sequence thereof, said box or kit comprising:
1) a pair of nucleotide primers in accordance with the invention, whose hybridization position is located respectively on the 5' side and 3' side of the target nucleic acid whose amplification is sought; and optionally,
2) reagents necessary for an amplification reaction.

Such an amplification box or kit will preferably comprise at least one pair of nucleotide primers as described above.

The invention also relates to a box or kit for detecting the presence of a nucleic acid according to the invention in a sample, said box or kit comprising:
a) one or more nucleotide probes according to the invention;
b) appropriate reagents necessary for a hybridisation reaction.

According to a first aspect, the detection box or kit is characterized in that the nucleotide probe(s) and primer(s)are immobilized on a support.

According to a second aspect, the detection box or kit is characterized in that the nucleotide probe(s) and primer(s) comprise a detectable marker.

According to a specific embodiment of the detection kit described above, such a kit will comprise a plurality of oligonucleotide probes and/or primers in accordance with the invention which may be used to detect target nucleic acids of interest or alternatively to detect mutations in the coding regions or the non-coding regions of the nucleic acids according to the invention. According to preferred embodiment of the invention, the target nucleic acid comprises a nucleotide sequence of any one of SEQ ID NOs: 1-4 and 9-126, or of a complementary nucleic acid sequence. Alternatively, the target nucleic acid is a nucleic acid fragment or variant of a nucleic acid comprising any one of SEQ ID NOs: 1-4 and 9-126, or of a complementary nucleotide sequence.

According to another preferred embodiment, a primer according to the invention comprises, generally, all or part of any one of SEQ ID NOs: 1-4, and 9-217, or a complementary sequence.

The invention also relates to a recombinant vector comprising a nucleic acid according to the invention. Preferably, such a recombinant vector will comprise a nucleic acid selected from the group consisting of
a) a nucleic acid comprising a nucleotide sequence of any one of SEQ ID NOs: 1-4 and 9-126, or a complementary nucleotide sequence thereof,
b) a nucleic acid comprising a nucleotide sequence as depicted in any one of SEQ ID NOs: 1-4 and 9-126, or a complementary nucleotide sequence thereof,
c) a nucleic acid having at least eight consecutive nucleotides of a nucleic acid comprising a nucleotide sequence of any one of SEQ ID NOs: 1-4 and 9-126, or a complementary nucleotide sequence thereof;
d) a nucleic acid having at least 80% nucleotide identity with a nucleic acid comprising a nucleotide sequence of any one of SEQ ID NOs: 1-4 and 9-126, or a complementary nucleotide sequence thereof;
e) a nucleic acid having 85%, 90%, 95%, or 98% nucleotide identity with a nucleic acid comprising a nucleotide sequence of any one of SEQ ID NOs: 1-4 and 9-126, or a complementary nucleotide sequence thereof;
f) a nucleic acid hybridizing, under high stringency hybridization conditions, with a nucleic acid comprising a nucleotide sequence of any one of SEQ ID NOs: 1-4 and 9-126, or a complementary nucleotide sequence; and
g) a nucleic acid encoding a polypeptide comprising an amino acid sequence of SEQ ID NO: 5-8.

According to a first embodiment, a recombinant vector according to the invention is used to amplify a nucleic acid inserted therein, following transformation or transfection of a desired cellular host.

According to a second embodiment, a recombinant vector according to the invention corresponds to an expression vector comprising, in addition to a nucleic acid in accordance with the invention, a regulatory signal or nucleotide sequence that directs or controls transcription and/or translation of the nucleic acid and its encoded mRNA.

According to a preferred embodiment, a recombinant vector according to the invention will comprise in particular the following components:
(1) an element or signal for regulating the expression of the nucleic acid to be inserted, such as a promoter and/or enhancer sequence;
(2) a nucleotide coding region comprised within the nucleic acid in accordance with the invention to be inserted into such a vector, said coding region being placed in phase with the regulatory element or signal described in (1); and
(3) an appropriate nucleic acid for initiation and termination of transcription of the nucleotide coding region of the nucleic acid described in (2).

The present invention also relates to a defective recombinant virus comprising a cDNA nucleic acid encoding any one of ABCA5, ABCA6, ABCA9, and ABCA10 polypeptides involved in the transport of lipophilic substances, in particular mediators of inflammation , or in any pathology whose candidate chromosomal region is situated on chromosome 17, more precisely on the 17q arm and still more precisely in the 17q24 locus.

In another preferred embodiment of the invention, the defective recombinant virus comprises a gDNA nucleic acid encoding any one of ABCA5, ABCA6, ABCA9, ABCA10 polypeptides involved in the transport of lipophilic substances, inflammatory lipophilic substances. Preferably, the ABCA5, ABCA6, ABCA9, and ABCA10 polypeptides comprise amino acid sequences selected from SEQ ID NO: 5-8, respectively.

In another preferred embodiment, the invention relates to a defective recombinant virus comprising a nucleic acid encoding any one of ABCA5, ABCA6, ABCA9, ABCA10 polypeptides involved in the transport of inflammatory lipophilic substances, under the control of a promoter chosen from RSV-LTR or the CMV early promoter.

According to a specific embodiment, a method of introducing a nucleic acid according to the invention into a host cell, in particular a host cell obtained from a mammal, *in vivo*, comprises a step during which a preparation comprising a pharmaceutically compatible vector and a "naked" nucleic acid according to the invention, placed under the control of appropriate regulatory sequences, is introduced by local injection at the level of the chosen tissue, for example a smooth muscle tissue, the "naked" nucleic acid being absorbed by the cells of this tissue.

According to a specific embodiment of the invention, a composition is provided for the *in vivo* production of any one of the ABCA5, ABCA6, ABCA9, and ABCA10 proteins. This composition comprises a nucleic acid encoding the ABCA5, ABCA6, ABCA9, and ABCA10 polypeptides placed under the control of appropriate regulatory sequences, in solution in a physiologically acceptable vehicle and/or excipient.

Therefore, the present invention also relates to a composition comprising a nucleic acid encoding any one of ABCA5, ABCA6, ABCA9, ABCA10 polypeptides comprising an amino acid sequence selected from SEQ ID NO: 5-8, wherein the nucleic acid is placed under the control of appropriate regulatory elements.

Consequently, the invention also relates to a pharmaceutical composition intended for the prevention of or treatment of a patient or subject affected by a dysfunction in the reverse transport of cholesterol or in the transport of inflammatory lipophilic substances comprising a nucleic acid encoding any one of ABCA5, ABCA6, ABCA9, and ABCA10 proteins, in combination with one or more physiologically compatible excipients.

Preferably, such a composition will comprise a nucleic acid comprising a nucleotide sequence of any one of SEQ ID NO:1-4 and 9-126, wherein the nucleic acid is placed under the control of an appropriate regulatory element or signal.

In addition, the present invention is directed to a pharmaceutical composition intended for the prevention of or treatment of a patient or a subject affected by a dysfunction in the reverse transport of cholesterol or in the transport of liphophilic substances mediating inflammation, comprising a recombinant vector according to the invention, in combination with one or more physiologically compatible excipients.

The invention also relates to the use of a nucleic acid according to the invention encoding any one of ABCA5, ABCA6, ABCA9, and ABCA10 proteins for the manufacture of a medicament intended for the prevention of arteriosclerosis in various forms or more particularly for the treatment of subjects affected by a dysfunction of cholesterol reverse transport or transport of liphophilic substances mediating inflammation.

The invention also relates to the use of a recombinant vector according to the invention comprising a nucleic acid encoding any one of ABCA5, ABCA6, ABCA9, and ABCA10 proteins for the manufacture of a medicament intended for the prevention of arteriosclerosis in various forms or more particularly for the treatment of subjects affected by a dysfunction of cholesterol reverse transport or transport of liphophilic substances mediating inflammation.

The subject of the invention is therefore also a recombinant vector comprising a nucleic acid according to the invention that encodes any one of ABCA5, ABCA6, ABCA9 and ABCA10 proteins or polypeptides involved in the metabolism of cholesterol or transport of liphophilic substances mediating inflammation.

The invention also relates to the use of such a recombinant vector for the preparation of a pharmaceutical composition intended for the treatment and/or for the prevention of diseases or conditions associated with deficiency of lipophilic substances signaling inflammation, or deficiency of in the cholesterol reverse transport, or transport of inflammatory lipophilic substances.

The present invention also relates to the use of cells genetically modified *ex vivo* with such a recombinant vector according to the invention, or cells producing a recombinant vector, wherein the cells are implanted in the body, to allow a prolonged and effective expression *in vivo* of any one biologically active ABCA5, ABCA6, ABCA9, and ABCA10 polypeptides.

The invention also relates to the use of a nucleic acid according to the invention encoding any one of ABCA5, ABCA6, ABCA9 and ABCA10 proteins for the manufacture of a medicament intended for the prevention and/or the treatment of subjects affected by a dysfunction of cholesterol reverse transport or inflammatory lipophilic substances transport.

The invention also relates to the use of a recombinant vector according to the invention comprising a nucleic acid encoding any one of ABCA5, ABCA6, ABCA9 and ABCA10 polypeptides according to the invention for the manufacture of a medicament intended for the prevention and/or the treatment of subjects affected by a dysfunction of the reverse transport of cholesterol or inflammatory lipophilic substances transport.

The invention also relates to the use of a recombinant host cell according to the invention, comprising a nucleic acid encoding any one of ABCA5, ABCA6, ABCA9 and ABCA10 polypeptides according to the invention for the manufacture of a medicament intended for the prevention and/or the treatment of subjects affected by a dysfunction of cholesterol reverse transport.

The present invention also relates to the use of a recombinant vector according to the invention, preferably a defective recombinant virus, for the preparation of a pharmaceutical composition for the treatment and/or prevention of pathologies linked to the dysfunction of cholesterol reverse transport or inflammatory lipophilic substances transport.

The invention relates to the use of such a recombinant vector or defective recombinant virus for the preparation of a pharmaceutical composition intended for the treatment and/or for the prevention of cardiovascular disease linked to a deficiency in the reverse transport of cholesterol. Thus, the present invention also relates to a pharmaceutical composition comprising one or more recombinant vectors or defective recombinant viruses according to the invention.

The present invention also relates to the use of cells genetically modified *ex vivo* with a virus according to the invention, or of cells producing such viruses, implanted in the body, allowing a prolonged and effective expression *in vivo* of any one biologically active of ABCA5, ABCA6, ABCA9 and ABCA10 proteins.

The present invention shows that it is possible to incorporate a nucleic acid encoding any one of ABCA5, ABCA6, ABCA9 and ABCA10 polypeptides according to the invention into a viral vector, and that these vectors make it possible to effectively express a biologically active, mature polypeptide. More particularly, the invention shows that the *in vivo* expression of any one of ABCA5, ABCA6, ABCA9 and ABCA10 proteins may be obtained by direct administration of an adenovirus or by implantation of a producing cell or of a cell genetically modified by an adenovirus or by a retrovirus incorporating such a nucleic acid.

In this regard, another subject of the invention relates to any mammalian cell infected with one or more defective recombinant viruses according to the invention. More particularly, the invention relates to any population of human cells infected with these viruses. These may be in particular cells of blood origin (totipotent stem cells or precursors), fibroblasts, myoblasts, hepatocytes, keratinocytes, smooth muscle and endothelial cells, glial cells and the like.

Another subject of the invention relates to an implant comprising mammalian cells infected with one or more defective recombinant viruses according to the invention or cells producing recombinant viruses, and an extracellular matrix. Preferably, the implants according to the invention comprise 10⁵ to 10¹⁰ cells. More preferably, they comprise 10⁶ to 10⁸ cells.

More particularly, in the implants of the invention, the extracellular matrix comprises a gelling compound and optionally, a support allowing the anchorage of the cells.

The invention also relates to a recombinant host cell comprising a nucleic acid of the invention, and more particularly, a nucleic acid comprising any one of SEQ ID NO: 1-4 and 9-126, or of a complementary nucleotide sequence.

The invention also relates to a recombinant host cell comprising a nucleic acid of the invention, and more particularly a nucleic acid comprising a nucleotide sequence as depicted in any one SEQ ID NO: 1-4 and 9-126, or of a complementary nucleotide sequence.

According to another aspect, the invention also relates to a recombinant host cell comprising a recombinant vector according to the invention. Therefore, the invention also relates to a recombinant host cell comprising a recombinant vector comprising any of the nucleic acids of the invention, and more particularly a nucleic acid comprising any one nucleotide sequence of SEQ ID NO: 1-4 and 9-126, or a complementary nucleotide sequence thereof.

Specifically, the invention relates to a recombinant host cell comprising a recombinant vector comprising a nucleic acid comprising any one of SEQ ID NOs: 1-4 and 9-126, or a complementary nucleotide sequence thereof.

The invention also relates to a recombinant host cell comprising a recombinant vector comprising a nucleic acid comprising a nucleotide sequence as depicted in any one of SEQ ID NOs: 1-4 and 9-126, or of a complementary nucleotide sequence thereof.

The invention also relates to a recombinant host cell comprising a recombinant vector comprising a nucleic acid encoding a polypeptide comprising any one amino acid sequence of SEQ ID NO:5-8.

The invention also relates to a method for the production of a polypeptide comprising an amino acid sequence of any one of SEQ ID NOs: 1-4 and 9-126, or of a peptide fragment or a variant thereof, said method comprising the steps of:
a) inserting a nucleic acid encoding said polypeptide into an appropriate vector;
b) culturing, in an appropriate culture medium, a previously transformed host cell or transfecting a host cell with the recombinant vector of step a);
c) recovering the conditioned culture medium or lysing the host cell, for example by sonication or by osmotic shock;
d) separating and purifying said polypeptide from said culture medium or alternatively from the cell lysates obtained in step c); and
e) where appropriate, characterizing the recombinant polypeptide produced.

A polypeptide termed "homologous" to a polypeptide having an amino acid sequence selected from SEQ ID NO: 5-8 also forms part of the invention. Such a homologous polypeptide comprises an amino acid sequence possessing one or more substitutions of an amino acid by an equivalent amino acid.

The ABCA5, ABCA6, ABCA9, ABCA10 polypeptides according to the invention, in particular 1) a polypeptide comprising an amino acid sequence of any one of SEQ ID NOs: 5-8, 2) a polypeptide fragment or variant of a polypeptide comprising an amino acid sequence of any one of SEQ ID NOs: 5-8, or 3) a polypeptide termed "homologous" to a polypeptide comprising amino acid sequence selected from SEQ ID NO:5-8.

In a specific embodiment, an antibody according to the invention is directed against 1) a polypeptide comprising an amino acid sequence of any one of SEQ ID NOs: 5-8, 2) a polypeptide fragment or variant of a polypeptide comprising an amino acid sequence selected from SEQ ID NOs: 5-8, or 3) a polypeptide termed "homologous" to a polypeptide comprising amino acid sequence selected from SEQ ID NO: 5-8. Such antibody is produced by using the trioma technique or the hybridoma technique described by Kozbor et al. (Immunology Today, (1983) 4:72).

Thus, the subject of the invention is, in addition, a method of detecting the presence of any one of the polypeptides according to the invention in a sample, said method comprising the steps of:
a) bringing the sample to be tested into contact with an antibody directed against 1) a polypeptide comprising an amino acid sequence of any one of SEQ ID NOs: 5-8, 2) a polypeptide fragment or variant of a polypeptide comprising an amino acid sequence selected from SEQ ID NOs: 5-8, 3) a polypeptide termed "homologous" to a polypeptide comprising amino acid sequence of any one of SEQ ID NO: 5-8, and
b) detecting the antigen/antibody complex formed.

The invention also relates to a box or kit for diagnosis or for detecting the presence of any one of polypeptide in accordance with the invention in a sample, said box comprising:
a) an antibody directed against 1) a 5-8, 2) a polypeptide fragment or variant of a polypeptide comprising an amino acid sequence of any one of SEQ ID NOs: 5-8, or 3) a polypeptide "homologous" to a polypeptide comprising amino acid sequence of SEQ ID NO: 5-8, and
b) a reagent allowing the detection of the antigen/antibody complexes formed.

The invention also relates to a pharmaceutical composition comprising a nucleic acid according to the invention.

The invention also provides pharmaceutical compositions comprising a nucleic acid encoding any one of ABCA5, ABCA6, ABCA9, and ABCA10 polypeptides according to the invention and pharmaceutical compositions comprising any one of ABCA5, ABCA6, ABCA9, ABCA10 polypeptides according to the invention intended for the treatment of diseases linked to a deficiency of cholesterol reverse transport or inflammatory lipophilic substances transport.

The present invention also relates to a new therapeutic approach for the treatment of pathologies linked to deficiency of cholesterol reverse transport or inflammatory lipophilic substances transport, comprising transferring and expressing *in vivo* nucleic acids encoding any one of ABCA5, ABCA6, ABCA9, and ABCA10 proteins according to the invention.

Thus, the present invention offers a new approach for the treatment and/or prevention of pathologies linked to the abnormalities of cholesterol reverse transport or inflammatory lipophilic substances. Specifically, the present invention provides methods to restore or promote improved cholesterol reverse transport or improved inflammatory lipophilic substances transport in a patient or subject.

Consequently, the invention also relates to a pharmaceutical composition intended for the prevention and/or treatment of subjects affected by, a dysfunction of cholesterol reverse transport or inflammatory lipophilic substances transport, comprising a nucleic acid encoding any one of the ABCA5, ABCA6, ABCA9, and ABCA10 proteins, in combination with one or more physiologically compatible vehicle and/or excipient.

According to a specific embodiment of the invention, a composition is provided for the *in vivo* production of any one of the ABCA5, ABCA6, ABCA9, and ABCA10 proteins. This composition comprises a nucleic acid encoding any one of the ABCA5, ABCA6, ABCA9, and ABCA10 polypeptides placed under the control of appropriate regulatory sequences, in solution in a physiologically compatible vehicle and/or excipient.

Therefore, the present invention also relates to a composition comprising a nucleic acid encoding a polypeptide comprising an amino acid sequence of any one of ID NO: 5-8, wherein the nucleic acid is placed under the control of appropriate regulatory elements.

Preferably, such a composition will comprise a nucleic acid comprising a nucleotide sequence of any one of SEQ ID NO: 1-4 and 9-126, placed under the control of appropriate regulatory elements.

The invention also relates to a pharmaceutical composition intended for the prevention of or treatment of subjects affected by, a dysfunction of cholesterol reverse transport or inflammatory lipophilic substances transport, comprising a recombinant vector according to the invention, in combination with one or more physiologically compatible vehicle and/or excipient.

According to another aspect, the subject of the invention is also a preventive or curative therapeutic method of treating diseases caused by a deficiency of cholesterol reverse transport or inflammatory lipophilic substances transport, such a method comprising administering to a patient a nucleic acid encoding any one of ABCA5, ABCA6, ABCA9, and ABCA10 polypeptides according to the invention, said nucleic acid being combined with one or more physiologically appropriate vehicles and/or excipients.

The invention relates to a pharmaceutical composition for the prevention and/or treatment of a patient or subject affected by a dysfunction of cholesterol reverse transport or inflammatory lipophilic substances transport, comprising a therapeutically effective quantity of a polypeptide having an amino acid sequence selected from SEQ ID NO: 5-8, combined with one or more physiologically appropriate vehicles and/or excipients.

According to a specific embodiment, a method of introducing at least a nucleic acid according to the invention into a host cell, in particular a host cell obtained from a mammal, *in vivo*, comprises a step during which a preparation comprising a pharmaceutically compatible vector and a "naked" nucleic acid according to the invention, placed under the control of appropriate regulatory sequences, is introduced by local injection at the level of the chosen tissue, for example a smooth muscle tissue, the "naked" nucleic acid being absorbed by the cells of this tissue.

According to yet another aspect, the subject of the invention is also a preventive or curative therapeutic method of treating diseases caused by a deficiency of cholesterol reverse transport or inflammatory lipophilic substances transport, such a method comprising administering to a patient a therapeutically effective quantity of at least one ABCA5, ABCA6, ABCA9, or ABCA10 polypeptide according to the invention, said polypeptide being combined with one or more physiologically appropriate vehicles and/or excipients.

The invention also provides methods for screening small molecules and compounds that act on any one of ABCA5, ABCA6, ABCA9, and ABCA10 proteins to identify agonists and antagonists of such polypeptides that can restore or promote improved cholesterol reverse transport or inflammatory lipophilic substances transport to effectively cure and or prevent dysfunctions thereof. These methods are useful to identify small molecules and compounds for therapeutic use in the treatment of diseases due to a deficiency of cholesterol reverse transport or inflammatory lipophilic substances transport.

Therefore, the invention also relates to the use of any one of ABCA5, ABCA6, ABCA9, and ABCA10 polypeptides or a cell expressing any one of ABCA5, ABCA6, ABCA9, and ABCA10 polypeptides according to the invention, for screening active ingredients for the prevention and/or treatment of diseases resulting from a dysfunction cholesterol reverse transport or inflammatory lipophilic substances transport.

The invention also relates to a method of screening a compound or small molecule, an agonist or antagonist of any one of ABCA5, ABCA6, ABCA9, and ABCA10 polypeptides, said method comprising the following steps:
a) preparing a membrane vesicle comprising any one of ABCA5, ABCA6, ABCA9, and ABCA10 polypeptides and a lipid substrate comprising a detectable marker;
b) incubating the vesicle obtained in step a) with an agonist or antagonist candidate compound;
c) qualitatively and/or quantitatively measuring release of the lipid substrate comprising a detectable marker; and
d) comparing the release measurement obtained in step b) with a measurement of release of a labelled lipid substrate by a vesicle that has not been previously incubated with the agonist or antagonist candidate compound.

In a first specific embodiment, the ABCA5, ABCA6, ABCA9, and ABCA10 polypeptides comprise SEQ ID NO: 5-8, respectively.

The invention also relates to a method of screening a compound or small molecule, an agonist or antagonist of any one of ABCA5, ABCA6, ABCA9, and ABCA10 polypeptides, said method comprising the following steps:
a) obtaining a cell, for example a cell line, that, either naturally or after transfecting the cell with any one of ABCA5, ABCA6, ABCA9, and ABCA10 ABC1 encoding nucleic acids, expressing the corresponding ABCA5, ABCA6, ABCA9, and ABCA10 polypeptides;
b) incubating the cell of step a) in the presence of an anion labelled with a detectable marker;
c) washing the cell of step b) in order to remove the excess of the labelled anion which has not penetrated into these cells;
d) incubating the cell obtained in step c) with an agonist or antagonist candidate compound for the any one of ABCA5, ABCA6, ABCA9, and ABCA10 polypeptides;
e) measuring efflux of the labelled anion; and
f) comparing the value of efflux of the labelled anion determined in step e) with a value of efflux of a labelled anion measured with cell which has not been previously incubated in the presence of the agonist or antagonist candidate compound for any one of the ABCA5, ABCA6, ABCA9, and ABCA10 polypeptides.

In a first specific embodiment, the ABCA5, ABCA6, ABCA9, and ABCA10 polypeptides comprise SEQ ID NO: 5-8, respectively.

The invention also relates to a method of screening a compound or small molecule active, an agonist or antagonist of any one of ABCA5, ABCA6, ABCA9, and ABCA10 polypeptides, said method comprising the following steps:
a) culturing cells of a human monocytic line in an appropriate culture medium, in the presence of purified human albumin;
b) incubating the cells of step a) simultaneously in the presence of a compound stimulating the production of IL-1 beta and of the agonist or antagonist candidate compound;
c) incubating the cells obtained in step b) in the presence of an appropriate concentration of ATP;
d) measuring IL-1 beta released into the cell culture supernatant; and
e) comparing the value of the release of the IL-1 beta obtained in step d) with the value of the IL-1 beta released into the culture supernatant of cells which have not been previously incubated in the presence of the agonist or antagonist candidate compound.

### BRIEF DESCRIPTION OF THE DRAWINGS

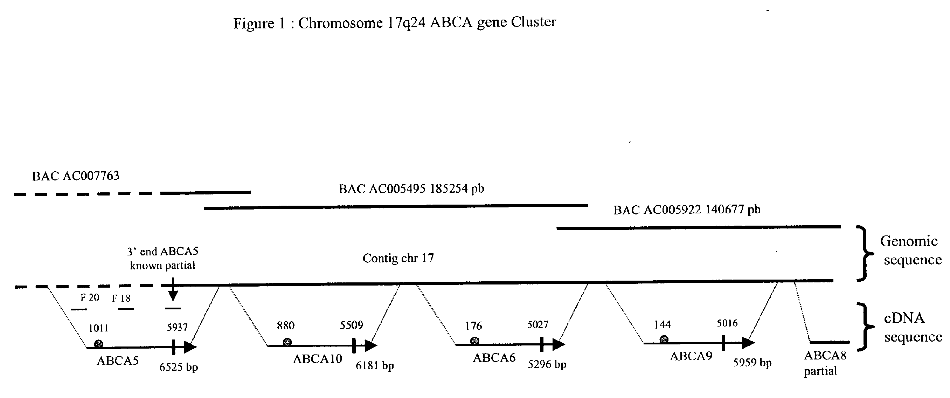

- Figure 1:: represents the Map of the 17q24 region containing the ABCA5,6,8-10 genes. A physical map of the portion of chromosome 17q24 is shown containing the 5 *ABCA* genes. Location of the microsatellite marker *D17S940* locus is indicated, along with the boundaries of BAC clones hRPK.235_I_10 and hRPK.293_K_20 (GenBank accession #s AC005495, AC005922). Genes orientation is indicated by the arrows, and the size and location of the corresponding transcripts is shown below the map. • indicates the initiation codon; | represents the stop codon;--- symbolizes the working draft sequences.

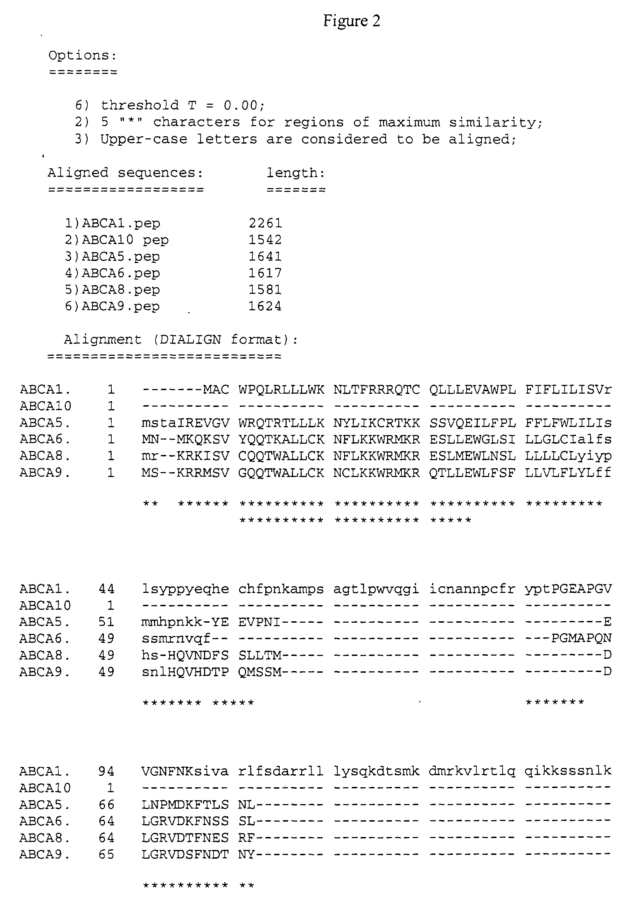

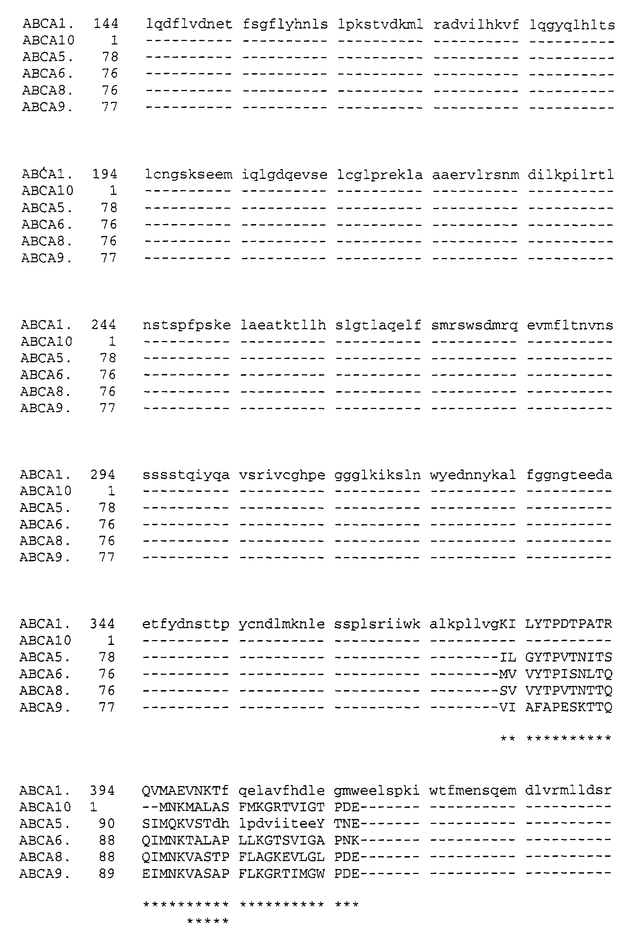

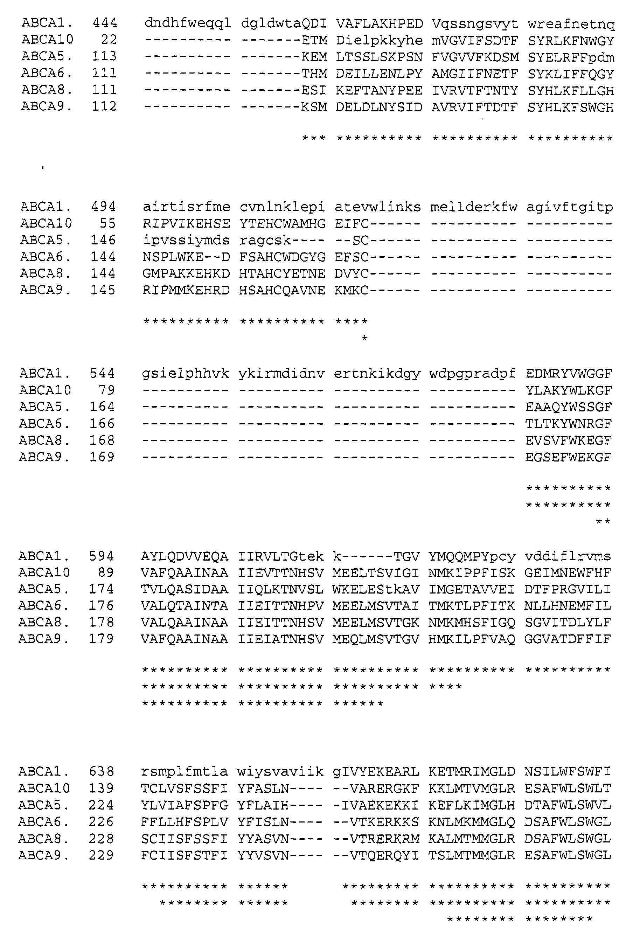

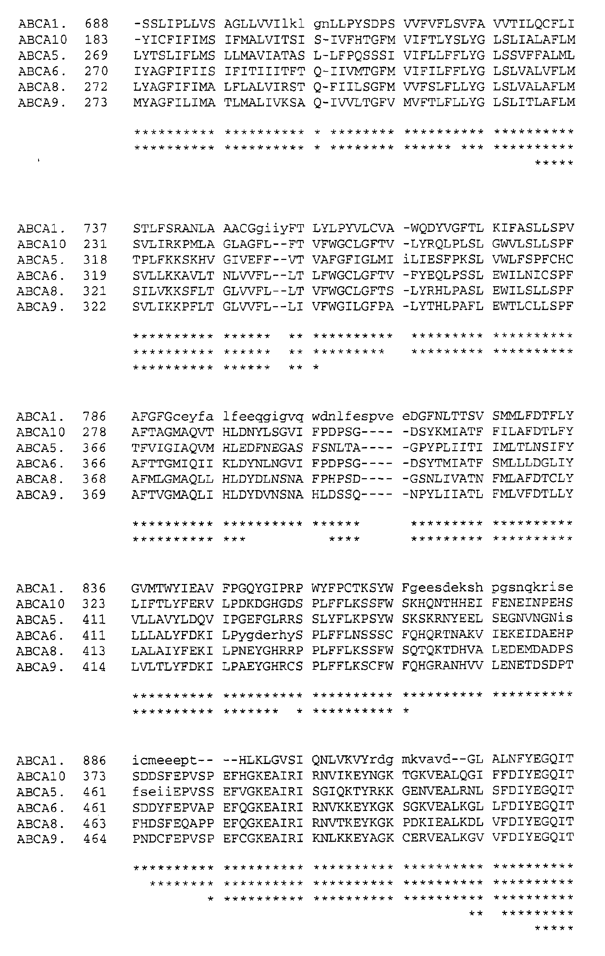

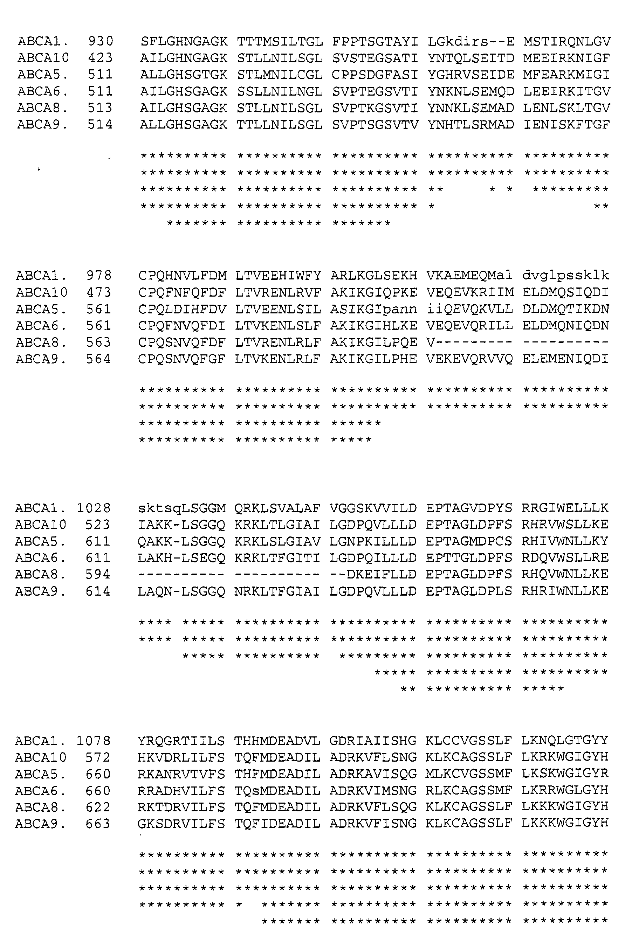

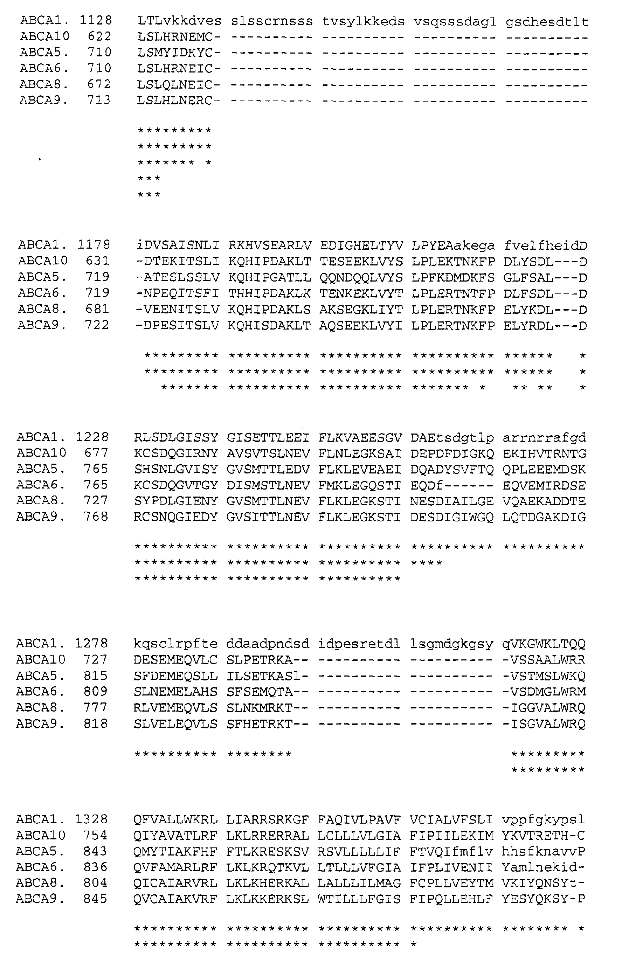

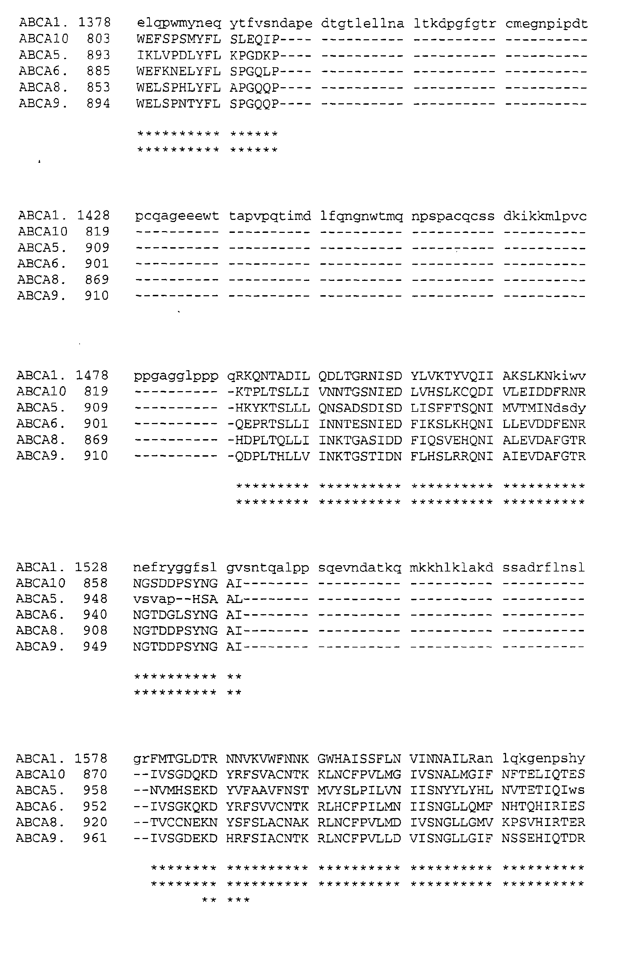

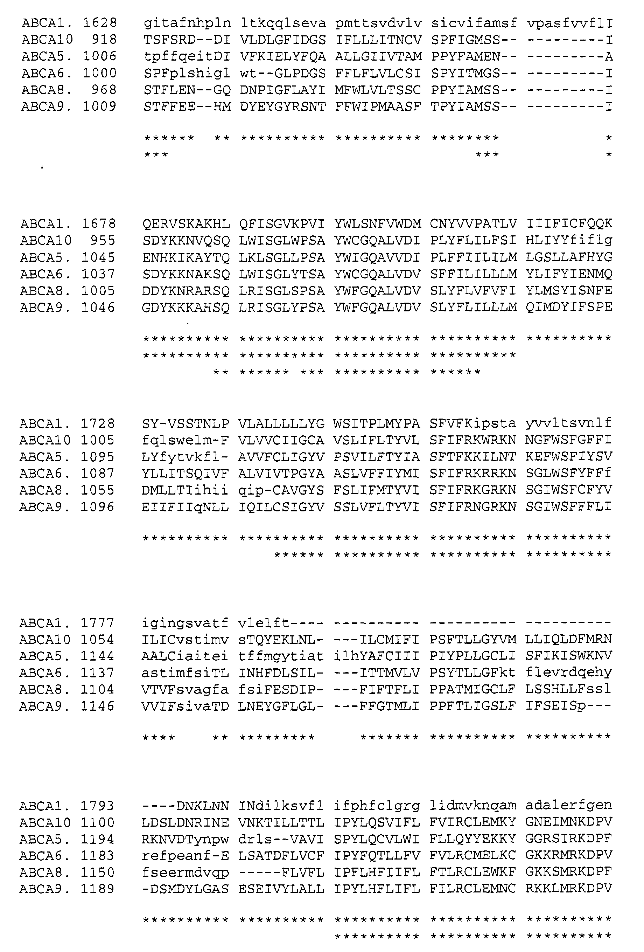

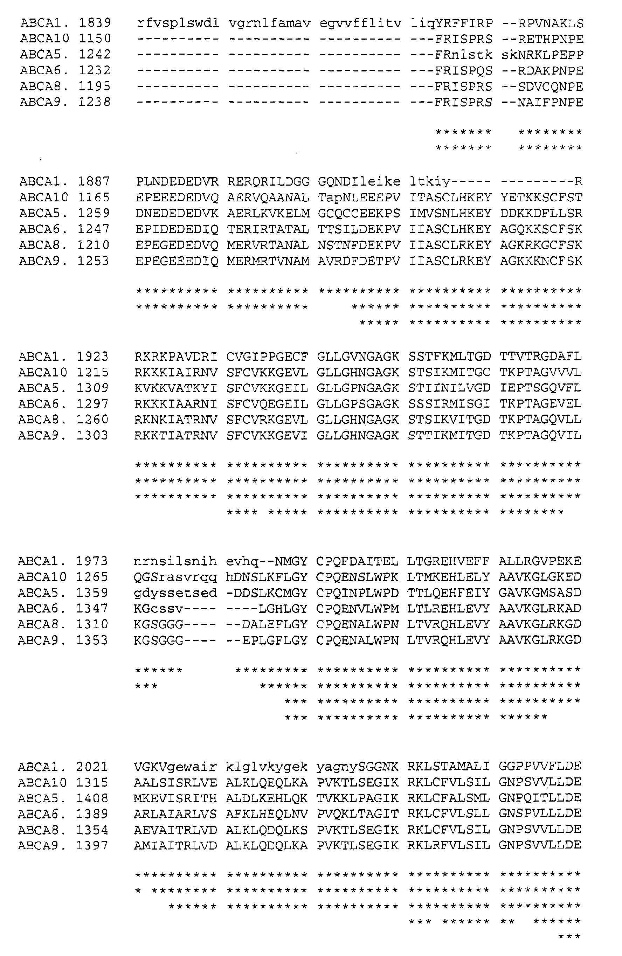

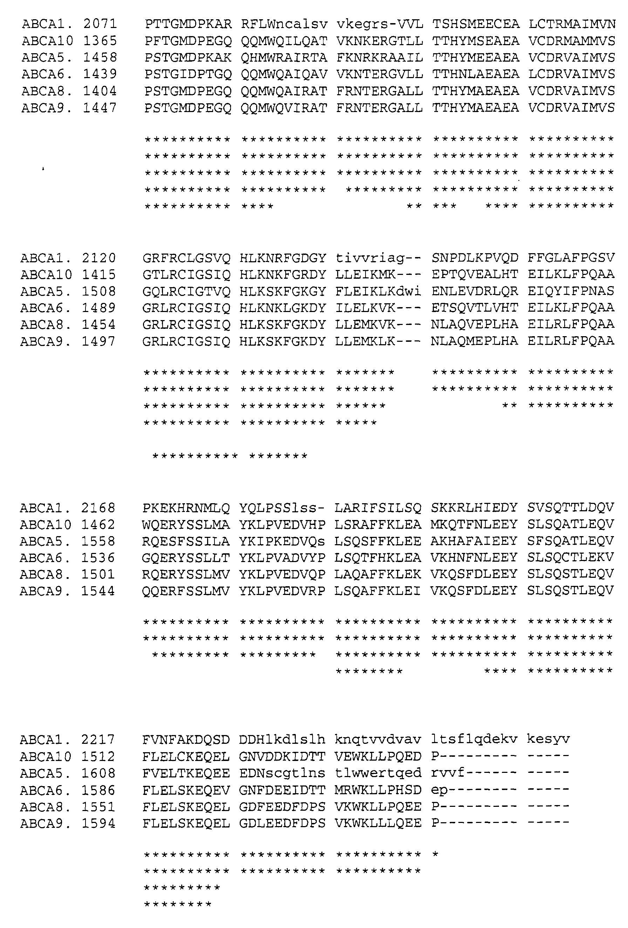

- Figure 2:: represents the alignment of ABC1-like genes. An alignment of the amino acid sequence of the full-length ABCA6, 8, 9, and 10 open reading frames, and the partial sequence of ABCA5 and ABCA10 is shown as aligned to ABCA1.

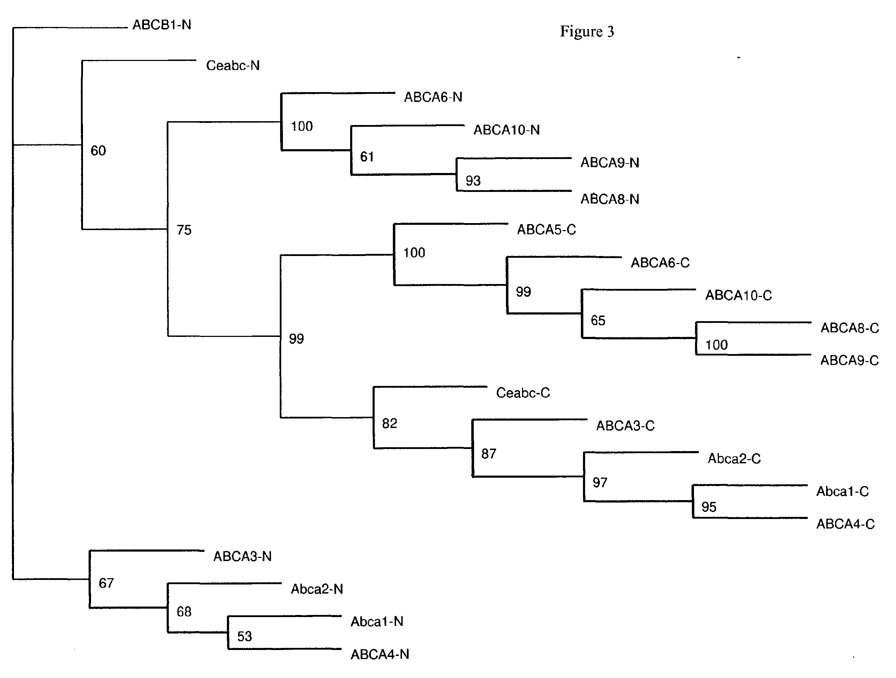

- Figure 3:: Maximum parsimony tree of ABC1-like genes. Phylogenetic trees were constructed with the alignments of the N- and C- terminal ATP-binding domains' sequence by both neighbor joining and maximum parsimony methods.

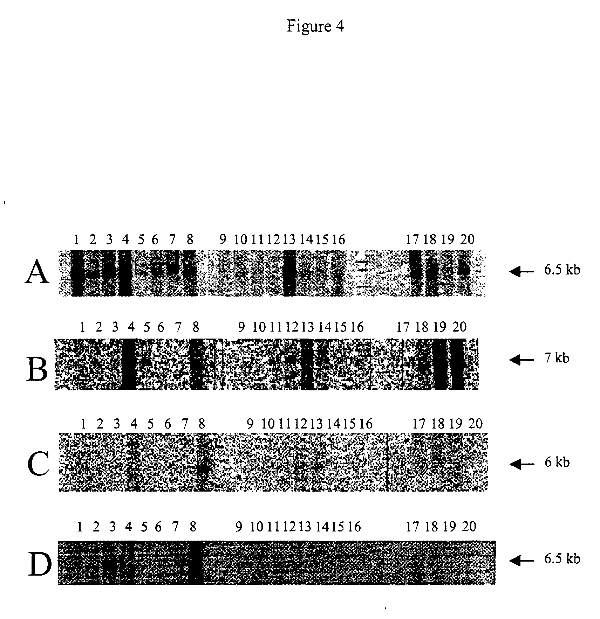

- Figure 4.: Northern blot analysis of poly(A)+ RNA from 20 human tissues: pancreas (lane 1), kidney (2), skeletal muscle (3), liver (4), lung (5), placenta (6), brain (7), heart (8), leukocyte (9), colon (10), small intestine (11), ovary (12), testis (13), prostate (14), thymus (15), spleen (16), fetal kidney (17), fetal liver (18), fetal lung (19) and fetal brain (20). Hybridization with a probe specific of ABCA5 (A), ABCA6 (B), ABCA9 (C), and ABCA10 (D).

### DETAILED DESCRIPTION OF THE INVENTION

### GENERAL DEFINITIONS

The present invention contemplates isolation of human genes encoding ABCA5, ABCA6, ABCA9, and ABCA10 polypeptides of the invention, including full lengths, or naturally occurring forms of ABCA5, ABCA6, ABCA9, and ABCA10 and any antigenic fragments thereof from any animal, particularly mammalian or avian, and more particularly human source.

In accordance with the present invention, conventional molecular biology, microbiology, and recombinant DNA techniques within the skill of the art are used. Such techniques are fully explained in the literature (Sambrook et al., 1989. Molecular cloning a laboratory manual. 2ed. Cold Spring Harbor Laboratory, Cold spring Harbor, New York; Glover, 1985, DNA Cloning: A pratical approach, volumes I and II oligonucleotide synthesis, MRL Press, LTD.., Oxford, U.K.; Hames and Higgins, 1985, Transcription and translation; Hames and Higgins, 1984, Animal Cell Culture; Freshney, 1986, Immobilized Cells And Enzymes, IRL Press; and Perbal, 1984, A practical guide to molecular cloning).

As used herein, the term "gene" refers to an assembly of nucleotides that encode a polypeptide, and includes cDNA and genomic DNA nucleic acids.

The term "isolated" for the purposes of the present invention designates a biological material (nucleic acid or protein) which has been removed from its original environment (the environment in which it is naturally present).

For example, a polynucleotide present in the natural state in a plant or an animal is not isolated. The same nucleotide separated from the adjacent nucleic acids in which it is naturally inserted in the genome of the plant or animal is considered as being "isolated".

Such a polynucleotide may be included in a vector and/or such a polynucleotide may be included in a composition and remains nevertheless in the isolated state because of the fact that the vector or the composition does not constitute its natural environment.

The term "purified" does not require the material to be present in a form exhibiting absolute purity, exclusive of the presence of other compounds. It is rather a relative definition.

A polynucleotide is in the "purified" state after purification of the starting material or of the natural material by at least one order of magnitude, preferably 2 or 3 and preferably 4 or 5 orders of magnitude.

For the purposes of the present description, the expression "nucleotide sequence" may be used to designate either a polynucleotide or a nucleic acid. The expression "nucleotide sequence" covers the genetic material itself and is therefore not restricted to the information relating to its sequence.

The terms "nucleic acid", "polynucleotide", "oligonucleotide" or "nucleotide sequence" cover RNA, DNA, or cDNA sequences or alternatively RNA/DNA hybrid sequences of more than one nucleotide, either in the single-stranded form or in the duplex, double-stranded form.

A "nucleic acid" is a polymeric compound comprised of covalently linked subunits called nucleotides. Nucleic acid includes polyribonucleic acid (RNA) and polydeoxyribonucleic acid (DNA), both of which may be single-stranded or double-stranded. DNA includes cDNA, genomic DNA, synthetic DNA, and semi-synthetic DNA. The sequence of nucleotides that encodes a protein is called the sense sequence or coding sequence.

The term "nucleotide" designates both the natural nucleotides (A, T, G, C) as well as the modified nucleotides that comprise at least one modification such as (1) an analog of a purine, (2) an analog of a pyrimidine, or (3) an analogous sugar, examples of such modified nucleotides being described, for example, in the PCT application No. WO 95/04 064.

For the purposes of the present invention, a first polynucleotide is considered as being "complementary" to a second polynucleotide when each base of the first nucleotide is paired with the complementary base of the second polynucleotide whose orientation is reversed. The complementary bases are A and T (or A and U), or C and G.

"Heterologous" DNA refers to DNA not naturally located in the cell, or in a chromosomal site of the cell. Preferably, the heterologous DNA includes a gene foreign to the cell.

As used herein, the term "homologous" in all its grammatical forms and spelling variations refers to the relationship between proteins that possess a "common evolutionary origin," including proteins from superfamilies (*e.g.*, the immunoglobulin superfamily) and homologous proteins from different species (*e.g.*, myosin light chain, etc.) (Reeck et al., 1987, Cell 50 :667)). Such proteins (and their encoding genes) have sequence homology, as reflected by their high degree of sequence similarity.

Accordingly, the term "sequence similarity" in all its grammatical forms refers to the degree of identity or correspondence between nucleic acid or amino acid sequences of proteins that may or may not share a common evolutionary origin (*see* Reeck et al., *supra*). However, in common usage and in the instant application, the term "homologous," when modified with an adverb such as "highly," may refer to sequence similarity and not a common evolutionary origin.

In a specific embodiment, two DNA sequences are "substantially homologous" or "substantially similar" when at least about 50% (preferably at least about 75%, and more preferably at least about 90 or 95%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, e.g., Maniatis et al., *supra*; Glover et al. (1985. DNA Cloning: A practical approach, volumes I and II oligonucleatide synthesis, MRL Press, Ltd, Oxford, U.K.); Hames and Higgins (1985. Transcription and Translation).

Similarly, in a particular embodiment, two amino acid sequences are "substantially homologous" or "substantially similar" when greater than 30% of the amino acids are identical, or greater than about 60% are similar (functionally identical). Preferably, the similar or homologous sequences are identified by alignment using, for example, the GCG (Genetics Computer Group, Program Manual for the GCG Package, *Version 7*, Madison, Wisconsin) pileup program.

The "percentage identity" between two nucleotide or amino acid sequences, for the purposes of the present invention, may be determined by comparing two sequences aligned optimally, through a window for comparison.

The portion of the nucleotide or polypeptide sequence in the window for comparison may thus comprise additions or deletions (for example "gaps") relative to the reference sequence (which does not comprise these additions or these deletions) so as to obtain an optimum alignment of the two sequences.

The percentage is calculated by determining the number of positions at which an identical nucleic base or an identical amino acid residue is observed for the two sequences (nucleic or peptide) compared, and then by dividing the number of positions at which there is identity between the two bases or amino acid residues by the total number of positions in the window for comparison, and then multiplying the result by 100 in order to obtain the percentage sequence identity.

The optimum sequence alignment for the comparison may be achieved using a computer with the aid of known algorithms contained in the package from the company WISCONSIN GENETICS SOFTWARE PACKAGE, GENETICS COMPUTER GROUP (GCG), 575 Science Doctor, Madison, WISCONSIN.

By way of illustration, it will be possible to produce the percentage sequence identity with the aid of the BLAST software (versions BLAST 1.4.9 of March 1996, BLAST 2.0.4 of February 1998 and BLAST 2.0.6 of September 1998), using exclusively the default parameters (Altschul et al, 1990, . Mol. Biol., 215:403-410; Altschul et al, 1997, Nucleic Acids Res., 25:3389-3402). Blast searches for sequences similar/homologous to a reference "request" sequence, with the aid of the Altschul et al. algorithm. The request sequence and the databases used may be of the peptide or nucleic types, any combination being possible.

The term "corresponding to" is used herein to refer to similar or homologous sequences, whether the exact position is identical or different from the molecule to which the similarity or homology is measured. A nucleic acid or amino acid sequence alignment may include spaces. Thus, the term "corresponding to" refers to the sequence similarity, and not the numbering of the amino acid residues or nucleotide bases.

A gene encoding any one of ABCA5, ABCA6, ABCA9 and ABCA10 polypeptides of the invention, whether genomic DNA or cDNA, can be isolated from any source, particularly from a human cDNA or genomic library. Methods for obtaining genes are well known in the art, as described above (*see, e.g.*, Sambrook et al., 1989, *Molecular cloning: a laboratory manual*. 2ed. Cold Spring Harbor Laboratory, Cold spring Harbor, New York).

Accordingly, any animal cell potentially can serve as the nucleic acid source for the molecular cloning of any one of ABCA5, ABCA6, ABCA9, and ABCA10 genes. The DNA may be obtained by standard procedures known in the art from cloned DNA (*e.g.*, a DNA "library"), and preferably is obtained from a cDNA library prepared from tissues with high level expression of the protein and/or the transcripts, by chemical synthesis, by cDNA cloning, or by the cloning of genomic DNA, or fragments thereof, purified from the desired cell (See, for example, Sambrook et al., 1989, *Molecular cloning: a laboratory manual.* 2ed. Cold Spring Harbor Laboratory, Cold spring Harbor, New York; Glover, 1985, *DNA Cloning: A Practical Approach, Volumes I and II Oligonucleotide Synthesis*, MRL Press, Ltd., Oxford, U.K).

Clones derived from genomic DNA may contain regulatory and intron DNA regions in addition to coding regions; clones derived from cDNA will not contain intron sequences. Whatever the source, the gene should be molecularly cloned into a suitable vector for propagation of the gene.

In the molecular cloning of the gene from genomic DNA, DNA fragments are generated, some of which will encode the desired gene. The DNA may be cleaved at specific sites using various restriction enzymes. Alternatively, one may use DNAse in the presence of manganese to fragment the DNA, or the DNA can be physically sheared, as for example, by sonication. The linear DNA fragments can then be separated according to size by standard techniques, including but not limited to, agarose and polyacrylamide gel electrophoresis and column chromatography.

Once the DNA fragments are generated, identification of the specific DNA fragment containing the desired ABCA5, ABCA, ABCA9, and ABCA10 genes may be accomplished in a number of ways. For example, if an amount of a portion of one of ABCA5, ABCA6, ABCA9, and ABCA10 genes or its specific RNA, or a fragment thereof, is available and can be purified and labelled, the generated DNA fragments may be screened by nucleic acid hybridization to the labelled probe (Benton and Davis, *Science* (1977), 196:180; Grunstein et al., *Proc.Natl. Acad. Sci. U.S.A.* (1975) 72:3961). For example, a set of oligonucleotides corresponding to the partial coding sequence information obtained for the ABCA5, ABCA6, ABCA9, ABCA10 proteins can be prepared and used as probes for DNA encoding any one of ABCA5, ABCA6, ABCA9 and ABCA10, as was done in a specific example, *infra*, or as primers for cDNA or mRNA (*e.g.*, in combination with a poly-T primer for RT-PCR). Preferably, a fragment is selected that is highly unique to one of the ABCA5, ABCA6, ABCA9, and ABCA10 nucleic acids or polypeptide of the invention. Those DNA fragments with substantial homology to the probe will hybridize. As noted above, the greater the degree of homology, the more stringent hybridization conditions can be used. In a specific embodiment, various stringency hybridization conditions are used to identify homologous ABCA5, ABCA6, ABCA9, and ABCA10 genes.

Further selection can be carried out on the basis of the properties of the gene, *e*.*g*., if the gene encodes a protein product having the isoelectric, electrophoretic, amino acid composition, or partial amino acid sequence of one of the ABCA5, ABCA6, ABCA9, and ABCA10 proteins as disclosed herein. Thus, the presence of the gene may be detected by assays based on the physical, chemical, or immunological properties of its expressed product. For example, cDNA clones, or DNA clones which hybrid-select the proper mRNAs, can be selected which produce a protein that, *e.g.*, has similar or identical electrophoretic migration, isoelectric focusing or non-equilibrium pH gel electrophoresis behaviour, proteolytic digestion maps, or antigenic properties as known for ABCA5, ABCA6, ABCA9, and ABCA10.

ABCA5, ABCA6, ABCA9, and ABCA10 genes of the invention may also be identified by mRNA selection, *i.e.*, by nucleic acid hybridization followed by *in vitro* translation. According to this procedure, nucleotide fragments are used to isolate complementary mRNAs by hybridization. Such DNA fragments may represent available, purified ABCA5, ABCA6, ABCA9, ABCA10 DNAs, or may be synthetic oligonucleotides designed from the partial coding sequence information. Immunoprecipitation analysis or functional assays (*e.g.*, tyrosine phosphatase activity) of the *in vitro* translation products of the products of the isolated mRNAs identifies the mRNA and, therefore, the complementary DNA fragments, that contain the desired sequences. In addition, specific mRNAs may be selected by adsorption of polysomes isolated from cells to immobilized antibodies specifically directed against any one of the ABCA5, ABCA6, ABCA9, and ABCA10 polypeptides of the invention.

Radiolabeled ABCA5, ABCA6, ABCA 9, and ABCA10 cDNAs can be synthesized using the selected mRNA (from the adsorbed polysomes) as a template. The radiolabeled mRNA or cDNA may then be used as a probe to identify homologous ABCA5, ABCA6, ABCA9, and ABCA10 DNA fragments from among other genomic DNA fragments.

"Variant" of a nucleic acid according to the invention will be understood to mean a nucleic acid which differs by one or more bases relative to the reference polynucleotide. A variant nucleic acid may be of natural origin, such as an allelic variant which exists naturally, or it may also be a nonnatural variant obtained, for example, by mutagenic techniques.

In general, the differences between the reference (generally, wild-type) nucleic acid and the variant nucleic acid are small such that the nucleotide sequences of the reference nucleic acid and of the variant nucleic acid are very similar and, in many regions, identical. The nucleotide modifications present in a variant nucleic acid may be silent, which means that they do not alter the amino acid sequences encoded by said variant nucleic acid.

However, the changes in nucleotides in a variant nucleic acid may also result in substitutions, additions or deletions in the polypeptide encoded by the variant nucleic acid in relation to the polypeptides encoded by the reference nucleic acid. In addition, nucleotide modifications in the coding regions may produce conservative or non-conservative substitutions in the amino acid sequence of the polypeptide.

Preferably, the variant nucleic acids according to the invention encode polypeptides which substantially conserve the same function or biological activity as the polypeptide of the reference nucleic acid or alternatively the capacity to be recognized by antibodies directed against the polypeptides encoded by the initial reference nucleic acid.

Some variant nucleic acids will thus encode mutated forms of the polypeptides whose systematic study will make it possible to deduce structure-activity relationships of the proteins in question. Knowledge of these variants in relation to the disease studied is essential since it makes it possible to understand the molecular cause of the pathology.

"Fragment" will be understood to mean a nucleotide sequence of reduced length relative to the reference nucleic acid and comprising, over the common portion, a nucleotide sequence identical to the reference nucleic acid. Such a nucleic acid "fragment" according to the invention may be, where appropriate, included in a larger polynucleotide of which it is a constituent. Such fragments comprise, or alternatively consist of, oligonucleotides ranging in length from 8, 10, 12, 15, 18, 20 to 25, 30, 40, 50, 70, 80, 100, 200, 500, 1000 or 1500 consecutive nucleotides of a nucleic acid according to the invention.

A "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester anologs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, *inter alia*, in linear or circular DNA molecules (*e.g.*, restriction fragments), plasmids, and chromosomes. In discussing the structure of particular double-stranded DNA molecules, sequences may be described herein according to the normal convention of giving only the sequence in the 5' to 3' direction along the nontranscribed strand of DNA (*i.e*., the strand having a sequence homologous to the mRNA). A "recombinant DNA molecule" is a DNA molecule that has undergone a molecular biological manipulation.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (*see* Sambrook et al., *supra*). The conditions of temperature and ionic strength determine the "stringency" of the hybridization. For preliminary screening for homologous nucleic acids, low stringency hybridization conditions, corresponding to a Tₘ of 55°, can be used, *e.g.*, 5x SSC, 0.1% SDS, 0.25% milk, and no formamide; or 30% formamide, 5x SSC, 0.5% SDS. Moderate stringency hybridization conditions correspond to a higher Tₘ, *e.g.,* 40% formamide, with 5x or 6x SCC. High stringency hybridization conditions correspond to the highest Tₘ, *e.g.,* 50% formamide, 5x or 6x SCC. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tₘ for hybrids of nucleic acids having those sequences. The relative stability (corresponding to higher Tₘ) of nucleic acid hybridizations decreases in the following order: RNA:RNA, DNA:RNA, DNA:DNA. For hybrids of greater than 100 nucleotides in length, equations for calculating Tₘ have been derived *(see* Sambrook et al., *supra).* For hybridization with shorter nucleic acids, *i.e.,* oligonucleotides, the position of mismatches becomes more important, and the length of the oligonucleotide determines its specificity *(see* Sambrook et al., *supra).* Preferably a minimum length for a hybridizable nucleic acid is at least about 10 nucleotides; preferably at least about 15 nucleotides; and more preferably the length is at least about 20 nucleotides.

In a specific embodiment, the term "standard hybridization conditions" refers to a Tₘ of 55°C, and utilizes conditions as set forth above. In a preferred embodiment, the Tₘ is 60°C; in a more preferred embodiment, the Tₘ is 65°C.

"High stringency hybridization conditions" for the purposes of the present invention will be understood to mean the following conditions:

### 1- Membrane competition and PREHYBRIDIZATION:

- Mix: 40 µl salmon sperm DNA (10 mg/ml)
   + 40 µl human placental DNA (10 mg/ml)
- Denature for 5 minutes at 96°C, then immerse the mixture in ice.
- Remove the 2X SSC and pour 4 ml of formamide mix in the hybridization tube containing the membranes.
- Add the mixture of the two denatured DNAs.
- Incubation at 42°C for 5 to 6 hours, with rotation.

### 2- Labeled probe competition:

- Add to the labeled and purified probe 10 to 50 µl Cot I DNA, depending on the quantity of repeats.
- Denature for 7 to 10 minutes at 95°C.
- Incubate at 65°C for 2 to 5 hours.

### 3- HYBRIDIZATION:

- Remove the prehybridization mix.
- Mix 40 µl salmon sperm DNA + 40 µl human placental DNA; denature for 5 min at 96°C, then immerse in ice.
- Add to the hybridization tube 4 ml of formamide mix, the mixture of the two DNAs and the denatured labeled probe/Cot I DNA .
- Incubate 15 to 20 hours at 42°C, with rotation.

### 4- Washes and Exposure:

- One wash at room temperature in 2X SSC, to rinse.
- Twice 5 minutes at room temperature 2X SSC and 0.1% SDS at 65°C.
- Twice 15 minutes 0.1X SSC and 0.1% SDS at 65°C.
- Envelope the membranes in clear plastic wrap and expose.

The hybridization conditions described above are adapted to hybridization, under high stringency conditions, of a molecule of nucleic acid of varying length from 20 nucleotides to several hundreds of nucleotides. It goes without saying that the hybridization conditions described above may be adjusted as a function of the length of the nucleic acid whose hybridization is sought or of the type of labeling chosen, according to techniques known to one skilled in the art. Suitable hybridization conditions may, for example, be adjusted according to the teaching contained in the manual by Hames and Higgins (1985, *supra*).

As used herein, the term "oligonucleotide" refers to a nucleic acid, generally of at least 15 nucleotides, that is hybridizable to a nucleic acid according to the invention. Oligonucleotides can be labelled, *e.g.*, with ³²P-nucleotides or nucleotides to which a label, such as biotin, has been covalently conjugated. In one embodiment, a labeled oligonucleotide can be used as a probe to detect the presence of a nucleic acid encoding an ABCA5-6, 9-10 polypeptide of the invention. In another embodiment, oligonucleotides (one or both of which may be labelled) can be used as PCR primers, either for cloning full lengths or fragments of any one of the ABCA5, ABCA6, ABCA9,and ABCA10 nucleic acids, or to detect the presence of nucleic acids encoding any one of the ABCA5, ABCA6, ABCA9, and ABCA10. In a further embodiment, an oligonucleotide of the invention can form a triple helix with any one of the ABCA5, ABCA6, ABCA9, and ABCA10 DNA molecules. Generally, oligonucleotides are prepared synthetically, preferably on a nucleic acid synthesizer. Accordingly, oligonucleotides can be prepared with non-naturally occurring phosphoester analog bonds, such as thioester bonds, etc.

"Homologous recombination" refers to the insertion of a foreign DNA sequence of a vector in a chromosome. Preferably, the vector targets a specific chromosomal site for homologous recombination. For specific homologous recombination, the vector will contain sufficiently long regions of homology to sequences of the chromosome to allow complementary binding and incorporation of the vector into the chromosome. Longer regions of homology, and greater degrees of sequence similarity, may increase the efficiency of homologous recombination.

A DNA "coding sequence" is a double-stranded DNA sequence which is transcribed and translated into a polypeptide in a cell *in vitro* or *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (*e.g.*, mammalian) DNA, and even synthetic DNA sequences. If the coding sequence is intended for expression in a eukaryotic cell, a polyadenylation signal and transcription termination sequence will usually be located 3' to the coding sequence.

Transcriptional and translational control sequences are DNA regulatory sequences, such as promoters, enhancers, terminators, and the like, that provide for the expression of a coding sequence in a host cell. In eukaryotic cells, polyadenylation signals are control sequences.

"Regulatory region" means a nucleic acid sequence which regulates the expression of a nucleic acid. A regulatory region may include sequences which are naturally responsible for expressing a particular nucleic acid (a homologous region) or may include sequences of a different origin (responsible for expressing different proteins or even synthetic proteins). In particular, the sequences can be sequences of eukaryotic or viral genes or derived sequences which stimulate or repress transcription of a gene in a specific or non-specific manner and in an inducible or non-inducible manner. Regulatory regions include origins of replication, RNA splice sites, enhancers, transcriptional termination sequences, signal sequences which direct the polypeptide into the secretory pathways of the target cell, and promoters.

A regulatory region from a "heterologous source" is a regulatory region which is not naturally associated with the expressed nucleic acid. Included among the heterologous regulatory regions are regulatory regions from a different species, regulatory regions from a different gene, hybrid regulatory sequences, and regulatory sequences which do not occur in nature, but which are designed by one having ordinary skill in the art.

A "cassette" refers to a segment of DNA that can be inserted into a vector at specific restriction sites. The segment of DNA encodes a polypeptide of interest, and the cassette and restriction sites are designed to ensure insertion of the cassette in the proper reading frame for transcription and translation.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase.

A coding sequence is "under the control" of transcriptional and translational control sequences in a cell when RNA polymerase transcribes the coding sequence into mRNA, which is then trans-RNA spliced and translated into the protein encoded by the coding sequence.

A "signal sequence" is included at the beginning of the coding sequence of a protein to be expressed on the surface of a cell. This sequence encodes a signal peptide, N-terminal to the mature polypeptide, that directs the host cell to translocate the polypeptide. The term "translocation signal sequence" is used herein to refer to this sort of signal sequence. Translocation signal sequences can be found associated with a variety of proteins native to eukaryotes and prokaryotes, and are often functional in both types of organisms.

A "polypeptide" is a polymeric compound comprised of covalently linked amino acid residues. Amino acids have the following general structure: 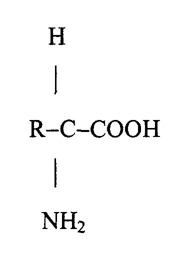 Amino acids are classified into seven groups on the basis of the side chain R: (1) aliphatic side chains, (2) side chains containing a hydroxylic (OH) group, (3) side chains containing sulfur atoms, (4) side chains containing an acidic or amide group, (5) side chains containing a basic group, (6) side chains containing an aromatic ring, and (7) proline, an imino acid in which the side chain is fused to the amino group.

A "protein" is a polypeptide which plays a structural or functional role in a living cell.

The polypeptides and proteins of the invention may be glycosylated or unglycosylated.

"Homology" means similarity of sequence reflecting a common evolutionary origin. Polypeptides or proteins are said to have homology, or similarity, if a substantial number of their amino acids are either (1) identical, or (2) have a chemically similar R side chain. Nucleic acids are said to have homology if a substantial number of their nucleotides are identical.

"Isolated polypeptide" or "isolated protein" is a polypeptide or protein which is substantially free of those compounds that are normally associated therewith in its natural state (e.g., other proteins or polypeptides, nucleic acids, carbohydrates, lipids). "Isolated" is not meant to exclude artificial or synthetic mixtures with other compounds, or the presence of impurities which do not interfere with biological activity, and which may be present, for example, due to incomplete purification, addition of stabilizers, or compounding into a pharmaceutically acceptable preparation. "Fragment" of a polypeptide according to the invention will be understood to mean a polypeptide whose amino acid sequence is shorter than that of the reference polypeptide and which comprises, over the entire portion with these reference polypeptides, an identical amino acid sequence. Such fragments may, where appropriate, be included in a larger polypeptide of which they are a part. Such fragments of a polypeptide according to the invention may have a length of 5, 10, 15, 20, 30 to 40, 50, 100, 200 or 300 amino acids.

"Variant" of a polypeptide according to the invention will be understood to mean mainly a polypeptide whose amino acid sequence contains one or more substitutions, additions or deletions of at least one amino acid residue, relative to the amino acid sequence of the reference polypeptide, it being understood that the amino acid substitutions may be either conservative or nonconservative.

A "variant" of a polypeptide or protein is any analogue, fragment, derivative, or mutant which is derived from a polypeptide or protein and which retains at least one biological property of the polypeptide or protein. Different variants of the polypeptide or protein may exist in nature. These variants may be allelic variations characterized by differences in the nucleotide sequences of the structural gene coding for the protein, or may involve differential splicing or post-translational modification. Variants also include a related protein having substantially the same biological activity, but obtained from a different species.

The skilled artisan can produce variants having single or multiple amino acid substitutions, deletions, additions, or replacements. These variants may include, *inter alia*: (a) variants in which one or more amino acid residues are substituted with conservative or non-conservative amino acids, (b) variants in which one or more amino acids are added to the polypeptide or protein, (c) variants in which one or more of the amino acids includes a substituent group, and (d) variants in which the polypeptide or protein is fused with another polypeptide such as serum albumin. The techniques for obtaining these variants, including genetic (suppressions, deletions, mutations, etc.), chemical, and enzymatic techniques, are known to persons having ordinary skill in the art.

If such allelic variations, analogues, fragments, derivatives, mutants, and modifications, including alternative mRNA splicing forms and alternative post-translational modification forms result in derivatives of the polypeptide which retain any of the biological properties of the polypeptide, they are intended to be included within the scope of this invention.

A "vector" is a replicon, such as plasmid, virus, phage or cosmid, to which another DNA segment may be attached so as to bring about the replication of the attached segment. A "replicon" is any genetic element (e.g., plasmid, chromosome, virus) that functions as an autonomous unit of DNA replication *in vivo, i.e.*, capable of replication under its own control.

The present invention also relates to cloning vectors containing genes encoding analogs and derivatives any of the ABCA5, ABCA6, ABCA9, and ABCA10 polypeptides of the invention, that have the same or homologous functional activity as that of ABCA5, ABCA6, ABCA9, ABCA10 polypeptides, and homologs thereof from other species. The production and use of derivatives and analogs related to ABCA5, ABCA6, ABCA9, and ABCA10 are within the scope of the present invention. In a specific embodiment, the derivatives or analogs are functionally active, *i.e*., capable of exhibiting one or more functional activities associated with a full-length, wild-type ABCA5, ABCA6, ABCA9, and ABCA10 polypeptides of the invention.

ABCA5, ABCA6, ABCA9, and ABCA10 derivatives can be made by altering encoding nucleic acid sequences by substitutions, additions or deletions that provide for functionally equivalent molecules. Preferably, derivatives are made that have enhanced or increased functional activity relative to native ABCA5, ABCA6, ABCA9, and ABCA10. Alternatively, such derivatives may encode soluble fragments of the ABCA5, ABCA6, ABCA9, and ABCA10 extracellular domains that have the same or greater affinity for the natural ligand of ABCA5, ABCA6, ABCA9, and ABCA10 polypeptides of the invention. Such soluble derivatives may be potent inhibitors of ligand binding to ABCA5, ABCA6, ABCA9, and ABCA10.

Due to the degeneracy of nucleotide coding sequences, other DNA sequences which encode substantially same amino acid sequences as that of ABCA5, ABCA6, ABCA9, and ABCA10 genes may be used in the practice of the present invention. These include but are not limited to allelic genes, homologous genes from other species, and nucleotide sequences comprising all or portions of ABCA5, ABCA6, ABCA9, and ABCA10 genes which are altered by the substitution of different codons that encode the same amino acid residue within the sequence, thus producing a silent change. Likewise, the ABCA5, ABCA6, ABCA9, and ABCA10 derivatives of the invention include, but are not limited to, those containing, as a primary amino acid sequence, all or part of the amino acid sequence of any one of the ABCA5, ABCA6, ABCA9, and ABCA10 proteins including altered sequences in which functionally equivalent amino acid residues are substituted for residues within the sequence resulting in a conservative amino acid substitution. For example, one or more amino acid residues within the sequence can be substituted by another amino acid of a similar polarity, which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. Amino acids containing aromatic ring structures are phenylalanine, tryptophan, and tyrosine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Such alterations will not be expected to affect apparent molecular weight as determined by polyacrylamide gel electrophoresis, or isoelectric point.

Particularly preferred substitutions are:
- Lys for Arg and vice versa such that a positive charge may be maintained;
- Glu for Asp and vice versa such that a negative charge may be maintained;
- Ser for Thr such that a free -OH can be maintained; and
- Gln for Asn such that a free CONH₂ can be maintained.

Amino acid substitutions may also be introduced to substitute an amino acid with a particularly preferable property. For example, a Cys may be introduced as a potential site for disulfide bridges with another Cys. A His may be introduced as a particularly "catalytic" site (i.e., His can act as an acid or base and is the most common amino acid in biochemical catalysis). Pro may be introduced because of its particularly planar structure, which induces b-turns in the protein's structure.

The genes encoding ABCA5, ABCA6, ABCA9, and ABCA10 derivatives and analogs of the invention can be produced by various methods known in the art. The manipulations which result in their production can occur at the gene or protein level. For example, the cloned ABCA5, ABCA6, ABCA9, and ABCA10 sequences can be modified by any of numerous strategies known in the art (Sambrook et al., 1989, *supra*). The sequence can be cleaved at appropriate sites with restriction endonuclease(s), followed by further enzymatic modification if desired, isolated, and ligated *in vitro*. Production of a gene encoding a derivative or analog of any one of the ABCA5, ABCA6, ABCA9, and ABCA10, should ensure that the modified gene remains within the same translational reading frame as the ABCA5, ABCA6, ABCA9, and ABCA10 genes, uninterrupted by translational stop signals, in the region where the desired activity is encoded.

Additionally, the ABCA5, ABCA6, ABCA9, and ABCA10-encoding nucleic acids can be mutated *in vitro* or *in vivo*, to create and/or destroy translation, initiation, and/or termination sequences, or to create variations in coding regions and/or form new restriction endonuclease sites or destroy pre-existing ones, to facilitate further *in vitro* modification. Preferably, such mutations enhance the functional activity of the mutated ABCA5, ABCA6, ABCA9, and ABCA10 genes products. Any technique for mutagenesis known in the art may be used, including *inter alia, in vitro* site-directed mutagenesis (Hutchinson et al., (1978) Biol. Chem. 253:6551; Zoller and Smith, (1984) DNA, 3:479-488; Oliphant et al., (1986) *Gene* 44:177; Hutchinson et al., (1986) *Proc. Natl. Acad. Sci. U.S.A.* 83:710; Huygen et al., (1996) Nature Medicine, 2(8):893-898) and use of TAB® linkers (Pharmacia). PCR techniques are preferred for site-directed mutagenesis (Higuchi, 1989, "Using PCR to Engineer DNA", in *PCR Technology: Principles and Applications for DNA Amplification,* H. Erlich, ed., Stockton Press, Chapter 6, pp. 61-70).

Identified and isolated ABCA5, ABCA6, ABCA9, and ABCA10 genes may then be inserted into an appropriate cloning vector. A large number of vector-host systems known in the art may be used. Possible vectors include, but are not limited to, plasmids or modified viruses, but the vector system must be compatible with the host cell used. Examples of vectors include, but are not limited to, *Escherichia coli*, bacteriophages such as lambda derivatives, or plasmids such as pBR322 derivatives or pUC plasmid derivatives, *e.g.*, pGEX vectors, pmal-c, pFLAG, etc. The insertion into a cloning vector can, for example, be accomplished by ligating the DNA fragment into a cloning vector which has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. Recombinant molecules can be introduced into host cells via transformation, transfection, infection, electroporation, etc., so that many copies of the gene sequence are generated. Preferably, the cloned gene is contained on a shuttle vector plasmid, which provides for expansion in a cloning cell, *e.g., Escherichia coli*, and facile purification for subsequent insertion into an appropriate expression cell line, if such is desired. For example, a shuttle vector, which is a vector that can replicate in more than one type of organism, can be prepared for replication in both *Escherichia coli* and *Saccharomyces cerevisiae* by linking sequences from an *Escherichia coli* plasmid with sequences form the yeast 2m plasmid.

In an alternative method, the desired gene may be identified and isolated after insertion into a suitable cloning vector in a "shot gun" approach. Enrichment for the desired gene, for example, by size fractionation, can be done before insertion into the cloning vector.

The nucleotide sequence coding for ABCA5, ABCA6, ABCA9, and ABCA10 polypeptides or antigenic fragments, derivatives or analogs thereof, or functionally active derivatives, including chimeric proteins thereof, may be inserted into an appropriate expression vector, *i.e.*, a vector which contains the necessary elements for the transcription and translation of the inserted protein-coding sequence. Such elements are termed herein a "promoter." Thus, nucleic acids encoding ABCA5, ABCA6, ABCA9, and ABCA10 polypeptides of the invention are operationally associated with a promoter in an expression vector of the invention. Both cDNA and genomic sequences can be cloned and expressed under control of such regulatory sequences. An expression vector also preferably includes a replication origin.

The necessary transcriptional and translational signals can be provided on a recombinant expression vector, or they may be supplied by a native gene encoding ABCA5, ABCA6, ABCA9, and ABCA10 and/or its flanking regions.

Potential host-vector systems include but are not limited to mammalian cell systems infected with virus (*e.g*., vaccinia virus, adenovirus, etc.); insect cell systems infected with virus (*e.g.*, baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

A recombinant ABCA5, ABCA6, ABCA9, and ABCA10 proteins of the invention, or functional fragments, derivatives, chimeric constructs, or analogs thereof, may be expressed chromosomally, after integration of the coding sequence by recombination. In this regard, any of a number of amplification systems may be used to achieve high levels of stable gene expression (*See* Sambrook et al., 1989, *supra*).

The cell into which the recombinant vector comprising the nucleic acid encoding any one of the ABCA5, ABCA6, ABCA9, and ABCA10 polypeptides according to the invention is cultured in an appropriate cell culture medium under conditions that provide for expression of any one of the ABCA5, ABCA6, ABCA9, and ABCA10 polypeptides by the cell.

Any of the methods previously described for the insertion of DNA fragments into a cloning vector may be used to construct expression vectors containing a gene consisting of appropriate transcriptional/translational control signals and the protein coding sequences. These methods may include *in vitro* recombinant DNA and synthetic techniques and *in vivo* recombination (genetic recombination).

Expression of ABCA5, ABCA6, ABCA9, and ABCA10 polypeptides may be controlled by any promoter/enhancer element known in the art, but these regulatory elements must be functional in the host selected for expression. Promoters which may be used to control ABCA5, ABCA6, ABCA9, and ABCA10 genes expression include, but are not limited to, the SV40 early promoter region (Benoist and Chambon, 1981 Nature 290:304-310), the promoter contained in the 3' long terminal repeat of Rous sarcoma virus (Yamamoto, et al., 1980 Cell 22:787-797), the herpes thymidine kinase promoter (Wagner et al., 1981 Proc. Natl. Acad. Sci. U.S.A. 78:1441-1445), the regulatory sequences of the metallothionein gene (Brinster et al., 1982 Nature 296:39-42); prokaryotic expression vectors such as the β-lactamase promoter (Villa-Kamaroff, et al., 1978 Proc. Natl. Acad. Sci. U.S.A. 75:3727-3731), or the *tac* promoter (DeBoer, et al., 1983 Proc. Natl. Acad. Sci. U.S.A. 80:21-25); see also "Useful proteins from recombinant bacteria" in Scientific American, 1980, 242:74-94; promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals: elastase I gene control region which is active in pancreatic acinar cells (Swift et al., 1984 Cell 38:639-646; Ornitz et al., 1986 Cold Spring Harbor Symp. Quant. Biol. 50:399-409; MacDonald, 1987); insulin gene control region which is active in pancreatic beta cells (Hanahan, 1985 Nature: 315:115-122), immunoglobulin gene control region which is active in lymphoid cells (Grosschedl et al., 1984 Cell 38:647-658; Adames et al., 1985 Nature 318:533-538; Alexander et al., 1987 Mol. Cell. Biol. 7:1436-1444), mouse mammary tumor virus control region which is active in testicular, breast, lymphoid and mast cells (Leder et al., 1986 Cell 45:485-495), albumin gene control region which is active in liver (Pinkert et al., 1987 Genes and Devel. 1:268-276), alpha-fetoprotein gene control region which is active in liver (Krumlauf et al., 1985 Mol. Cell. Biol. 5:1639-1648; Hammer et al., 1987 Science 235:53-58), alpha 1-antitrypsin gene control region which is active in the liver (Kelsey et al., 1987 Genes and Devel. 1:161-171) beta-globin gene control region which is active in myeloid cells (Mogram et al., 1985 Nature 315:338-340; Kollias et al., 1986 Cell 46:89-94), myelin basic protein gene control region which is active in oligodendrocyte cells in the brain (Readhead et al., 1987 Cell 48:703-712), myosin light chain-2 gene control region which is active in skeletal muscle (Sani, 1985 Nature 314:283-286), and gonadotropic releasing hormone gene control region which is active in the hypothalamus (Mason et al., 1986 Science 234:1372-1378).

Expression vectors containing a nucleic acid encoding one of ABCA5, ABCA6, ABCA9, and ABCA10 polypeptides of the invention can be identified by five general approaches: (a) polymerase chain reaction (PCR) amplification of the desired plasmid DNA or specific mRNA, (b) nucleic acid hybridization, (c) presence or absence of selection marker gene functions, (d) analyses with appropriate restriction endonucleases, and (e) expression of inserted sequences. In the first approach, the nucleic acids can be amplified by PCR to provide for detection of the amplified product. In the second approach, the presence of a foreign gene inserted in an expression vector can be detected by nucleic acid hybridization using probes comprising sequences that are homologous to an inserted marker gene. In the third approach, the recombinant vector/host system can be identified and selected based upon the presence or absence of certain "selection marker" gene functions (*e.g.*, b-galactosidase activity, thymidine kinase activity, resistance to antibiotics, transformation phenotype, occlusion body formation in baculovirus, etc.) caused by the insertion of foreign genes in the vector. In another example, if the nucleic acid encoding any one of the ABCA5, ABCA6, ABCA9, and ABCA10 polypeptides is inserted within the "selection marker" gene sequence of the vector, recombinants containing ABCA5, ABCA6, ABCA9, and ABCA10 nucleic acids inserts can be identified by the absence of the ABCA5, ABCA6, ABCA9, and ABCA10 genes functions. In the fourth approach, recombinant expression vectors are identified by digestion with appropriate restriction enzymes. In the fifth approach, recombinant expression vectors can be identified by assaying for the activity, biochemical, or immunological characteristics of the gene product expressed by the recombinant, provided that the expressed protein assumes a functionally active conformation.

A wide variety of host/expression vector combinations may be employed in expressing the nucleic acids of this invention. Useful expression vectors, for example, may consist of segments of chromosomal, non-chromosomal and synthetic DNA sequences. Suitable vectors include derivatives of SV40 and known bacterial plasmids, e.g., *Escherichia coli* plasmids col E1, pCRl, pBR322, pMal-C2, pET, pGEX (Smith *et al*., 1988, Gene 67:31-40), pMB9 and their derivatives, plasmids such as RP4; phage DNAs, e.g., the numerous derivatives of phage 1, e.g., NM989, and other phage DNA, e.g., M13 and filamentous single stranded phage DNA; yeast plasmids such as the 2m plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

For example, in a baculovirus expression systems, both non-fusion transfer vectors, such as but not limited to pVL941 (*Bam*H1 cloning site; Summers), pVL1393 (*Bam*H1, *Sma*I, *Xba*I, *Eco*R1, *Not*I, *Xma*III, *Bgl*II, and *Pst*I cloning site; Invitrogen), pVL1392 (*Bgl*II, *Pst*I, *Not*I, *Xma*III, *Eco*RI, *Xba*I, *Sma*I, and *Bam*H1 cloning site; Summers and Invitrogen), and pBlue*Bac*III (*Bam*H1, *Bgl*II, *Pst*I, *Nco*I, and *Hind*III cloning site, with blue/white recombinant screening possible; Invitrogen), and fusion transfer vectors, such as but not limited to pAc700 (*Bam*H1 and *Kpn*I cloning site, in which the *Bam*H1 recognition site begins with the initiation codon; Summers), pAc701 and pAc702 (same as pAc700, with different reading frames), pAc360 (*Bam*H1 cloning site 36 base pairs downstream of a polyhedrin initiation codon; Invitrogen(195)), and pBlueBacHisA, B, C (three different reading frames, with *Bam*H1, *Bgl*II, *Pst*I, *Nco*I, and *Hind*III cloning site, an N-terminal peptide for ProBond purification, and blue/white recombinant screening of plaques; Invitrogen (220) can be used.

Mammalian expression vectors contemplated for use in the invention include vectors with inducible promoters, such as the dihydrofolate reductase (DHFR) promoter, *e.g.,* any expression vector with a *DHFR* expression vector, or a *DHFR*/methotrexate co-amplification vector, such as pED (*Pst*I, *Sal*I, *Sba*I, *Sma*I, and *Eco*RI cloning site, with the vector expressing both the cloned gene and *DHFR*; See, Kaufman, *Current Protocols in Molecular Biology*, 16.12 (1991). Alternatively, a glutamine synthetase/methionine sulfoximine co-amplification vector, such as pEE14 (*Hind*III, *Xba*I, *Sma*I, *Sba*I, *Eco*RI, and *Bcl*I cloning site, in which the vector expresses glutamine synthase and the cloned gene; Celltech). In another embodiment, a vector that directs episomal expression under control of Epstein Barr Virus (EBV) can be used, such as pREP4 (*Bam*H1, *Sfi*I, *Xho*I, *Not*I, *Nhe*I, *Hind*III, *Nhe*I, *Pvu*II, and *Kpn*I cloning site, constitutive RSV-LTR promoter, hygromycin selectable marker; Invitrogen), pCEP4 (*Bam*H1, *Sfi*I, *Xho*I, *Not*I, *Nhe*I, *Hind*III, *Nhe*I, *Pvu*II, and *Kpn*I cloning site, constitutive hCMV immediate early gene, hygromycin selectable marker; Invitrogen), pMEP4 (*Kpn*I, *Pvu*I, *Nhe*I, *Hind*III, *Not*I, *Xho*I, *Sfi*I, *Bam*H1 cloning site, inducible methallothionein IIa gene promoter, hygromycin selectable marker: Invitrogen), pREP8 (*Bam*H1, *Xho*I, *Not*I, *Hind*III, *Nhe*I, and *Kpn*I cloning site, RSV-LTR promoter, histidinol selectable marker; Invitrogen), pREP9 (*Kpn*I, *Nhe*I, *Hind*III, *Not*I, *Xho*I, *Sfi*I, and BamHI cloning site, RSV-LTR promoter, G418 selectable marker; Invitrogen), and pEBVHis (RSV-LTR promoter, hygromycin selectable marker, N-terminal peptide purifiable via ProBond resin and cleaved by enterokinase; Invitrogen). Selectable mammalian expression vectors for use in the invention include pRc/CMV (*Hind*III, *Bst*XI, *Not*I, *Sba*I, and *Apa*I cloning site, G418 selection; Invitrogen), pRc/RSV (*Hind*III, *Spe*I, *Bst*XI, *Not*I, *Xba*I cloning site, G418 selection; Invitrogen), and others. Vaccinia virus mammalian expression vectors (*see*, Kaufman, 1991, *supra*) for use according to the invention include but are not limited to pSC11 (*Sma*I cloning site, TK- and b-gal selection), pMJ601 (*Sal*I, *Sma*I, *Afl*I, *Nar*I, *Bsp*MII, *Bam*HI, *Apa*I, *Nhe*I, *Sac*II, *Kpn*I, and *Hind*III cloning site; TK- and b-gal selection), and pTKgptF1S (*Eco*RI, *Pst*I, *Sal*I, *Acc*I, *Hind*II, *Sba*I, *Bam*HI, and Hpa cloning site, TK or XPRT selection).

Yeast expression systems can also be used according to the invention to express any one of the ABCA5, ABCA6, ABCA9, and ABCA10 polypeptides. For example, the non-fusion pYES2 vector (*Xba*I, *Sph*I, *Shot, Not*I, *Gst*XI, *Eco*RI, *Bst*XI, *Bam*H1, *Sac*I, *Kpn*1, and *Hind*III cloning sit; Invitrogen) or the fusion pYESHisA, B, C (*Xba*I, *Sph*I, *Shot, Not*I, *Bst*XI, *Eco*RI, *Bam*H1, *Sac*I, *Kpn*I, and *Hind*III cloning site, N-terminal peptide purified with ProBond resin and cleaved with enterokinase; Invitrogen), to mention just two, can be employed according to the invention.

Once a particular recombinant DNA molecule is identified and isolated, several methods known in the art may be used to propagate it. Once a suitable host system and growth conditions are established, recombinant expression vectors can be propagated and prepared in quantity. As previously explained, the expression vectors which can be used include, but are not limited to, the following vectors or their derivatives: human or animal viruses such as vaccinia virus or adenovirus; insect viruses such as baculovirus; yeast vectors; bacteriophage vectors (*e.g.*, lambda), and plasmid and cosmid DNA vectors, to name but a few.

In addition, a host cell strain may be chosen which modulates the expression of the inserted sequences, or modifies and processes the gene product in the specific fashion desired. Different host cells have characteristic and specific mechanisms for the translational and post-translational processing and modification (*e.g.*, glycosylation, cleavage for example of the signal sequence) of proteins. Appropriate cell lines or host systems can be chosen to ensure the desired modification and processing of the foreign protein expressed. For example, expression in a bacterial system can be used to produce an nonglycosylated core protein product. However, the transmembrane ABCA5, ABCA6, ABCA9, and ABCA10 proteins expressed in bacteria may not be properly folded. Expression in yeast can produce a glycosylated product. Expression in eukaryotic cells can increase the likelihood of "native" glycosylation and folding of a heterologous protein. Moreover, expression in mammalian cells can provide a tool for reconstituting, or constituting, ABCA5, ABCA6, ABCA9, and ABCA10 activities. Furthermore, different vector/host expression systems may affect processing reactions, such as proteolytic cleavages, to a different extent.

Vectors are introduced into the desired host cells by methods known in the art, *e.g.*, transfection, electroporation, microinjection, transduction, cell fusion, DEAE dextran, calcium phosphate precipitation, lipofection (lysosome fusion), use of a gene gun, or a DNA vector transporter (see, *e.g.*, Wu et al., 1992, J. Biol. Chem. 267:963-967; Wu and Wu, 1988, J. Biol. Chem. 263:14621-14624; Hartmut et al., Canadian Patent Application No. 2,012,311, filed March 15, 1990).

A cell has been "transfected" by exogenous or heterologous DNA when such DNA has been introduced inside the cell. A cell has been "transformed" by exogenous or heterologous DNA when the transfected DNA effects a phenotypic change. Preferably, the transforming DNA should be integrated (covalently linked) into chromosomal DNA making up the genome of the cell.

A recombinant marker protein expressed as an integral membrane protein can be isolated and purified by standard methods. Generally, the integral membrane protein can be obtained by lysing the membrane with detergents, such as but not limited to, sodium dodecyl sulfate (SDS), Triton X-100 polyoxyethylene ester, Ipagel/nonidet P-40 (NP-40) (octylphenoxy)-polyethoxyethanol, digoxin, sodium deoxycholate, and the like, including mixtures thereof. Solubilization can be enhanced by sonication of the suspension. Soluble forms of the protein can be obtained by collecting culture fluid, or solubilizing inclusion bodies, *e.g.*, by treatment with detergent, and if desired sonication or other mechanical processes, as described above. The solubilized or soluble protein can be isolated using various techniques, such as polyacrylamide gel electrophoresis (PAGE), isoelectric focusing, 2-dimensional gel electrophoresis, chromatography (*e.g.*, ion exchange, affinity, immunoaffinity, and sizing column chromatography), centrifugation, differential solubility, immunoprecipitation, or by any other standard technique for the purification of proteins.

Alternatively, a nucleic acid or vector according to the invention can be introduced *in vivo* by lipofection. For the past decade, there has been increasing use of liposomes for encapsulation and transfection of nucleic acids *in vitro*. Synthetic cationic lipids designed to limit the difficulties and dangers encountered with liposome mediated transfection can be used to prepare liposomes for *in vivo* transfection of a gene encoding a marker (Felgner, et. al. (1987. PNAS 84/7413); Mackey, et al. (1988. Proc. Natl. Acad. Sci. USA 85 :8027-8031); Ulmer et al. (1993. Science 259 :1745-1748). The use of cationic lipids may promote encapsulation of negatively charged nucleic acids, and also promote fusion with negatively charged cell membranes (Felgner and Ringold, (1989. Science 337:387-388)). Particularly useful lipid compounds and compositions for transfer of nucleic acids are described in International Patent Publications WO95/18863 and WO96/17823, and in U.S. Patent No. 5,459,127. The use of lipofection to introduce exogenous genes into the specific organs *in vivo* has certain practical advantages. Molecular targeting of liposomes to specific cells represents one area of benefit. It is clear that directing transfection to particular cell types would be particularly preferred in a tissue with cellular heterogeneity, such as pancreas, liver, kidney, and the brain. Lipids may be chemically coupled to other molecules for the purpose of targeting [*see* Mackey, et. al., *supra*]. Targeted peptides, *e.g.,* hormones or neurotransmitters, and proteins such as antibodies, or non-peptide molecules could be coupled to liposomes chemically.

Other molecules are also useful for facilitating transfection of a nucleic acid *in vivo*, such as a cationic oligopeptide (*e.g*., International Patent Publication WO95/21931), peptides derived from DNA binding proteins (*e.g*., International Patent Publication WO96/25508), or a cationic polymer (*e.g*., International Patent Publication WO95/21931).

It is also possible to introduce the vector *in vivo* as a naked DNA plasmid (see U.S. Patents 5,693,622, 5,589,466 and 5,580,859). Naked DNA vectors for gene therapy can be introduced into the desired host cells by methods known in the art, *e.g.*, transfection, electroporation, microinjection, transduction, cell fusion, DEAE dextran, calcium phosphate precipitation, use of a gene gun, or use of a DNA vector transporter (*see*, Wu et al., 1992, *supra*; Wu and Wu, 1988, *supra*; Hartmut et al., Canadian Patent Application No. 2,012,311, filed March 15, 1990; Williams et al., 1991, Proc. Natl. Acad. Sci. USA 88:2726-2730). Receptor-mediated DNA delivery approaches can also be used (Curiel et al., 1992, Hum. Gene Ther. 3:147-154; Wu and Wu, 1987, J. Biol. Chem. 262:4429-4432).

"Pharmaceutically acceptable vehicle or excipient " includes diluents and fillers which are pharmaceutically acceptable for method of administration, are sterile, and may be aqueous or oleaginous suspensions formulated using suitable dispersing or wetting agents and suspending agents. The particular pharmaceutically acceptable carrier and the ratio of active compound to carrier are determined by the solubility and chemical properties of the composition, the particular mode of administration, and standard pharmaceutical practice.

Any nucleic acid, polypeptide, vector, or host cell of the invention will preferably be introduced *in vivo* in a pharmaceutically acceptable vehicle or excipient. The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "excipient" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as excipients, particularly for injectable solutions. Suitable pharmaceutical excipients are described in "Remington's Pharmaceutical Sciences" by E.W. Martin.

Naturally, the invention contemplates delivery of a vector that will express a therapeutically effective amount of any one of ABCA5, ABCA6, ABCA9, and ABCA10 polypeptides for gene therapy applications. The phrase "therapeutically effective amount" is used herein to mean an amount sufficient to reduce by at least about 15 percent, preferably by at least 50 percent, more preferably by at least 90 percent, and still more preferably prevent, a clinically significant deficit in the activity, function and response of the host. Alternatively, a therapeutically effective amount is sufficient to cause an improvement in a clinically significant condition in the host.

"Lipid profile" means the set of concentrations of cholesterol, triglyceride, lipoprotein cholesterol and other lipids in the body of a human or other animal.

An "undesirable lipid profile" is the condition in which the concentrations of cholesterol, triglyceride, or lipoprotein cholesterol are outside of the age- and gender-adjusted reference ranges. Generally, a concentration of total cholesterol > 200 mg/dl, of plasma triglycerides > 200 mg/dl, of LDL cholesterol > 130 mg/dl, of HDL cholesterol < 39 mg/dl, or a ratio of total cholesterol to HDL cholesterol > 4.0 is considered to be an undesirable lipid profile. An undesirable lipid profile is associated with a variety of pathological conditions, including hyperlipidaemias, diabetes hypercholesterolaemia, arteriosclerosis, and other forms of coronary artery disease.

### NUCLEIC ACIDS OF THE ABCA5, A6, A9, A10 GENES

The applicants have identified a novel human ABCA-like cluster of genes, designated ABCA5, ABCA6, ABCA9, and ABCA10. The applicants have also determined that the new genes are closely spaced and arranged head-to-tail in the following order ABCA5, ABCA10, ABCA6, and ABCA9 on same region of chromosome 17q24, and encode putative full transporters (figure 1).

The applicants have also determined that each ABCA gene has a unique expression pattern, suggesting that the corresponding proteins may perform tissue-specialized functions (Example 3).

In effect, expression patterns showed that the 6.5 kb ABCA5 transcript is almost ubiquitous, but the strongest expression was found in testis, skeletal muscle, fetal kidney and fetal liver. The ABCA9 transcript of 6 kb was found in heart and a weak signal was detected in the ovary, small intestine and testis. The ABCA6 transcript of 7 kb-long was detected with a strong signal in the liver and fetal liver, a weaker signal was detected in heart, kidney (fetal and adult), lung (fetal and adult), colon, small intestine, ovary and testis. No signal was detected in the same conditions in the brain (fetal and adult), pancreas, placenta, skeletal muscle, leukocyte, pancreas, spleen, thymus. The ABCA10 transcript of 6.5 kb was strongly and specifically detected in skeletal muscle and heart (Figure 4).

The applicants have further determined potential transcript sequences that should correspond to the full coding sequence (CDS) of ABCA5, ABCA6, and ABCA9, and ABCA10, and that ABCA6, ABCA9, genes according to the invention comprise 39 exons and 38 introns, and ABCA10 gene according to the invention comprises at least 40 exons and 39 introns. Table 1 hereinafter presents predicted splice donors and acceptors scores (Rᵢ, bits) which are consistent with that of exons in other mammalian genes (Rogan et al., Hum Mutat (1998) 12, 153-171). Exons are located in exactly the same positions in all genes, although the length of some of the exons varies. Furthermore there is a high correlation coefficient (0.990-0.997) for exon size between these genes and significant correlations (0.27-0.64) for some of the comparisons of intron sizes and R; values, clearly suggesting that the genes from the 17q24 cluster arose by duplication from a common ancestor.

**Table 1:**

| **Correlations of exon size, intron size and Ri values.** | | | | |
|---|---|---|---|---|
| | Exons | Introns | Ri-SD | Ri-SA |
| ABCA6-ABCA9 | 0.997 | 0.62 | 0.45 | 0.28 |
| ABCA6-ABCA10 | 0.990 | 0.46 | 0.64 | 0.53 |
| ABCA9-ABCA10 | 0.995 | 0.43 | 0.47 | 0.46 |

The applicants have thus characterized new exonic sequences of human, ABCA6, ABCA9, and ABCA10 genes, which are particularly useful according to the invention for the production of various means of detection of corresponding, ABCA6, ABCA9, and ABCA10 genes, or nucleotide expression products in a sample.

Several exons of ABCA6 gene have been characterized by their nucleotide sequence and are identified in Table 2.

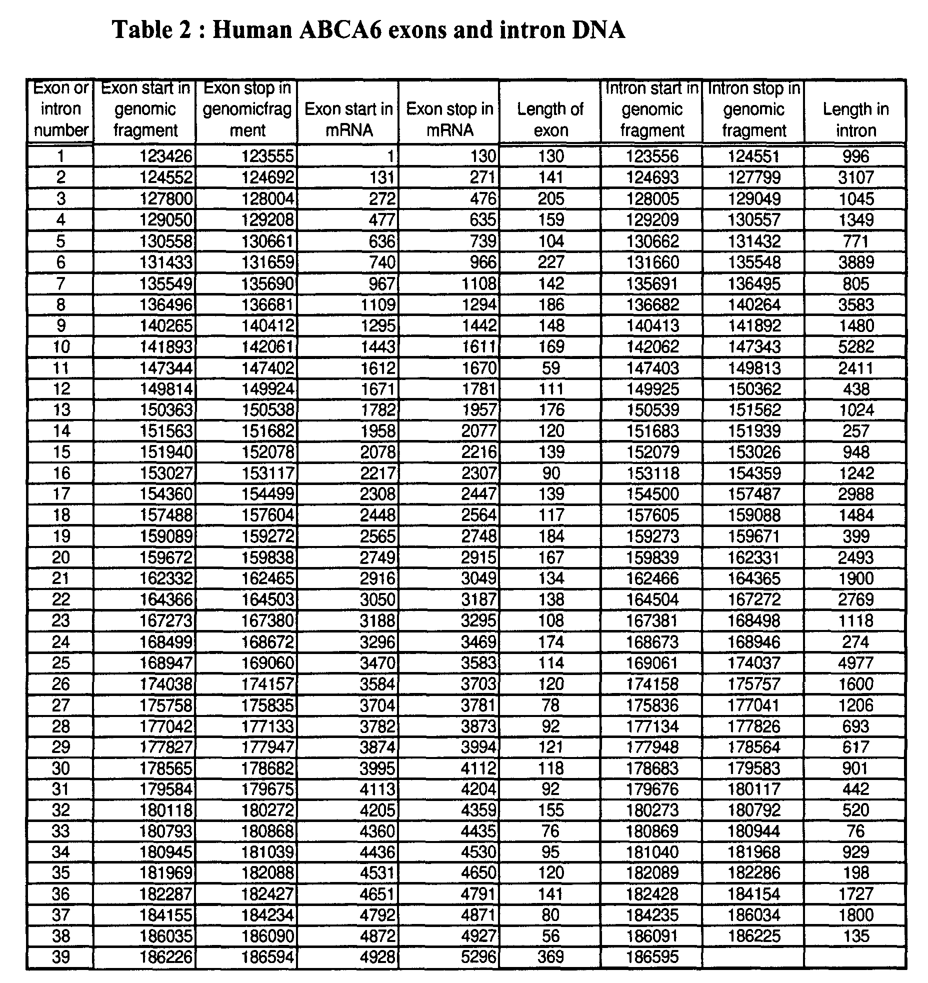

Thus the present invention also relates to a nucleic acid comprising any one of SEQ ID NOs: 9-47, or a complementary sequence.

The invention also relates to a nucleic acid comprising a nucleotide sequence as depicted in any one of SEQ ID NOs: 9-47, or a complementary nucleotide sequence.

The invention also relates to a nucleic acid comprising at least 8 consecutive nucleotides of any one of SEQ ID NOs: 9-47, or a complementary nucleotide sequence.

The subject of the invention is, in addition, a nucleic acid having at least 80% nucleotide identity with a nucleic acid comprising any one of SEQ ID NOs: 9-47, or a complementary nucleotide sequence.

The invention also relates to a nucleic acid having at least 85%, preferably 90%, more preferably 95% and still more preferably 98% nucleotide identity with a nucleic acid comprising any one of SEQ ID NOs: 9-47.

The invention also relates to a nucleic acid hybridizing, under high stringency conditions, with a nucleic acid comprising any one of SEQ ID NOs: 9-47, or a complementary nucleotide sequence.

Several exons of the ABCA9 gene have been characterized by their nucleotide sequence and are identified in Table 3.

**Table 3 :**

| **Human ABCA9 exons and intron DNA** | | | | | | | | |
|---|---|---|---|---|---|---|---|---|
| Exon or intron number | Exon start in genomic fragment | Exon stop in genomicfragment | Exon start in mRNA | Exon stop in mRNA | Length of exon | Intron start in genomic fragment | Intron stop in genomic fragment | Length in intron |
| 1 | 204305 | 204434 | 1 | 130 | 130 | 204435 | 214160 | 9726 |
| 2 | 214161 | 214269 | 131 | 239 | 109 | 214270 | 215809 | 1540 |
| 3 | 215810 | 216017 | 240 | 447 | 208 | 216018 | 219963 | 3946 |
| 4 | 219964 | 220128 | 448 | 612 | 165 | 220129 | 220699 | 571 |
| 5 | 220700 | 220803 | 613 | 716 | 104 | 220804 | 221584 | 781 |
| 6 | 221585 | 221811 | 717 | 943 | 227 | 221812 | 229498 | 7687 |
| 7 | 229499 | 229640 | 944 | 1085 | 142 | 229641 | 229868 | 228 |
| 8 | 229869 | 230054 | 1086 | 1271 | 186 | 230055 | 231426 | 1372 |
| 9 | 231427 | 231574 | 1272 | 1419 | 148 | 231575 | 233023 | 1449 |
| 10 | 233024 | 233192 | 1420 | 1588 | 169 | 233193 | 236072 | 2880 |
| 11 | 236073 | 236131 | 1589 | 1647 | 59 | 236132 | 236654 | 523 |
| 12 | 236655 | 236765 | 1648 | 1758 | 111 | 236766 | 237484 | 719 |
| 13 | 237485 | 237660 | 1759 | 1934 | 176 | 237661 | 237850 | 190 |
| 14 | 237851 | 237970 | 1935 | 2054 | 120 | 237971 | 238185 | 215 |
| 15 | 238186 | 238324 | 2055 | 2193 | 139 | 238325 | 238832 | 508 |
| 16 | 238833 | 238923 | 2194 | 2284 | 91 | 238924 | 240946 | 2023 |
| 17 | 240947 | 241086 | 2285 | 2424 | 140 | 241087 | 243438 | 2352 |
| 18 | 243439 | 243558 | 2425 | 2544 | 120 | 243559 | 244713 | 1155 |
| 19 | 244714 | 244912 | 2545 | 2743 | 199 | 244913 | 246720 | 1808 |
| 20 | 246721 | 246887 | 2744 | 2910 | 167 | 246888 | 247510 | 623 |
| 21 | 247511 | 247644 | 2911 | 3044 | 134 | 247645 | 248909 | 1265 |
| 22 | 248910 | 249047 | 3045 | 3182 | 138 | 249048 | 253216 | 4169 |
| 23 | 253217 | 253324 | 3183 | 3290 | 108 | 253325 | 257064 | 3740 |
| 24 | 257065 | 257238 | 3291 | 3464 | 174 | 257239 | 257427 | 189 |
| 25 | 257428 | 257541 | 3465 | 3578 | 114 | 257542 | 269285 | 11744 |
| 26 | 269286 | 269405 | 3579 | 3698 | 120 | 269406 | 272215 | 2810 |
| 27 | 272216 | 272284 | 3699 | 3767 | 69 | 272285 | 273033 | 749 |
| 28 | 273034 | 273125 | 3768 | 3859 | 92 | 273126 | 274342 | 1217 |
| 29 | 274343 | 274463 | 3860 | 3980 | 121 | 274464 | 275369 | 906 |
| 30 | 275370 | 275487 | 3981 | 4098 | 118 | 275488 | 276181 | 694 |
| 31 | 276182 | 276273 | 4099 | 4190 | 92 | 276274 | 278975 | 2702 |
| 32 | 278976 | 279136 | 4191 | 4351 | 161 | 279137 | 280171 | 1035 |
| 33 | 280172 | 280247 | 4352 | 4427 | 76 | 280248 | 280320 | 73 |
| 34 | 280321 | 280415 | 4428 | 4522 | 95 | 280416 | 281124 | 709 |
| 35 | 281125 | 281244 | 4523 | 4642 | 120 | 281245 | 281450 | 206 |
| 36 | 281451 | 281591 | 4643 | 4783 | 141 | 281592 | 282658 | 1067 |
| 37 | 282659 | 282738 | 4784 | 4863 | 80 | 282739 | 289109 | 6371 |
| 38 | 289110 | 289165 | 4864 | 4919 | 56 | 289166 | 289286 | 121 |
| 39 | 289287 | 290352 | 4920 | 5981 | 1062 | 290353 | | |

Thus the present invention also relates to a nucleic acid comprising any one of SEQ ID NO: 48-86, or a complementary sequence.

The invention also relates to a nucleic acid comprising a nucleotide sequence as depicted in any one of SEQ ID NOs: 48-86, or a complementary nucleotide sequence.

The invention also relates to a nucleic acid comprising at least 8 consecutive nucleotides of any one of SEQ ID NOs: 48-86, or a complementary nucleotide sequence.

The subject of the invention is, in addition, a nucleic acid having at least 80% nucleotide identity with a nucleic acid comprising any one of SEQ ID NOs: 48-86, or a complementary nucleotide sequence.

The invention also relates to a nucleic acid having at least 85%, preferably 90%, more preferably 95% and still more preferably 98% nucleotide identity with a nucleic acid comprising any one of SEQ ID NOs: 48-86.

The invention also relates to a nucleic acid hybridizing, under high stringency conditions, with a nucleic acid comprising any one of SEQ ID NOs: 48-86, or a complementary nucleotide sequence.

Several exons of the ABCA10 gene have been characterized by their nucleotide sequence and are identified in Table 4.

**Table 4 :**

| **Human ABCA10 exons and introns DNA** | | | | | | | | |
|---|---|---|---|---|---|---|---|---|
| Exon or intron number | Exon start in genomic fragment | Exon stop in genomicfragment | Exon start in mRNA | Exon stop in mRNA | Length of exon | Intron start in genomic fragment | Intron stop in genomic fragment | Length in intron |
| 1 | 20483 | 20769 | 1 | 287 | 287 | 20770 | 36437 | 15668 |
| 2 | 36438 | 36717 | 288 | 567 | 280 | 36718 | 38012 | 1295 |
| 3 | 38013 | 38153 | 568 | 708 | 141 | 38154 | 39768 | 1615 |
| 4 | 39769 | 39973 | 709 | 913 | 205 | 39974 | 42600 | 2627 |
| 5 | 42601 | 42765 | 914 | 1078 | 165 | 42766 | 43402 | 637 |
| 6 | 43403 | 43506 | 1079 | 1182 | 104 | 43507 | 45526 | 2020 |
| 7 | 45527 | 45753 | 1183 | 1409 | 227 | 45754 | 48939 | 3186 |
| 8 | 48940 | 49081 | 1410 | 1551 | 142 | 49082 | 49297 | 216 |
| 9 | 49298 | 49483 | 1552 | 1737 | 186 | 49484 | 50446 | 963 |
| 10 | 50447 | 50594 | 1738 | 1885 | 148 | 50595 | 63629 | 13035 |
| 11 | 63630 | 63798 | 1886 | 2054 | 169 | 63799 | 68175 | 4377 |
| 12 | 68176 | 68234 | 2055 | 2113 | 59 | 68235 | 70803 | 2569 |
| 13 | 70804 | 70914 | 2114 | 2224 | 111 | 70915 | 71309 | 395 |
| 14 | 71310 | 71485 | 2225 | 2400 | 176 | 71486 | 71686 | 201 |
| 15 | 71687 | 71806 | 2401 | 2520 | 120 | 71807 | 72050 | 244 |
| 16 | 72051 | 72189 | 2521 | 2659 | 139 | 72190 | 72645 | 456 |
| 17 | 72646 | 72736 | 2660 | 2750 | 91 | 72737 | 73983 | 1247 |
| 18 | 73984 | 74123 | 2751 | 2890 | 140 | 74124 | 74821 | 698 |
| 19 | 74822 | 74941 | 2891 | 3010 | 120 | 74942 | 77419 | 2478 |
| 20 | 77420 | 77618 | 3011 | 3209 | 199 | 77619 | 79655 | 2037 |
| 21 | 79656 | 79822 | 3210 | 3376 | 167 | 79823 | 82490 | 2668 |
| 22 | 82491 | 82624 | 3377 | 3510 | 134 | 82625 | 83008 | 384 |
| 23 | 83009 | 83146 | 3511 | 3648 | 138 | 83147 | 89785 | 6639 |
| 24 | 89786 | 89893 | 3649 | 3756 | 108 | 89894 | 90521 | 628 |
| 25 | 90522 | 90692 | 3757 | 3927 | 171 | 90693 | 90904 | 212 |
| 26 | 90905 | 91018 | 3928 | 4041 | 114 | 91019 | 100216 | 9198 |
| 27 | 100217 | 100336 | 4042 | 4161 | 120 | 100337 | 101145 | 809 |
| 28 | 101146 | 101226 | 4162 | 4242 | 81 | 101227 | 108376 | 7150 |
| 29 | 108377 | 108468 | 4243 | 4334 | 92 | 108469 | 109374 | 906 |
| 30 | 109375 | 109495 | 4335 | 4455 | 121 | 109496 | 110163 | 668 |
| 31 | 110164 | 110281 | 4456 | 4573 | 118 | 110282 | 110973 | 692 |
| 32 | 110974 | 111065 | 4574 | 4665 | 92 | 111066 | 111290 | 225 |
| 33 | 111291 | 111469 | 4666 | 4844 | 179 | 111470 | 111753 | 284 |
| 34 | 111754 | 111829 | 4845 | 4920 | 76 | 111830 | 111900 | 71 |
| 35 | 111901 | 111995 | 4921 | 5015 | 95 | 111996 | 112818 | 823 |
| 36 | 112819 | 112938 | 5016 | 5135 | 120 | 112939 | 113116 | 178 |
| 37 | 113117 | 113257 | 5136 | 5276 | 141 | 113258 | 115236 | 1979 |
| 38 | 115237 | 115316 | 5277 | 5356 | 80 | 115317 | 116211 | 895 |
| 39 | 116212 | 116267 | 5357 | 5412 | 56 | 116268 | 116374 | 107 |
| | 116375 | 117143 | 5413 | 6181 | 769 | 117144 | | |

Thus, the invention also relates to a nucleic acid comprising any one of SEQ ID NOs: 87-126, or a complementary nucleotide sequence thereof.

The invention also relates to a nucleic acid comprising a nucleotide sequence as depicted in any one of SEQ ID NOs: 87-126, or a complementary nucleotide sequence thereof.

The invention also relates to a nucleic acid comprising at least 8 consecutive nucleotides of any one of SEQ ID NOs: 87-126, or a complementary nucleotide sequence.

The subject of the invention is, in addition, a nucleic acid having at least 80% nucleotide identity with a nucleic acid comprising any one of SEQ ID NOs: 87-126, or a complementary nucleotide sequence.

The invention also relates to a nucleic acid having at least 85%, preferably 90%, more preferably 95% and still more preferably 98% nucleotide identity with a nucleic acid comprising a) any one of SEQ ID NOs: 87-126, or a complementary nucleotide sequence.

The invention also relates to a nucleic acid hybridizing, under high stringency conditions, with a nucleic acid comprising any one of SEQ ID NOs: 87-126, or a complementary nucleotide sequence.

### cDNA MOLECULES ENCODING A FULL LENGTH ABCA5, ABCA6, ABCA9, and ABCA10 PROTEINS

The applicants have further determined the cDNA sequences and the full coding sequences (CDS) corresponding to human ABCA5, A6, A9, and A10 genes, which belong to the same chromosome 17 cluster, and encode full length human corresponding proteins (Example 2).

Table 5 summarizes for each gene the mRNA length, the codingnucleotide sequence length as well as the predicted protein size.

**Table 5 :**

| **Characterization of the four ABCA on the chromosome 17 cluster** | | | | | |
|---|---|---|---|---|---|
| | **mRNA length (bp)** | **CDS (bp)** | **Polyadenylation site** | **Putative protein (AA)** | **Number of coding exons** |
| **ABCA5** | 6525 | 4929 | - | 1642 | Nd* |
| **ABCA6** | 5296 | 4854 | AATAAA (position 5284) | 1617 | 38 |
| **ABCA9** | 5959 | 4875 | - | 1624 | 38 |
| **ABCA10** | 6181 | 4632 | - | 1543 | 37 |
| Nd : not determined; the genomic sequence is in progress (working draft BAC). | | | | | |

The cDNA sequence ABCA5 consists of 6525 nucleotides and contains a 4929 nucleotide coding sequence corresponding to a 1642 amino acid (aa) ABCA5 polypeptide produced in subjects not affected by disorders associated with cholesterol reverse transport or inflammatory lipid mediators transport. The cDNA molecule of the novel human ABCA5 gene having the nucleotide sequence as set forth in SEQ ID NO: 1 comprises an open reading frame beginning from the nucleotide at position 1011 (base A of the ATG codon for initiation of translation) to the nucleotide at position 5939 (base A of the TGA stop codon).

According to the invention, the ABCA5 cDNA comprising SEQ ID NO: 1 encodes a full length ABCA5 polypeptide of 1642 amino acids comprising the amino acid sequence of SEQ ID NO: 5.

The cDNA molecule of the novel human ABCA6 gene having the nucleotide sequence as set forth in SEQ ID NO: 2 comprises an open reading frame beginning from the nucleotide at position 176 (base A of the ATG codon for initiation of translation) to the nucleotide at position 5029 (second base A of the TAA stop codon). A polyadenylation signal (having the sequence AATAAA) is present, starting from the nucleotide at position 5284 of the sequence SEQ ID NO: 2.

According to the invention, the ABCA6 cDNA comprising SEQ ID NO: 2 consists of 5296 nucleotides and contains a 4854 nucleotide coding sequence which encodes a full length ABCA6 polypeptide of 1617 amino acids comprising the amino acid sequence of SEQ ID NO: 6.

The cDNA molecule of the novel human ABCA9 gene having the nucleotide sequence as set forth in SEQ ID NO: 3 comprises a coding sequence beginning from the nucleotide at position 144 (base A of the ATG codon for initiation of translation) to the nucleotide at position 5018 (second base A of the TAA stop codon).

According to the invention, the ABCA9 cDNA comprising SEQ ID NO: 3 consists of 5959 nucleotides and contains a 4875 nucleotide coding sequence which encodes a full length ABCA9 polypeptide of 1624 amino acids comprising the amino acid sequence of SEQ ID NO: 7.

The cDNA molecule of the novel human ABCA10 gene having the nucleotide sequence as set forth in SEQ ID NO: 4 comprises a coding sequence beginning from the nucleotide at position 880 (base A of the ATG codon for initiation of translation) to the nucleotide at position 5511 (second base A of the TAA stop codon).

According to the invention, the ABCA10 cDNA comprising SEQ ID NO: 4 consists of 6181 nucleotides and contains a 4632 nucleotide coding sequence which encodes a full length ABCA10 polypeptide of 1543 amino acids comprising the amino acid sequence of SEQ ID NO: 8.

The present invention is thus directed to a nucleic acid comprising SEQ ID NOs: 1-4, or a complementary nucleotide sequence thereof.

The invention also relates to a nucleic acid comprising a nucleotide sequence as depicted in SEQ ID NO :1-4 or a complementary nucleotide sequence thereof.

The invention also relates to a nucleic acid comprising at least eight consecutive nucleotides of SEQ ID NO: 1-4 or a complementary nucleotide sequence thereof.

The subject of the invention is also a nucleic acid having at least 80% nucleotide identity with a nucleic acid comprising nucleotides of SEQ ID NO:1-4 or a nucleic acid having a complementary nucleotide sequence thereof.

The invention also relates to a nucleic acid having at least 85%, preferably 90%, more preferably 95% and still more preferably 98% nucleotide identity with a nucleic acid comprising a nucleotides of SEQ ID NO:1-4 or a nucleic acid having a complementary nucleotide sequence thereof.

Another subject of the invention is a nucleic acid hybridizing, under high stringency conditions, with a nucleic acid comprising nucleotides of SEQ ID NO: 1-4, or a nucleic acid having a complementary nucleotide sequence thereof.

The invention also relates to a nucleic acid encoding a polypeptide comprising an amino acid sequence of SEQ ID NO: 5-8.

The invention relates to a nucleic acid encoding a polypeptide comprising an amino acid sequence as depicted in SEQ ID NO:5-8.

The invention also relates to a polypeptide comprising amino acid sequence of SEQ ID NO: 5-8.

The invention also relates to a polypeptide comprising amino acid sequence as depicted in SEQ ID NO: 5-8.

The invention also relates to a polypeptide comprising an amino acid sequence having at least 80% amino acid identity with a polypeptide comprising an amino acid sequence of SEQ ID NO: 5-8, or a peptide fragment thereof.

The invention also relates to a polypeptide having at least 85%, preferably 90%, more preferably 95% and still more preferably 98% amino acid identity with a polypeptide comprising an amino acid sequence of SEQ ID NO: 5-8.

Preferably, a polypeptide according to the invention will have a length of 4, 5 to 10, 15, 18 or 20 to 25, 35, 40, 50, 70, 80, 100 or 200 consecutive amino acids of a polypeptide according to the invention comprising an amino acid sequence of SEQ ID NO: 5-8.

Like ABCA1 and ABCA4 transporters, which present 45 to 66 % amino acid sequences identity, ABCA5, ABCA6, ABCA9, and ABCA10 proteins also demonstrate high conservation as set forth in Tables 6-10 (figure 2). Alignment of the amino acid sequences of ABCA5, ABCA6, aBCA9 and ABCA10 genes reveals an identity ranging from 43 to 62% along the entire sequence (Table 6). Particularly, ABCA5, ABCA6, ABCA9, and ABCA10 proteins show 32 to 60% and 34 to 48% identity in the N-terminal (Table 7) and C-terminal (Table 8) trans-membrane domains (TMC and TMN) respectively, and 56 to 77% identity in the ATP-binding domains (NBD1 and NBD2; Tables 9 and 10).

**Table 6:**

| **Homology / Identity percentages between the amino acid sequences of ABCA5, ABCA6, ABCA8, ABCA9, ABCA10, and ABCA1 along the entire sequence** | | | | | | |
|---|---|---|---|---|---|---|
| **Total sequence** | **ABCA5** | **ABCA6** | **ABCA8** | **ABCA9** | **ABCA10** | **ABCA1** |
| **ABCA5** | 100 / 100 | | | | | |
| **ABCA6** | 52.9/42.8 | 100/100 | | | | |
| **ABCA8** | 52.4/42.4 | 67/59.7 | 100/100 | | | |
| **ABCA9** | 52.6/42.7 | 67.4/59.4 | 78.2/71.6 | 100/100 | | |
| **ABCA10** | 53.2/43.4 | 69.5/62.3 | 68.1/61.1 | 70.3/62.1 | 100/100 | |
| **ABCA1** | 41.5/30.8 | 42.8/31 | 42.832 | 41.1/30.9 | 41.2/30.6 | 100/100 |

**Table 7:**

| **Homology / Identity percentages between the amino acid sequences of ABCA5, ABCA6, ABCA9, ABCA10, ABCA1, and ABCA8 in the N terminal transmembrane domain** | | | | | | |
|---|---|---|---|---|---|---|
| **TMN domain** | **ABCA5** | **ABCA6** | **ABCA8** | **ABCA9** | **ABCA10** | **ABCA1** |
| **ABCA5** | 100/100 | | | | | |
| **ABCA6** | 47/34.2 | 100/100 | | | | |
| **ABCA8** | 46.5/35 | 70.2/59.1 | 100/100 | | | |
| **ABCA9** | 46.3/37.8 | 64.2/55.7 | 76.9/68.5 | 100/100 | | |
| **ABCA10** | 43.4/32.3 | 68.5/60.4 | 70.7/60.8 | 65.5/57.9 | 100/100 | |
| **ABCA1** | 36.5/23.1 | 34.2/20 | 39.8/27.6 | 40.6/27.9 | 35.4/24.4 | 100/100 |

**Table 8:**

| **Homology / Identity percentages between the amino acid sequences of ABCA5, ABCA6, ABCA9, ABCA10, ABCA1 and ABCA8 in the C terminal transmembrane domain** | | | | | | |
|---|---|---|---|---|---|---|
| **TMC domain** | **ABCA5** | **ABCA6** | **ABCA8** | **ABCA9** | **ABCA10** | **ABCA1** |
| **ABCA5** | 100/100 | | | | | |
| **ABCA6** | 43.7/33.7 | 100/100 | | | | |
| **ABCA8** | 48.2/31.8 | 53.8/44.2 | 100/100 | | | |
| **ABCA9** | 47.3/33.7 | 57.2/48.2 | 64.1/52.9 | 100/100 | | |
| **ABCA10** | 47/35.4 | 57/47 | 54.3/43 | 57.4/44.4 | 100/100 | |
| **ABCA1** | 33/21.6 | 32/21.4 | 39/24.8 | 35.3/26.8 | 34.7/22.4 | 100/100 |

**Table 9:**

| **Homology / Identity percentages between the amino acid sequences of ABCA5, ABCA6, ABCA9, ABCA10, ABCA1, and ABCA8 in the nucleotide Binding Domain 1 (NBD1)** | | | | | | |
|---|---|---|---|---|---|---|
| **NBD1 domain** | **ABCA5** | **ABCA6** | **ABCA8** | **ABCA9** | **ABCA10** | **ABCA1** |
| **ABCA5** | 100/100 | | | | | |
| **ABCA6** | 70.2/60.3 | 100/100 | | | | |
| **ABCA8** | 71.8/62.5 | 85.4/78.6 | 100/100 | | | |
| **ABCA9** | 65.5/58,2 | 80.2/72.8 | 88.5/81.8 | 100/100 | | |
| **ABCA10** | 69.8/62.1 | 83.2/77,2 | 82.8/79.2 | 81.9/75.8 | 100/100 | |
| **ABCA1** | 56.8/48.5 | 53.5/43.5 | 61.2/50.5 | 51.7/43.8 | 56.5/45.6 | 100/100 |

**Table 10 :**

| **Homology / Identity percentages between the amino acid sequences of ABCA5, ABCA6, ABCA9, ABCA10, ABCA1, and ABCA8 in the nucleotide Binding Domain 2 (NBD2)** | | | | | | |
|---|---|---|---|---|---|---|
| **NBD2 domain** | **ABCA5** | **ABCA6** | **ABCA8** | **ABCA9** | **ABCA10** | **ABCA1** |
| **ABCA5** | 100/100 | | | | | |
| **ABCA6** | 63/56.1 | 100/100 | | | | |
| **ABCA8** | 66.9/58.4 | 78/73.5 | 100/100 | | | |
| **ABCA9** | 65.2/57.0 | 77.6/72.6 | 94.5/91.8 | 100/100 | | |
| **ABCA10** | 63.7/56.2 | 74.9/71.2 | 81/77.4 | 82.3/77.8 | 100/100 | |
| **ABCA1** | 46.4/37.8 | 46.3/37.9 | 46.9/38 | 47.3/39 | 46.4/37.7 | 100/100 |

Phylogenetic analysis of the ATP-binding domains demonstrate that the N- and C-terminal domains form separate branches (figure 3). The C-terminal ATP-binding domains of the 17q24 genes are more closely related to the C-terminal domains of the other ABC1-like genes than to the N-terminal domains of the same proteins. Thus the entire ABC1 subfamily appears to have arisen from a single ancestral full transporter gene. However, the genes in the 17q24 cluster form a distinct group within the ABC1 subfamily.

### NUCLEOTIDE PROBES AND PRIMERS

Nucleotide probes and primers hybridizing with a nucleic acid (genomic DNA, messenger RNA, cDNA) according to the invention also form part of the invention.

According to the invention, nucleic acid fragments derived from a polynucleotide comprising any one of SEQ ID NOs: 1-4 and 9-126 or of a complementary nucleotide sequence are useful for the detection of the presence of at least one copy of a nucleotide sequence of any one of the ABCA5, ABCA6, ABCA9, and ABCA10 genes or of a fragment or of a variant (containing a mutation or a polymorphism) thereof in a sample.

The nucleotide probes or primers according to the invention comprise a nucleotide sequence comprising any one of SEQ ID NOs: 1-4 and 9-126, or a complementary nucleotide sequence.

The nucleotide probes or primers according to the invention comprise at least 8 consecutive nucleotides of a nucleic acid comprising any one of SEQ ID NOs: 1-4 and 9-126, or a complementary nucleotide sequence.

Preferably, nucleotide probes or primers according to the invention have a length of 10, 12, 15, 18 or 20 to 25, 35, 40, 50, 70, 80, 100, 200, 500, 1000, 1500 consecutive nucleotides of a nucleic acid according to the invention, in particular of a nucleic acid comprising any one of SEQ ID NOs: 1-4 and 9-126, or a complementary nucleotide sequence.

Alternatively, a nucleotide probe or primer according to the invention consists of and/or comprise the fragments having a length of 12, 15, 18, 20, 25, 35, 40, 50, 100, 200, 500, 1000, 1500 consecutive nucleotides of a nucleic acid according to the invention, more particularly of a nucleic acid comprising any one of SEQ IDNOs: 1-4 and 9-126, or a complementary nucleotide sequence.

The definition of a nucleotide probe or primer according to the invention therefore covers oligonucleotides which hybridize, under the high stringency hybridization conditions defined above, with a nucleic acid comprising any one of SEQ ID NOs: 1-4 and 9-126, or a complementary nucleotide sequence.

According to a preferred embodiment, a nucleotide primer according to the invention comprises a nucleotide sequence of any one of SEQ ID NOs: 127-144, or a complementary nucleic acid sequence.

Examples of primers and pairs of primers which make it possible to amplify various regions of the ABCA5 gene are presented in Table 11 below. The location of each primer of SEQ ID NOs: 127-144 within SEQ ID NOs: 1, and its hybridizing region is indicated in Table 11. The abbreviation "Comp" refers to the complementary nucleic acid sequence.

**Table 11:**

| **Primers for the amplification of nucleic fragments of the ABCA5 gene** | | |
|---|---|---|
| Primer SEQ ID NO: | Located in SEQ ID NO: | Position in the sequence |
| 127 | 1 | 3842-3860 |
| 128 | 1 | Comp 4858-4876 |
| 129 | 1 | Comp 4783-4801 |
| 130 | 1 | Comp 5789-5807 |
| 131 | 1 | 5630-5648 |
| 132 | 1 | 4858-4876 |
| 133 | 1 | Comp 3998-4016 |
| 134 | 1 | Comp 2987-3005 |
| 135 | 1 | Comp 3186-3208 |
| 136 | 1 | 2528-2547 |
| 137 | 1 | Comp 3088-3107 |
| 138 | 1 | Comp 2528-2547 |
| 139 | 1 | Comp 845-862 |
| 140 | 1 | 789-807 |
| 141 | 1 | Comp 1614-1633 |
| 142 | 1 | 1614-1633 |
| 143 | 1 | Comp 537-566 |
| 144 | 1 | Comp 202-231 |

According to a preferred embodiment, a nucleotide primer according to the invention comprises a nucleotide sequence of any one of SEQ ID NOs: 145-172, or a complementary nucleic acid sequence.

Examples of primers and pairs of primers which make it possible to amplify various regions of the ABCA6 gene are presented in Table 12 below. The location of each primer of SEQ ID NOs: 145-172 within SEQ ID NOs: 2, and its hybridizing region is indicated in Table 12. The abbreviation "Comp" refers to the complementary nucleic acid sequence.

**Table 12:**

| **Primers for the amplification of nucleic fragments of the ABCA6 gene** | | | |
|---|---|---|---|
| Primer SEQ ID NO: | Located in SEQ ID NO: | Position in the sequence | Region for hybridization |
| 145 | 2 | 202-221 | Exon 2 |
| 146 | 2 | Comp 435-461 | Exon 3 |
| 147 | 2 | Comp 645-672 | Exon 5 |
| 148 | 2 | 637-656 | Exon 5 |
| 149 | 2 | 754-772 | Exon 6 |
| 150 | 2 | Comp 758-778 | Exon 6 |
| 151 | 2 | Comp 773-792 | Exon 6 |
| 152 | 2 | 1288-1307 | Exon 8-9 |
| 153 | 2 | 1321-1341 | Exon 9 |
| 154 | 2 | Comp 1322-1343 | Exon 9 |
| 155 | 2 | Comp 1592-1574 | Exon 10 |
| 156 | 2 | 1761-1782 | Exon 12 |
| 157 | 2 | Comp 1928-1949 | Exon 13 |
| 158 | 2 | 1944-1968 | Exon 13-14 |
| 159 | 2 | Comp 2041-2061 | Exon 14 |
| 160 | 2 | Comp 2371-2392 | Exon 17 |
| 161 | 2 | 2350-2371 | Exon 17 |
| 162 | 2 | 2806-2884 | Exon 20 |
| 163 | 2 | Comp 2884-2902 | Exon 20 |
| 164 | 2 | 3292-3313 | Exon 23-24 |
| 165 | 2 | Comp 3357-3339 | Exon24 |
| 166 | 2 | Comp 3746-3767 | Exon 27 |
| 167 | 2 | 3754-3775 | Exon 27 |
| 168 | 2 | 4176-4194 | Exon 31 |
| 169 | 2 | Comp 4248-4194 | Exon 31-32 |
| 170 | 2 | 4743-4763 | Exon 36 |
| 171 | 2 | Comp 4796-4778 | Exon 36-37 |
| 172 | 2 | Comp 5262-5244 | Exon 39 |

According to a preferred embodiment, a nucleotide primer according to the invention comprises a nucleotide sequence of any one of SEQ ID NOs: 173-203, or a complementary nucleic acid sequence.

Examples of primers and pairs of primers which make it possible to amplify various regions of the ABCA9 gene are presented in Table 13 below. The location of each primer of SEQ ID NOs: 173-203 within SEQ ID NOs: 3, and its hybridizing region is indicated in Table 13. The abbreviation "Comp" refers to the complementary nucleic acid sequence.

**Table 13:**

| **Primers for the amplification of nucleic fragments of the ABCA9 gene** | | | |
|---|---|---|---|
| Primer SEQ ID NO: | Located in SEQ ID NO: | Position in the sequence | Region for hybridization |
| 173 | 3 | 160-178 | Exon 2 |
| 174 | 3 | Comp 789-808 | Exon 6 |
| 175 | 3 | 786-804 | Exon 6 |
| 176 | 3 | Comp 1434-1455 | Exon 10 |
| 177 | 3 | 1305-1323 | Exon 9 |
| 178 | 3 | Comp 1632-1653 | Exon 11-12 |
| 179 | 3 | 1495-1516 | Exon 10 |
| 180 | 3 | 1866-1887 | Exon 13 |
| 181 | 3 | Comp 1905-1923 | Exon 13 |
| 182 | 3 | Comp 2349-2368 | Exon 17 |
| 183 | 3 | 2253-2272 | Exon 17 |
| 184 | 3 | Comp 2822-2843 | Exon 20 |
| 185 | 3 | 2645-2663 | Exon 19 |
| 186 | 3 | Comp 3089-3110 | Exon 22 |
| 187 | 3 | 3240-3260 | Exon 23 |
| 188 | 3 | 3023-3044 | Exon 21 |
| 189 | 3 | Comp 3801-3820 | Exon 28 |
| 190 | 3 | Comp 3377-3398 | Exon 24 |
| 191 | 3 | 3626-3646 | Exon 26 |
| 192 | 3 | Comp 4191-4209 | Exon 32 |
| 193 | 3 | 3964-3984 | Exon 29-30 |
| 194 | 3 | Comp 4784-4803 | Exon 37 |
| 195 | 3 | 5230-5247 | Exon 39 |
| 196 | 3 | 4694-4715 | Exon 36 |
| 197 | 3 | Comp 4977-4994 | Exon 39 |
| 198 | 3 | 5541-5561 | Exon 39 |
| 199 | 3 | Comp 5960-5981 | Exon 39 |
| 200 | 3 | Comp5541-5562 | Exon 39 |
| 201 | 3 | 24-45 | Exon 1 |
| 202 | 3 | Comp 384-408 | Exon 3 |
| 203 | 3 | Comp 311-337 | Exon 3 |

According to a preferred embodiment, a nucleotide primer according to the invention comprises a nucleotide sequence of any one of SEQ ID NOs: 204-217, or a complementary nucleic acid sequence thereof.

Examples of primers and pairs of primers which make it possible to amplify various regions of the ABCA10 gene are presented in Table 14 below. The location of each primer of SEQ ID NOs: 204-217 within SEQ ID NOs: 4, and its hybridizing region is indicated in Table 14. The abbreviation "Comp" refers to the complementary nucleic acid sequence.

**Table 14:**

| **Primers for the amplification of nucleic fragments of the ABCA10 gene** | | | |
|---|---|---|---|
| Primer SEQ ID NO: | Located in SEQ ID NO: | Position in the sequence | Region for hybridization |
| 204 | 4 | 1421-1440 | Exon 8 |
| 205 | 4 | Comp 1610-1629 | Exon 9 |
| 206 | 4 | 2417-2434 | Exon 15 |
| 207 | 4 | Comp 2605-2623 | Exon 16 |
| 208 | 4 | Comp 3737-3754 | Exon 24 |
| 209 | 4 | Comp 814-839 | Exon 4 |
| 210 | 4 | Comp 733-757 | Exon 4 |
| 211 | 4 | 61-86 | Exon 1 |
| 212 | 4 | 628-643 | Exon 3 |
| 213 | 4 | 3564-3583 | Exon 23 |
| 214 | 4 | Comp 4450-4468 | Exon 30-31 |
| 215 | 4 | Comp 5442-5459 | Exon 40 |
| 216 | 4 | 3050-3070 | Exon 20 |
| 217 | 4 | Comp 4848-4866 | Exon 34 |

According to a specific embodiment of preferred probes and primers according to the invention, they comprise all or part of a nucleotide sequence comprising any one of SEQ ID NOs: 127-217, or a nucleic acid having a complementary nucleic acid sequence.

A nucleotide primer or probe according to the invention may be prepared by any suitable method well known to persons skilled in the art, including by cloning and action of restriction enzymes or by direct chemical synthesis according to techniques such as the phosphodiester method by Narang et al. (1979, *Methods Enzymol, 68:90-98*) or by Brown et al. (1979, *Methods Enzymol, 68:109-151*), the diethylphosphoramidite method by Beaucage et al. (1981, Tetrahedron Lett, 22: 1859-1862) or the technique on a solid support described in EU patent No. EP 0,707,592.

Each of the nucleic acids according to the invention, including the oligonucleotide probes and primers described above, may be labeled, if desired, by incorporating a marker which can be detected by spectroscopic, photochemical, biochemical, immunochemical or chemical means. For example, such markers may consist of radioactive isotopes (³²P, ³³P, ³H, ³⁵S), fluorescent molecules (5-bromodeoxyuridine, fluorescein, acetylaminofluorene, digoxigenin) or ligands such as biotin. The labeling of the probes is preferably carried out by incorporating labeled molecules into the polynucleotides by primer extension, or alternatively by addition to the 5' or 3' ends. Examples of nonradioactive labeling of nucleic acid fragments are described in particular in French patent No. 78 109 75 or in the articles by Urdea et al. (1988, Nucleic Acids Research, 11:4937-4957) or Sanchezpescador et al. (1988, J. Clin. Microbiol., 26(10):1934-1938).

Preferably, the nucleotide probes and primers according to the invention may have structural characteristics of the type to allow amplification of the signal, such as the probes described by Urdea et al. (1991, Nucleic Acids Symp Ser., 24:197-200) or alternatively in European patent No. EP-0,225,807 (CHIRON).

The oligonucleotide probes according to the invention may be used in particular in Southern-type hybridizations with the genomic DNA or alternatively in northern-type hybridizations with the corresponding messenger RNA when the expression of the corresponding transcript is sought in a sample.

The probes and primers according to the invention may also be used for the detection of products of PCR amplification or alternatively for the detection of mismatches.

Nucleotide probes or primers according to the invention may be immobilized on a solid support. Such solid supports are well known to persons skilled in the art and comprise surfaces of wells of microtiter plates, polystyrene beads, magnetic beagds, nitrocellulose bands or microparticles such as latex particles.

Consequently, the present invention also relates to a method of detecting the presence of a nucleic acid comprising a nucleotide sequence of any one of SEQ ID NOs: 1-4 and 9-126, or of a complementary nucleotide sequence, or a nucleic acid fragment or variant of any one of SEQ ID NOs: 1-4 and 9-126, or of a complementary nucleotide sequence in a sample, said method comprising the steps of:
1) bringing one or more nucleotide probes or primers according to the invention into contact with the sample to be tested;
2) detecting the complex which may have formed between the probe(s) and the nucleic acid present in the sample.

According to a specific embodiment of the method of detection according to the invention, the oligonucleotide probes and primers are immobilized on a support.

According to another aspect, the oligonucleotide probes and primers comprise a detectable marker.

The invention relates, in addition, to a box or kit for detecting the presence of a nucleic acid according to the invention in a sample, said box or kit comprising:
a) one or more nucleotide probe(s) or primer(s) as described above;
b) where appropriate, the reagents necessary for the hybridization reaction.

According to a first aspect, the detection box or kit is characterized in that the probe(s) or primer(s) are immobilized on a support.

According to a second aspect, the detection box or kit is characterized in that the oligonucleotide probes comprise a detectable marker.

According to a specific embodiment of the detection kit described above, such a kit will comprise a plurality of oligonucleotide probes and/or primers in accordance with the invention which may be used to detect a target nucleic acid of interest or alternatively to detect mutations in the coding regions or the non-coding regions of the nucleic acids according to the invention, more particularly of nucleic acids comprising any one of SEQ ID NOs: 1-4 and 9-126, or a complementary nucleotide sequence.

Thus, the probes according to the invention, immobilized on a support, may be ordered into matrices such as "DNA chips". Such ordered matrices have in particular been described in US patent No. 5,143,854, in published PCT applications WO 90/15070 and WO 92/10092.

Support matrices on which oligonucleotide probes have been immobilized at a high density are for example described in US patent No. 5,412,087 and in published PCT application WO 95/11995.

The nucleotide primers according to the invention may be used to amplify any one of the nucleic acids according to the invention, and more particularly a nucleic acid comprising a nucleotide sequence of any one of SEQ ID NOs: 1-4 and 9-126, or of a complementary nucleotide sequence. Alternatively, the nucleotide primers according to the invention may be used to amplify a nucleic acid fragment or variant of any one of SEQ ID NOs: 1-4 and 9-126, or of a complementary nucleotide sequence.

In a particular embodiment, the nucleotide primers according to the invention may be used to amplify a nucleic acid comprising any one of SEQ ID NOs: 1-4 and 9-126, or as depicted in any one of SEQ ID NOs: 1-4 and 9-126, or of a complementary nucleotide sequence.

Another subject of the invention relates to a method of amplifying a nucleic acid according to the invention, and more particularly a nucleic acid comprising a) any one of SEQ ID NOs: 1-4 and 9-126, or a complementary nucleotide sequence, b) as depicted in any one of SEQ ID NOs: 1-4 and 9-126, or of a complementary nucleotide sequence, contained in a sample, said method comprising the steps of:
a) bringing the sample in which the presence of the target nucleic acid is suspected into contact with a pair of nucleotide primers whose hybridization position is located respectively on the 5' side and on the 3' side of the region of the target nucleic acid whose amplification is sought, in the presence of the reagents necessary for the amplification reaction; and
b) detecting the amplified nucleic acids.

To carry out the amplification method as defined above, use will be preferably made of any of the nucleotide primers described above.

The subject of the invention is, in addition, a box or kit for amplifying a nucleic acid according to the invention, and more particularly a nucleic acid comprising any one of SEQ ID NOs: 1-4 and 9-126, or a complementary nucleotide sequence, or as depicted in any one of SEQ ID NOs: 1-4 and 9-126, or of a complementary nucleotide sequence, said box or kit comprising:
a) a pair of nucleotide primers in accordance with the invention, whose hybridization position is located respectively on the 5' side and 3' side of the target nucleic acid whose amplification is sought; and optionally,
b) reagents necessary for the amplification reaction.

Such an amplification box or kit will preferably comprise at least one pair of nucleotide primers as described above.

The subject of the invention is, in addition, a box or kit for amplifying all or part of a nucleic acid comprising any one of SEQ ID NOs: 1-4 and 9-126, or a complementary nucleotide sequence, said box or kit comprising:
1) a pair of nucleotide primers in accordance with the invention, whose hybridization position is located respectively on the 5' side and 3' side of the target nucleic acid whose amplification is sought; and optionally,
2) reagents necessary for an amplification reaction.

Such an amplification box or kit will preferably comprise at least one pair of nucleotide primers as described above.

The invention also relates to a box or kit for detecting the presence of a nucleic acid according to the invention in a sample, said box or kit comprising:
a) one or more nucleotide probes according to the invention;
b) where appropriate, reagents necessary for a hybridization reaction.

According to a first aspect, the detection box or kit is characterized in that the nucleotide probe(s) and primer(s)are immobilized on a support.

According to a second aspect, the detection box or kit is characterized in that the nucleotide probe(s) and primer(s) comprise a detectable marker.

According to a specific embodiment of the detection kit described above, such a kit will comprise a plurality of oligonucleotide probes and/or primers in accordance with the invention which may be used to detect target nucleic acids of interest or alternatively to detect mutations in the coding regions or the non-coding regions of the nucleic acids according to the invention. According to preferred embodiment of the invention, the target nucleic acid comprises a nucleotide sequence of any one of SEQ ID NOs: 1-4 and 9-126, or of a complementary nucleic acid sequence. Alternatively, the target nucleic acid is a nucleic acid fragment or variant of a nucleic acid comprising any one of SEQ ID NOs: 1-4 and 9-126, or of a complementary nucleotide sequence.

According to the present invention, a primer according to the invention comprises, generally, all or part of any one of SEQ ID NOs: 127-217, or a complementary sequence.

The nucleotide primers according to the invention are particularly useful in methods of genotyping subjects and/or of genotyping populations, in particular in the context of studies of association between particular allele forms or particular forms of groups of alleles (haplotypes) in subjects and the existence of a particular phenotype (character) in these subjects, for example the predisposition of these subjects to develop diseases linked to a deficiency of cholesterol reverse transport and inflammation signaling lipids, or alternatively the predisposition of these subjects to develop a pathology whose candidate chromosomal region is situated on chromosome 17, more precisely on the 17q arm and still more precisely in the 17q24 locus.

### RECOMBINANT VECTORS

The invention also relates to a recombinant vector comprising a nucleic acid according to the invention. "Vector" for the purposes of the present invention will be understood to mean a circular or linear DNA or RNA molecule which is either in single-stranded or double-stranded form.

Preferably, such a recombinant vector will comprise a nucleic acid chosen from the following nucleic acids:
a) a nucleic acid comprising a nucleotide sequence of any one of SEQ ID NOs: 1-4 and 9-126, or of a complementary nucleotide sequence,
b) a nucleic acid comprising a nucleotide sequence as depicted in any one of SEQ ID NOs: 1-4 and 9-126, or of a complementary nucleotide sequence,
c) a nucleic acid having at least eight consecutive nucleotides of a nucleic acid comprising a nucleotide sequence of any one of SEQ ID NOs: 1-4 and 9-126, or of a complementary nucleotide sequence;
d) a nucleic acid having at least 80% nucleotide identity with a nucleic acid comprising a nucleotide sequence of any one of SEQ ID NOs: 1-4 and 9-126, or a complementary nucleotide sequence;
e) a nucleic acid having 85%, 90%, 95%, or 98% nucleotide identity with a nucleic acid comprising a nucleotide sequence of any one of SEQ ID NOs: 1-4 and 9-126, or a complementary nucleotide sequence;
f) a nucleic acid hybridizing, under high stringency hybridization conditions, with a nucleic acid comprising a nucleotide sequence of 1) any one of SEQ ID NOs: 1-4 and 9-126, or a complementary nucleotide sequence;
g) a nucleic acid encoding a polypeptide comprising an amino acid sequence of SEQ ID NO: 5-8; and
h) a nucleic acid encoding a polypeptide comprising amino acid sequence selected from SEQ ID NO: 5-8.

According to a first embodiment, a recombinant vector according to the invention is used to amplify a nucleic acid inserted therein, following transformation or transfection of a desired cellular host.

According to a second embodiment, a recombinant vector according to the invention corresponds to an expression vector comprising, in addition to a nucleic acid in accordance with the invention, a regulatory signal or nucleotide sequence that directs or controls transcription and/or translation of the nucleic acid and its encoded mRNA.

According to a preferred embodiment, a recombinant vector according to the invention will comprise in particular the following components:
(1) an element or signal for regulating the expression of the nucleic acid to be inserted, such as a promoter and/or enhancer sequence;
(2) a nucleotide coding region comprised within the nucleic acid in accordance with the invention to be inserted into such a vector, said coding region being placed in phase with the regulatory element or signal described in (1); and
(3) an appropriate nucleic acid for initiation and termination of transcription of the nucleotide coding region of the nucleic acid described in (2).

In addition, the recombinant vectors according to the invention may include one or more origins for replication in the cellular hosts in which their amplification or their expression is sought, markers or selectable markers.

By way of example, the bacterial promoters may be the LacI or LacZ promoters, the T3 or T7 bacteriophage RNA polymerase promoters, the lambda phage PR or PL promoters.

The promoters for eukaryotic cells will comprise the herpes simplex virus (HSV) virus thymidine kinase promoter or alternatively the mouse metallothionein-L promoter.

Generally, for the choice of a suitable promoter, persons skilled in the art can preferably refer to the book by Sambrook et al. (1989, *Molecular cloning: a laboratory* *manual.* 2ed. Cold Spring Harbor Laboratory, Cold spring Harbor, New York) cited above or to the techniques described by Fuller et al. (1996, *Immunology, In: Current Protocols in Molecular Biology,* Ausubel et al.(eds.).

When the expression of the genomic sequence of any one of the ABCA5, ABCA6, ABCA9, and ABCA10 genes will be sought, use will preferably be made of the vectors capable of containing large insertion sequences. In a particular embodiment, bacteriophage vectors such as the P1 bacteriophage vectors such as the vector p158 or the vector p158/neo8 described by Sternberg (1992, Trends Genet., 8:1-16; 1994, Mamm. Genome, 5:397-404) will be preferably used.

The preferred bacterial vectors according to the invention are for example the vectors pBR322(ATCC37017) or alternatively vectors such as pAA223-3 (Pharmacia, Uppsala, Sweden), and pGEM1 (Promega Biotech, Madison, WI, UNITED STATES).

There may also be cited other commercially available vectors such as the vectors pQE70, pQE60, pQE9 (Qiagen), psiX174, pBluescript SA, pNH8A, pNH16A, pNH18A, pNH46A, pWLNEO, pSV2CAT, pOG44, pXTI, pSG (Stratagene).

They may also be vectors of the baculovirus type such as the vector pVL1392/1393 (Pharmingen) used to transfect cells of the Sf9 line (ATCC No. CRL 1711) derived from *Spodoptera frugiperda.*

They may also be adenoviral vectors such as the human adenovirus of type 2 or 5.

A recombinant vector according to the invention may also be a retroviral vector or an adeno-associated vector (AAV). Such adeno-associated vectors are for example described by Flotte et al. (1992, Am. J. Respir. Cell Mol. Biol., 7:349-356), Samulski et al. (1989, J. Virol., 63:3822-3828), or McLaughlin BA et al. (1996, Am. J. Hum. Genet., 59:561-569).

To allow the expression of a polynucleotide according to the invention, the latter must be introduced into a host cell. The introduction of a polynucleotide according to the invention into a host cell may be carried out *in vitro*, according to the techniques well known to persons skilled in the art for transforming or transfecting cells, either in primer culture, or in the form of cell lines. It is also possible to carry out the introduction of a polynucleotide according to the invention *in vivo* or *ex vivo*, for the prevention or treatment of diseases linked to ABC A5, A6, A9 or A10 deficiencies .

To introduce a polynucleotide or vector of the invention into a host cell, a person skilled in the art can preferably refer to various techniques, such as the calcium phosphate precipitation technique (Graham et al., 1973, Virology, 52:456-457 ; Chen et al., 1987, Mol. Cell. Biol., 7 : 2745-2752), DEAE Dextran (Gopal, 1985, Mol. Cell. Biol., 5:1188-1190), electroporation (Tur-Kaspa, 1896, Mol. Cell. Biol., 6:716-718 ; Potter et al., 1984, Proc Natl Acad Sci U S A., 81(22):7161-5), direct microinjection (Harland et al., 1985, J. Cell. Biol., 101:1094-1095), liposomes charged with DNA (Nicolau et al., 1982, Methods Enzymol., 149:157-76; Fraley et al., 1979, Proc. Natl. Acad. Sci. USA, 76:3348-3352).

Once the polynucleotide has been introduced into the host cell, it may be stably integrated into the genome of the cell. The intregration may be achieved at a precise site of the genome, by homologous recombination, or it may be randomly integrated. In some embodiments, the polynucleotide may be stably maintained in the host cell in the form of an episome fragment, the episome comprising sequences allowing the retention and the replication of the latter, either independently, or in a synchronized manner with the cell cycle.

According to a specific embodiment, a method of introducing a polynucleotide according to the invention into a host cell, in particular a host cell obtained from a mammal, *in vivo*, comprises a step during which a preparation comprising a pharmaceutically compatible vector and a "naked" polynucleotide according to the invention, placed under the control of appropriate regulatory sequences, is introduced by local injection at the level of the chosen tissue, for example myocardial tissue, the "naked" polynucleotide being absorbed by the myocytes of this tissue.

Compositions for use *in vitro* and *in vivo* comprising "naked" polynucleotides are for example described in PCT Application No. WO 95/11307 (Institut Pasteur, Inserm, University of Ottawa) as well as in the articles by Tacson et al. (1996, Nature Medicine, 2(8):888-892) and Huygen et al. (1996, Nature Medicine, 2(8):893-898).

According to a specific embodiment of the invention, a composition is provided for the *in vivo* production of any one of ABCA5, ABCA6, ABCA9, and ABCA10 proteins. This composition comprises a polynucleotide encoding the ABCA5, ABCA6, ABCA9, and ABCA10 polypeptides placed under the control of appropriate regulatory sequences, in solution in a physiologically acceptable vector.

The quantity of vector which is injected into the host organism chosen varies according to the site of the injection. As a guide, there may be injected between about 0.1 and about 100 µg of polynucleotide encoding the ABCA5, ABCA6, ABCA9, and ABCA10 proteins into the body of an animal, preferably into a patient likely to develop a disease linked ABC A5, A6, A9 or A10 deficiencies Consequently, the invention also relates to a pharmaceutical composition intended for the prevention of or treatment of a patient or subject affected by ABC A5, A6, A9 or A10 deficiencies, comprising a nucleic acid encoding any one of the ABCA5, ABCA6, ABCA9, and ABCA10 proteins, in combination with one or more physiologically compatible excipients.

Preferably, such a composition will comprise a nucleic acid comprising a nucleotide sequence of any one of SEQ ID NO: 1-4, wherein the nucleic acid is placed under the control of an appropriate regulatory element or signal.

The subject of the invention is, in addition, a pharmaceutical composition intended for the prevention of or treatment of a patient or a subject affected ABC A5, A6, A9 or A10 deficiencies, comprising a recombinant vector according to the invention, in combination with one or more physiologically compatible excipients.

The invention also relates to the use of a nucleic acid according to the invention, encoding any one of the ABCA5, ABCA6, ABCA9, and ABCA10 proteins, for the manufacture of a medicament intended for the prevention of arteriosclerosis in various forms or more particularly for the treatment of subjects affected by a dysfunction of cholesterol reverse transport or inflammatory liphophilic substances transport.

The invention also relates to the use of a recombinant vector according to the invention, comprising a nucleic acid encoding any one of the ABCA5, ABCA6, ABCA9, and ABCA10 proteins, for the manufacture of a medicament intended for the prevention of arteriosclerosis in various forms or more particularly for the treatment of subjects affected by a dysfunction of cholesterol reverse transport or inflammatory liphophilic substances transport.

The subject of the invention is therefore also a recombinant vector comprising a nucleic acid according to the invention that encodes any one of ABCA5, ABCA6, ABCA9, and ABCA10 proteins or polypeptides..

The invention also relates to the use of such a recombinant vector for the preparation of a pharmaceutical composition intended for the treatment and/or for the prevention of diseases or conditions associated with deficiency of cholesterol reverse transport or inflammatory liphophilic substances transport.

The present invention also relates to the use of cells genetically modified *ex vivo* with such a recombinant vector according to the invention, or of cells producing a recombinant vector, wherein the cells are implanted in the body, to allow a prolonged and effective expression *in vivo* of at least a biologically active ABCA5, ABCA6, ABCA9, and ABCA10 polypeptides.

Vectors useful in methods of somatic gene therapy and compositions containing such vectors.

The present invention also relates to a new therapeutic approach for the treatment of pathologies linked to ABC A5, A6, A9 or A10 deficiencies.. It provides an advantageous solution to the disadvantages of the prior art, by demonstrating the possibility of treating the pathologies ABC A5, A6, A9 or A10 deficiencies by gene therapy, by the transfer and expression *in vivo* of a gene encoding at least one of ABCA5, ABCA6, ABCA9, and ABCA10 proteins involved in the transport of lipophilic substances. The invention thus offers a simple means allowing a specific and effective treatment of related pathologies such as, for example, arteriosclerosis, inflammation, cardiovascular diseases, metabolic diseases, lipophilic substances related pathologies.

Gene therapy consists in correcting a deficiency or an abnormality (mutation, aberrant expression and the like) and in bringing about the expression of a protein of therapeutic interest by introducing genetic information into the affected cell or organ. This genetic information may be introduced either *ex vivo* into a cell extracted from the organ, the modified cell then being reintroduced into the body, or directly *in vivo* into the appropriate tissue. In this second case, various techniques exist, among which various transfection techniques involving complexes of DNA and DEAE-dextran (Pagano et al. (1967. J. Virol., 1:891), of DNA and nuclear proteins (Kaneda et al., 1989, Science 243:375), of DNA and lipids (Felgner et al., 1987, PNAS 84:7413), the use of liposomes (Fraley et al., 1980, J.Biol.Chem., 255:10431), and the like. More recently, the use of viruses as vectors for the transfer of genes has appeared as a promising alternative to these physical transfection techniques. In this regard, various viruses have been tested for their capacity to infect certain cell populations. In particular, the retroviruses (RSV, HMS, MMS, and the like), the HSV virus, the adeno-associated viruses and the adenoviruses.

The present invention therefore also relates to a new therapeutic approach for the treatment of pathologies linked to ABC A5, A6, A9 or A10 deficiencies, consisting in transferring and in expressing *in vivo* genes encoding any one of ABCA5, ABCA6, ABCA9, and ABCA10. In a particularly preferred manner, the applicant has now found that it is possible to construct recombinant vectors comprising a nucleic acid encoding at least one of ABCA5, ABCA6, ABCA9, and ABCA10 proteins, to administer these recombinant vectors *in vivo,* and that this administration allows a stable and effective expression of at least one of the biologically active ABCA5, ABCA6, ABCA9, and ABCA10 proteins *in vivo*, with no cytopathological effect.

Adenoviruses constitute particularly efficient vectors for the transfer and the expression of any one of the ABCA5, ABCA6, ABCA9, and ABCA10 genes. The use of recombinant adenoviruses as vectors makes it possible to obtain sufficiently high levels of expression of this gene to produce the desired therapeutic effect. Other viral vectors such as retroviruses or adeno-associated viruses (AAV) can allow a stable expression of the gene are also claimed.

The present invention is thus likely to offer a new approach for the treatment and prevention of ABC A5, A6, A9 or A10 deficiencies .

The subject of the invention is therefore also a defective recombinant virus comprising a nucleic acid according to the invention that encodes at least one ABCA5, ABCA6, ABCA9, and ABCA10 proteins or polypeptides involved in the metabolism of lipophilic substances.

The invention also relates to the use of such a defective recombinant virus for the preparation of a pharmaceutical composition which may be useful for the treatment and/or for the prevention of ABC A5, A6, A9 or A10 deficiencies.

The present invention also relates to the use of cells genetically modified *ex vivo* with such a defective recombinant virus according to the invention, or of cells producing a defective recombinant virus, wherein the cells are implanted in the body, to allow a prolonged and effective expression *in vivo* of at least one biologically active ABCA5, ABCA6, ABCA9, and ABCA10 polypeptides.

The present invention is particularly advantageous because it is it possible to induce a controlled expression, and with no harmful effect, of ABCA5, ABCA6, ABCA9, and ABCA10 in organs which are not normally involved in the expression of this protein. In particular, a significant release of the ABCA5, ABCA6, ABCA9, and ABCA10 proteins is obtained by implantation of cells producing vectors of the invention, or infected ex vivo with vectors of the invention.

The activity of these ABC protein transporters produced in the context of the present invention may be of the human or animal ABCA5, ABCA6, ABCA9, and ABCA10 type. The nucleic sequence used in the context of the present invention may be a cDNA, a genomic DNA (gDNA), an RNA (in the case of retroviruses) or a hybrid construct consisting, for example, of a cDNA into which one or more introns (gDNA) would be inserted. It may also involve synthetic or semisynthetic sequences. In a particularly advantageous manner, a cDNA or a gDNA is used. In particular, the use of a gDNA allows a better expression in human cells. To allow their incorporation into a viral vector according to the invention, these sequences are preferably modified, for example by site-directed mutagenesis, in particular for the insertion of appropriate restriction sites. The sequences described in the prior art are indeed not constructed for use according to the invention, and prior adaptations may prove necessary, in order to obtain substantial expressions. In the context of the present invention, the use of a nucleic sequence encoding any one of human ABCA5, ABCA6, ABCA9, and ABCA10 proteins is preferred. Moreover, it is also possible to use a construct encoding a derivative of any one of ABCA5, ABCA6, ABCA9, and ABCA10 proteins. A derivative of any one of ABCA5, ABCA6, ABCA9, and ABCA10 proteins comprises, for example, any sequence obtained by mutation, deletion and/or addition relative to the native sequence, and encoding a product retaining the lipophilic subtances transport activity. These modifications may be made by techniques known to a person skilled in the art (see general molecular biological techniques below). The biological activity of the derivatives thus obtained can then be easily determined, as indicated in particular in the examples of the measurement of the efflux of the substrate from cells. The derivatives for the purposes of the invention may also be obtained by hybridization from nucleic acid libraries, using as probe the native sequence or a fragment thereof.

These derivatives are in particular molecules having a higher affinity for their binding sites, molecules exhibiting greater resistance to proteases, molecules having a higher therapeutic efficacy or fewer side effects, or optionally new biological properties. The derivatives also include the modified DNA sequences allowing improved expression *in vivo*.

In a first embodiment, the present invention relates to a defective recombinant virus comprising a cDNA encoding any one of ABCA5, ABCA6, ABCA9, and ABCA10 polypeptides. In another preferred embodiment of the invention, a defective recombinant virus comprises a genomic DNA (gDNA) encoding any one of ABCA5, ABCA6, ABCA9, and ABCA10 polypeptides. Preferably, the ABCA5, ABCA6, ABCA9, and ABCA10 polypeptides comprise an amino acid sequence selected from SEQ ID NO:5-8, respectively.

The vectors of the invention may be prepared from various types of viruses. Preferably, vectors derived from adenoviruses, adeno-associated viruses (AAV), herpesviruses (HSV) or retroviruses are used. It is preferable to use an adenovirus, for direct administration or for the ex vivo modification of cells intended to be implanted, or a retrovirus, for the implantation of producing cells.

The viruses according to the invention are defective, that is to say that they are incapable of autonomously replicating in the target cell. Generally, the genome of the defective viruses used in the context of the present invention therefore lacks at least the sequences necessary for the replication of said virus in the infected cell. These regions may be either eliminated (completely or partially), or made non functional, or substituted with other sequences and in particular with the nucleic sequence encoding any one of the ABCA5, ABCA6, ABCA9, and ABCA10 proteins. Preferably, the defective virus retains, nevertheless, the sequences of its genome which are necessary for the encapsidation of the viral particles.

As regards more particularly adenoviruses, various serotypes, whose structure and properties vary somewhat, have been characterized. Among these serotypes, human adenoviruses of type 2 or 5 (Ad 2 or Ad 5) or adenoviruses of animal origin (see Application WO 94/26914) are preferably used in the context of the present invention. Among the adenoviruses of animal origin which can be used in the context of the present invention, there may be mentioned adenoviruses of canine, bovine, murine (example: Mav1, Beard et al., Virology 75 (1990) 81), ovine, porcine, avian or simian (example: SAV) origin. Preferably, the adenovirus of animal origin is a canine adenovirus, more preferably a CAV2 adenovirus [Manhattan or A26/61 strain (ATCC VR-800) for example]. Preferably, adenoviruses of human or canine or mixed origin are used in the context of the invention. Preferably, the defective adenoviruses of the invention comprise the ITRs, a sequence allowing the encapsidation and the sequence encoding any one of the ABCA5, ABCA6, ABCA9, and ABCA10 proteins. Preferably, in the genome of the adenoviruses of the invention, the E1 region at least is made non functional. Still more preferably, in the genome of the adenoviruses of the invention, the E1 gene and at least one of the E2, E4 and L1-L5 genes are non functional. The viral gene considered may be made non functional by any technique known to a person skilled in the art, and in particular by total suppression, by substitution, by partial deletion or by addition of one or more bases in the gene(s) considered. Such modifications may be obtained *in vitro* (on the isolated DNA) or *in situ,* for example, by means of genetic engineering techniques, or by treatment by means of mutagenic agents. Other regions may also be modified, and in particular the E3 (WO95/02697), E2 (WO94/28938), E4 (WO94/28152, WO94/12649, WO95/02697) and L5 (WO95/02697) region. According to a preferred embodiment, the adenovirus according to the invention comprises a deletion in the E1 and E4 regions and the sequence encoding any one of ABCA5, ABCA6, ABCA9, and ABCA10 is inserted at the level of the inactivated E1 region. According to another preferred embodiment, it comprises a deletion in the E1 region at the level of which the E4 region and the sequence encoding any one of ABCA5, ABCA6, ABCA9, and ABCA10 (French Patent Application FR94 13355) are inserted.

The defective recombinant adenoviruses according to the invention may be prepared by any technique known to persons skilled in the art (Levrero et al., 1991 Gene 101; EP 185 573; and Graham, 1984, EMBO J., 3:2917). In particular, they may be prepared by homologous recombination between an adenovirus and a plasmid carrying, *inter alia*, the nucleic acid encoding any one of the ABCA5, ABCA6, ABCA9, and ABCA10 proteins. The homologous recombination occurs after cotransfection of said adenoviruses and plasmid into an appropriate cell line. The cell line used must preferably (i) be transformable by said elements, and (ii), contain the sequences capable of complementing the part of the defective adenovirus genome, preferably in integrated form in order to avoid the risks of recombination. By way of example of a line, there may be mentioned the human embryonic kidney line 293 (Graham et al., 1977, J. Gen. Virol., 36:59), which contains in particular, integrated into its genome, the left part of the genome of an Ad5 adenovirus (12%) or lines capable of complementing the E1 and E4 functions as described in particular in Applications No. WO 94/26914 and WO95/02697.

As regards the adeno-associated viruses (AAV), they are DNA viruses of a relatively small size, which integrate into the genome of the cells which they infect, in a stable and site-specific manner. They are capable of infecting a broad spectrum of cells, without inducing any effect on cellular growth, morphology or differentiation. Moreover, they do not appear to be involved in pathologies in humans. The genome of AAVs has been cloned, sequenced and characterized. It comprises about 4700 bases, and contains at each end an inverted repeat region (ITR) of about 145 bases, serving as replication origin for the virus. The remainder of the genome is divided into 2 essential regions carrying the encapsidation functions: the left hand part of the genome, which contains the rep gene, involved in the viral replication and the expression of the viral genes; the right hand part of the genome, which contains the cap gene encoding the virus capsid proteins.

The use of vectors derived from AAVs for the transfer of genes *in vitro* and *in vivo* has been described in the literature (see in particular WO 91/18088; WO 93/09239; US 4,797,368, US5,139,941, EP 488 528). These applications describe various constructs derived from AAVs, in which the rep and/or cap genes are deleted and replaced by a gene of interest, and their use for transferring *in vitro* (on cells in culture) or *in vivo* (directly into an organism) said gene of interest. However, none of these documents either describes or suggests the use of a recombinant AAV for the transfer and expression *in vivo* or *ex vivo* of any one of ABCA5, ABCA6, ABCA9, and ABCA10 proteins, or the advantages of such a transfer. The defective recombinant AAVs according to the invention may be prepared by cotransfection, into a cell line infected with a human helper virus (for example an adenovirus), of a plasmid containing the sequence encoding any one of the ABCA5, ABCA6, ABCA9, and ABCA10 proteins bordered by two AAV inverted repeat regions (ITR), and of a plasmid carrying the AAV encapsidation genes (rep and cap genes). The recombinant AAVs produced are then purified by conventional techniques.

As regards the herpesviruses and the retroviruses, the construction of recombinant vectors has been widely described in the literature: see in particular Breakfield et al., (1991.New Biologist, 3:203); EP 453242, EP178220, Bernstein et al. (1985); McCormick, (1985. BioTechnology, 3:689), and the like.

In particular, the retroviruses are integrating viruses, infecting dividing cells. The genome of the retroviruses essentially comprises two long terminal repeats (LTRs), an encapsidation sequence and three coding regions (gag, pol and env). In the recombinant vectors derived from retroviruses, the gag, pol and env genes are generally deleted, completely or partially, and replaced with a heterologous nucleic acid sequence of interest. These vectors may be produced from various types of retroviruses such as in particular MoMuLV ("murine moloney leukemia virus"; also called MoMLV), MSV ("murine moloney sarcoma virus"), HaSV ("harvey sarcoma virus"); SNV ("spleen necrosis virus"); RSV ("rous sarcoma virus") or Friend's virus.

To construct recombinant retroviruses containing a sequence encoding any one fo the ABCA5, ABCA6, ABCA9, and ABCA10 proteins according to the invention, a plasmid containing in particular the LTRs, the encapsidation sequence and said coding sequence is generally constructed, and then used to transfect a so-called encapsidation cell line, capable of providing in trans the retroviral functions deficient in the plasmid. Generally, the encapsidation lines are therefore capable of expressing the gag, pol and env genes. Such encapsidation lines have been described in the prior art, and in particular the PA317 line (US 4,861,719), the PsiCRIP line (WO 90/02806) and the GP+envAm-12 line (WO 89/07150). Moreover, the recombinant retroviruses may contain modifications at the level of the LTRs in order to suppress the transcriptional activity, as well as extended encapsidation sequences, containing a portion of the gag gene (Bender et al., 1987, J. Virol., 61:1639). The recombinant retroviruses produced are then purified by conventional techniques.

To carry out the present invention, it is preferable to use a defective recombinant adenovirus. The particularly advantageous properties of adenoviruses are preferred for the *in vivo* expression of a protein having a lipophilic subtrate transport activity. The adenoviral vectors according to the invention are particularly preferred for a direct administration *in vivo* of a purified suspension, or for the ex vivo transformation of cells, in particular autologous cells, in view of their implantation. Furthermore, the adenoviral vectors according to the invention exhibit, in addition, considerable advantages, such as in particular their very high infection efficiency, which makes it possible to carry out infections using small volumes of viral suspension.

According to another particularly preferred embodiment of the invention, a line producing retroviral vectors containing the sequence encoding any one of the ABCA5, ABCA6, ABCA9, and ABCA10 proteins is used for implantation *in vivo.* The lines which can be used to this end are in particular the PA317 (US 4,861,719), PsiCrip (WO 90/02806) and GP+envAm-12 (US 5,278,056) cells modified so as to allow the production of a retrovirus containing a nucleic sequence encoding any one of ABCA5, ABCA6, ABCA9, and ABCA10 proteins according to the invention. For example, totipotent stem cells, precursors of blood cell lines, may be collected and isolated from a subject. These cells, when cultured, may then be transfected with the retroviral vector containing the sequence encoding any one of ABCA5, ABCA6, ABCA9, and ABCA10 proteins under the control of viral, nonviral or nonviral promoters specific for macrophages or under the control of its own promoter. These cells are then reintroduced into the subject. The differentiation of these cells will be responsible for blood cells expressing at least one of ABCA5, ABCA6, ABCA9, and ABCA10 proteins.

Preferably, in the vectors of the invention, the sequence encoding any one of the ABCA5, ABCA6, ABCA9, and ABCA10 proteins is placed under the control of signals allowing its expression in the infected cells. These may be expression signals which are homologous or heterologous, that is to say signals different from those which are naturally responsible for the expression of the ABCA5, ABCA6, ABCA9, and ABCA10 proteins. They may also be in particular sequences responsible for the expression of other proteins, or synthetic sequences. In particular, they may be sequences of eukaryotic or viral genes or derived sequences, stimulating or repressing the transcription of a gene in a specific manner or otherwise and in an inducible manner or otherwise. By way of example, they may be promoter sequences derived from the genome of the cell which it is desired to infect, or from the genome of a virus, and in particular the promoters of the E1A or major late promoter (MLP) genes of adenoviruses, the cytomegalovirus (CMV) promoter, the RSV-LTR and the like. Among the eukaryotic promoters, there may also be mentioned the ubiquitous promoters (HPRT, vimentin, α-actin, tubulin and the like), the promoters of the intermediate filaments (desmin, neurofilaments, keratin, GFAP, and the like), the promoters of therapeutic genes (of the MDR, CFTR or factor VIII type, and the like), tissue-specific promoters (pyruvate kinase, villin, promoter of the fatty acid binding intestinal protein, promoter of the smooth muscle cell α-actin, promoters specific for the liver; Apo AI, Apo AII, human albumin and the like) or promoters corresponding to a stimulus (steroid hormone receptor, retinoic acid receptor and the like). In addition, these expression sequences may be modified by addition of enhancer or regulatory sequences and the like. Moreover, when the inserted gene does not contain expression sequences, it may be inserted into the genome of the defective virus downstream of such a sequence.

In a specific embodiment, the invention relates to a defective recombinant virus comprising a nucleic acid encoding any one of ABCA5, ABCA6, ABCA9, and ABCA10 proteins the control of a promoter chosen from RSV-LTR or the CMV early promoter.

As indicated above, the present invention also relates to any use of a virus as described above for the preparation of a pharmaceutical composition for the treatment and/or prevention of pathologies linked to the transport of lipophilic substances.

The present invention also relates to a pharmaceutical composition comprising one or more defective recombinant viruses as described above. These pharmaceutical compositions may be formulated for administration by the topical, oral, parenteral, intranasal, intravenous, intramuscular, subcutaneous, intraocular or transdermal route and the like. Preferably, the pharmaceutical compositions of the invention comprises a pharmaceutically acceptable vehicle or physiologically compatible excipient for an injectable formulation, in particular for an intravenous injection, such as for example into the patient's portal vein. These may relate in particular to isotonic sterile solutions or dry, in particular, freeze-dried, compositions which, upon addition depending on the case of sterilized water or physiological saline, allow the preparation of injectable solutions. Direct injection into the patient's portal vein is preferred because it makes it possible to target the infection at the level of the liver and thus to concentrate the therapeutic effect at the level of this organ.

The doses of defective recombinant virus used for the injection may be adjusted as a function of various parameters, and in particular as a function of the viral vector, of the mode of administration used, of the relevant pathology or of the desired duration of treatment. In general, the recombinant adenoviruses according to the invention are formulated and administered in the form of doses of between 10⁴ and 10¹⁴ pfu/ml, and preferably 10⁶ to 10¹⁰ pfu/ml. The term "pfu" (plaque forming unit) corresponds to the infectivity of a virus solution, and is determined by infecting an appropriate cell culture and measuring, generally after 48 hours, the number of plaques that result from infected cell lysis. The techniques for determining the pfu titer of a viral solution are well documented in the literature.

As regards retroviruses, the compositions according to the invention may directly contain the producing cells, with a view to their implantation.

In this regard, another subject of the invention relates to any mammalian cell infected with one or more defective recombinant viruses according to the invention. More particularly, the invention relates to any population of human cells infected with such viruses. These may be in particular cells of blood origin (totipotent stem cells or precursors), fibroblasts, myoblasts, hepatocytes, keratinocytes, smooth muscle and endothelial cells, glial cells and the like.

The cells according to the invention may be derived from primary cultures. These may be collected by any technique known to persons skilled in the art and then cultured under conditions allowing their proliferation. As regards more particularly fibroblasts, these may be easily obtained from biopsies, for example according to the technique described by Ham (1980). These cells may be used directly for infection with the viruses, or stored, for example by freezing, for the establishment of autologous libraries, in view of a subsequent use. The cells according to the invention may be secondary cultures, obtained for example from pre-established libraries (see for example EP 228458, EP 289034, EP 400047, EP 456640).

The cells in culture are then infected with a recombinant virus according to the invention, in order to confer on them the capacity to produce at least one biologically active ABCA5, ABCA6, ABCA9, and ABCA10 proteins. The infection is carried out *in vitro* according to techniques known to persons skilled in the art. In particular, depending on the type of cells used and the desired number of copies of virus per cell, persons skilled in the art can adjust the multiplicity of infection and optionally the number of infectious cycles produced. It is clearly understood that these steps must be carried out under appropriate conditions of sterility when the cells are intended for administration *in vivo*. The doses of recombinant virus used for the infection of the cells may be adjusted by persons skilled in the art according to the desired aim. The conditions described above for the administration *in vivo* may be applied to the infection *in vitro*. For the infection with a retrovirus, it is also possible to co-culture a cell to be infected with a cell producing the recombinant retrovirus according to the invention. This makes it possible to eliminate purification of the retrovirus.

Another subject of the invention relates to an implant comprising mammalian cells infected with one or more defective recombinant viruses according to the invention or cells producing recombinant viruses, and an extracellular matrix. Preferably, the implants according to the invention comprise 10⁵ to 10¹⁰ cells. More preferably, they comprise 10⁶ to 10⁸ cells.

More particularly, in the implants of the invention, the extracellular matrix comprises a gelling compound and optionally a support allowing the anchorage of the cells.

For the preparation of the implants according to the invention, various types of gelling agents may be used. The gelling agents are used for the inclusion of the cells in a matrix having the constitution of a gel, and for promoting the anchorage of the cells on the support, where appropriate. Various cell adhesion agents can therefore be used as gelling agents, such as in particular collagen, gelatin, glycosaminoglycans, fibronectin, lectins and the like. Preferably, collagen is used in the context of the present invention. This may be collagen of human, bovine or murine origin. More preferably, type I collagen is used.

As indicated above, the compositions according to the invention preferably comprise a support allowing the anchorage of the cells. The term anchorage designates any form of biological and/or chemical and/or physical interaction causing the adhesion and/or the attachment of the cells to the support. Moreover, the cells may either cover the support used, or penetrate inside this support, or both. It is preferable to use in the context of the invention a solid, nontoxic and/or biocompatible support. In particular, it is possible to use polytetrafluoroethylene (PTFE) fibers or a support of biological origin.

The present invention thus offers a very effective means for the treatment or prevention of pathologies linked to the transport of lipophilic substances.

In addition, this treatment may be applied to both humans and any animals such as ovines, bovines, domestic animals (dogs, cats and the like), horses, fish and the like.

### RECOMBINANT HOST CELLS

The invention relates to a recombinant host cell comprising a nucleic acid of the invention, and more particularly, a nucleic acid comprising a nucleotide sequence selected from SEQ ID NO: 1-4 and 9-126, or a complementary nucleotide sequence thereof.

The invention also relates to a recombinant host cell comprising a nucleic acid of the invention, and more particularly a nucleic acid comprising a nucleotide sequence as depicted in SEQ ID NO: 1-4 and 9-126, or a complementary nucleotide sequence thereof.

According to another aspect, the invention also relates to a recombinant host cell comprising a recombinant vector according to the invention. Therefore, the invention also relates to a recombinant host cell comprising a recombinant vector comprising any of the nucleic acids of the invention, and more particularly a nucleic acid comprising a nucleotide sequence of selected from SEQ ID NO: 1-4 and 9-126, or a complementary nucleotide sequence thereof.

The invention also relates to a recombinant host cell comprising a recombinant vector comprising a nucleic acid comprising a nucleotide sequence as depicted in any one of SEQ ID NOs: 1-4 and 9-126, or of a complementary nucleotide sequence thereof.

The preferred host cells according to the invention are for example the following:
a) prokaryotic host cells: strains of *Escherichia coli* (strain DH5-α), of *Bacillus subtilis,* of *Salmonella typhimurium,* or strains of genera such as *Pseudomonas, Streptomyces* and *Staphylococus* ;
b) eukaryotic host cells: HeLa cells (ATCC No. CCL2), Cv 1 cells (ATCC No. CCL70), COS cells (ATCC No. CRL 1650), Sf-9 cells (ATCC No. CRL 1711), CHO cells (ATCC No. CCL-61) or 3T3 cells (ATCC No. CRL-6361).

### METHODS FOR PRODUCING ABCA5, ABCA6, ABCA9, and ABCA10 POLYPEPTIDES

The invention also relates to a method for the production of a polypeptide comprising an amino acid sequence of any one of SEQ ID NOs: 5-8, said method comprising the steps of:
a) inserting a nucleic acid encoding said polypeptide into an appropriate vector;
b) culturing, in an appropriate culture medium, a previously transformed host cell or transfecting a host cell with the recombinant vector of step a);
c) recovering the conditioned culture medium or lysing the host cell, for example by sonication or by osmotic shock;
d) separating and purifying said polypeptide from said culture medium or alternatively from the cell lysates obtained in step c); and
e) where appropriate, characterizing the recombinant polypeptide produced.

The polypeptides according to the invention may be characterized by binding to an immunoaffinity chromatography column on which the antibodies directed against this polypeptide or against a fragment or a variant thereof have been previously immobilized.

According to another aspect, a recombinant polypeptide according to the invention may be purified by passing it over an appropriate series of chromatography columns, according to methods known to persons skilled in the art and described for example in F. Ausubel et al (1989, Current Protocols in Molecular Biology, Green Publishing Associates and Wiley Interscience, N.Y).

A polypeptide according to the invention may also be prepared by conventional chemical synthesis techniques either in homogeneous solution or in solid phase. By way of illustration, a polypeptide according to the invention may be prepared by the technique either in homogeneous solution described by Houben Weyl (1974, Meuthode der Organischen Chemie, E. Wunsch Ed., 15-I:15-II) or the solid phase synthesis technique described by Merrifield (1965, Nature, 207(996):522-523; 1965, Science, 150(693):178-185).

A polypeptide termed "homologous" to a polypeptide having an amino acid sequence selected from SEQ ID NO: 5-8 also forms part of the invention. Such a homologous polypeptide comprises an amino acid sequence possessing one or more substitutions of an amino acid by an equivalent amino acid of SEQ ID NO:5-8.

An "equivalent amino acid" according to the present invention will be understood to mean for example replacement of a residue in the L form by a residue in the D form or the replacement of a glutamic acid (E) by a pyro-glutamic acid according to techniques well known to persons skilled in the art. By way of illustration, the synthesis of peptide containing at least one residue in the D form is described by Koch (1977). According to another aspect, two amino acids belonging to the same class, that is to say two uncharged polar, nonpolar, basic or acidic amino acids, are also considered as equivalent amino acids.

Polypeptides comprising at least one nonpeptide bond such as a retro-inverse bond (NHCO), a carba bond (CH₂CH₂) or a ketomethylene bond (CO-CH₂) also form part of the invention.

Preferably, the polypeptides according to the invention comprising one or more additions, deletions, substitutions of at least one amino acid will retain their capacity to be recognized by antibodies directed against the nonmodified polypeptides.

### ANTIBODIES

The ABCA5, ABCA6, ABCA9, and ABCA10 polypeptides according to the invention, in particular 1) a polypeptide comprising an amino acid sequence of any one of SEQ ID NOs: 5-8, 2) a polypeptide fragment or variant of a polypeptide comprising an amino acid sequence of any one of SEQ ID NOs: 5-8, or 3) a polypeptide termed "homologous" to a polypeptide comprising amino acid sequence selected from SEQ ID NOs: 5-8, may be used for the preparation of an antibody, in particular for detecting the production of a normal or altered form of ABCA5, ABCA6, ABCA9, and ABCA10 polypeptides in a patient.

An antibody directed against a polypeptide termed "homologous" to a polypeptide having an amino acid sequence selected from SEQ ID NO: 5-8 also forms part of the invention. Such an antibody is directed against a homologous polypeptide comprising an amino acid sequence possessing one or more substitutions of an amino acid by an equivalent amino acid of SEQ ID NO: 5-8. "Antibody" for the purposes of the present invention will be understood to mean in particular polyclonal or monoclonal antibodies or fragments (for example the F(ab)'₂ and Fab fragments) or any polypeptide comprising a domain of the initial antibody recognizing the target polypeptide or polypeptide fragment according to the invention.

Monoclonal antibodies may be prepared from hybridomas according to the technique described by Kohler and Milstein (1975, Nature, 256:495-497).

According to the invention, a polypeptide produced recombinantly or by chemical synthesis, and fragments or other derivatives or analogs thereof, including fusion proteins, may be used as an immunogen to generate antibodies that recognize a polypeptide according to the invention. Such antibodies include but are not limited to polyclonal, monoclonal, chimeric, single chain, Fab fragments, and an Fab expression library. The anti-ABCA5, anti-ABCA6, anti-ABCA9, and anti-ABCA10 antibodies of the invention may be cross reactive, *e.g.*, they may recognize corresponding ABCA5, ABCA6, ABCA9, and ABCA10 polypeptides from different species. Polyclonal antibodies have greater likelihood of cross reactivity. Alternatively, an antibody of the invention may be specific for a single form of any one of ABCA5, ABCA6, ABCA9, and ABCA10. Preferably, such an antibody is specific for any one of human ABCA5, ABCA6, ABCA9, and ABCA10.

Various procedures known in the art may be used for the production of polyclonal antibodies to any one of ABCA5, ABCA6, ABCA9, and ABCA10 polypeptides or derivatives or analogs thereof. For the production of antibody, various host animals can be immunized by injection with any one of ABCA5, ABCA6, ABCA9, and ABCA10 polypeptides, or a derivatives (*e.g.*, fragment or fusion protein) thereof, including but not limited to rabbits, mice, rats, sheep, goats, etc. In one embodiment, any one of the ABCA5, ABCA6, ABCA9, and ABCA10 polypeptides or fragments thereof can be conjugated to an immunogenic carrier, *e.g.*, bovine serum albumin (BSA) or keyhole limpet hemocyanin (KLH). Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (*bacille Calmette-Guerin*) and *Corynebacterium parvum.*

For preparation of monoclonal antibodies directed toward any one of ABCA5, ABCA6, ABCA9, and ABCA10 polypeptides, or fragments, analogs, or derivatives thereof, any technique that provides for the production of antibody molecules by continuous cell lines in culture may be used. These include but are not limited to the hybridoma technique originally developed by Kohler and Milstein (1975, Nature, 256:495-497), as well as the trioma technique, the human B-cell hybridoma technique (Kozbor et al., 1983, Immunology Today, 4:72; Cote et al. 1983, Proc. Natl. Acad. Sci. U.S.A. 80:2026-2030), and the EBV-hybridoma technique to produce human monoclonal antibodies (Cole et al., 1985, In: *Monoclonal Antibodies and Cancer Therapy*, Alan R. Liss, Inc., pp. 77-96). In an additional embodiment of the invention, monoclonal antibodies can be produced in germ-free animals (WO 89/12690). In fact, according to the invention, techniques developed for the production of "chimeric antibodies" (Morrison et al., 1984, J. Bacteriol. 159:870; Neuberger et al., 1984, Nature, 312:604-608; Takeda et al., 1985, Nature 314:452-454) by splicing the genes from a mouse antibody molecule specific for any one of ABCA5, ABCA6, ABCA9, and ABCA10 polypeptides together with genes from a human antibody molecule of appropriate biological activity can be used; such antibodies are within the scope of this invention. Such human or humanized chimeric antibodies are preferred for use in therapy of human diseases or disorders (described *infra*), since the human or humanized antibodies are much less likely than xenogenic antibodies to induce an immune response, in particular an allergic response, themselves.

According to the invention, techniques described for the production of single chain antibodies (U.S. Patent Nos. 5,476,786 and 5,132,405 to Huston; U.S. Patent 4,946,778) can be adapted to produce ABCA5, ABCA6, ABCA9, and ABCA10 polypeptide-specific single chain antibodies. An additional embodiment of the invention utilizes the techniques described for the construction of Fab expression libraries (Huse et al., 1989, Science 246:1275-1281) to allow rapid and easy identification of monoclonal Fab fragments with the desired specificity for any one of ABCA5, ABCA6, ABCA9, and ABCA10 polypeptides, or its derivatives, or analogs.

Antibody fragments which contain the idiotype of the antibody molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, and the Fab fragments which can be generated by treating the antibody molecule with papain and a reducing agent.

In the production of antibodies, screening for the desired antibody can be accomplished by techniques known in the art, *e.g*., radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), western blots, precipitation reactions, agglutination assays (*e.g*., gel agglutination assays, hemagglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labelled. Many means are known in the art for detecting binding in an immunoassay and are within the scope of the present invention. For example, to select antibodies which recognize a specific epitope of any one of ABCA5, ABCA6, ABCA9, and ABCA10 polypeptides, one may assay generated hybridomas for a product which binds to any one of ABCA5, ABCA6, ABCA9, and ABCA10 polypeptide fragments containing such epitope. For selection of an antibody specific to any one of of ABCA5, ABCA6, ABCA9, and ABCA10 polypeptides from a particular species of animal, one can select on the basis of positive binding with any one of ABCA5, ABCA6, ABCA9, and ABCA10 polypeptides expressed by or isolated from cells of that species of animal.

The foregoing antibodies can be used in methods known in the art relating to the localization and activity of any one of ABCA5, ABCA6, ABCA9, and ABCA10 polypeptides, *e.g.*, for Western blotting, ABCA5, ABCA6, ABCA9, and ABCA10 polypeptides *in situ*, measuring levels thereof in appropriate physiological samples, etc. using any of the detection techniques mentioned above or known in the art.

In a specific embodiment, antibodies that agonize or antagonize the activity of any one of ABCA5, ABCA6, ABCA9, and ABCA10 polypeptides can be generated. Such antibodies can be tested using the assays described *infra* for identifying ligands.

The present invention relates to an antibody directed against 1) a polypeptide comprising an amino acid sequence of any one of SEQ ID NOs: 5-8, 2) a polypeptide fragment or variant of a polypeptide comprising an amino acid sequence of any one of SEQ ID NOs: 5-8, or 3) a polypeptide termed "homologous" to a polypeptide comprising amino acid sequence selected from SEQ ID NO:5-8, also forms part of the invention, as produced in the trioma technique or the hybridoma technique described by Kozbor et al. (1983, Hybridoma, 2(1):7-16).

The invention also relates to single-chain Fv antibody fragments (ScFv) as described in US patent No. 4,946,778 or by Martineau et al. (1998, J Mol Biol, 280(1):117-127).

The antibodies according to the invention also comprise antibody fragments obtained with the aid of phage libraries as described by Ridder et al., (1995, Biotechnology (NY), 13(3):255-260) or humanized antibodies as described by Reinmann et al. (1997, AIDS Res Hum Retroviruses, 13(11):933-943) and Leger et al., (1997, Hum Antibodies, 8(1):3-16).

The antibody preparations according to the invention are useful in immunological detection tests intended for the identification of the presence and/or of the quantity of antigens present in a sample.

An antibody according to the invention may comprise, in addition, a detectable marker which is isotopic or nonisotopic, for example fluorescent, or may be coupled to a molecule such as biotin, according to techniques well known to persons skilled in the art.

Thus, the subject of the invention is, in addition, a method of detecting the presence of a polypeptide according to the invention in a sample, said method comprising the steps of:
a) bringing the sample to be tested into contact with an antibody directed against 1) a polypeptide comprising an amino acid sequence of any one of SEQ ID NOs: 5-8, 2) a polypeptide fragment or variant of a polypeptide comprising an amino acid sequence of any one of SEQ ID NOs: 5-8, or 3) a polypeptide termed "homologous" to a polypeptide comprising amino acid sequence selected from SEQ ID NOs: 5-8, and
b) detecting the antigen/antibody complex formed.

The invention also relates to a box or kit for diagnosis or for detecting the presence of a polypeptide in accordance with the invention in a sample, said box comprising:
a) an antibody directed against 1) a polypeptide comprising an amino acid sequence of any one of SEQ ID NOs:5-8, 2) a polypeptide fragment or variant of a polypeptide comprising an amino acid sequence of any one of SEQ ID NOs: 5-8, or 3) a polypeptide termed "homologous" to a polypeptide comprising amino acid sequence selected from SEQ ID NOs: 5-8, and
b) a reagent allowing the detection of the antigen/antibody complexes formed.

### PHARMACEUTICAL COMPOSITIONS AND THERAPEUTIC METHODS OF TREATMENT

The invention also relates to pharmaceutical compositions intended for the prevention and/or treatment of a deficiency in the transport of cholesterol or inflammatory lipid substances, characterized in that they comprise a therapeutically effective quantity of a polynucleotide capable of giving rise to the production of an effective quantity of at least one of ABCA5, ABCA6, ABCA9, and ABCA10 functional polypeptides, in particular a polypeptide comprising an amino acid sequence of SEQ ID NOs: 5-8.

The invention also provides pharmaceutical compositions comprising a nucleic acid encoding any one of ABCA5, ABCA6, ABCA9, and ABCA10 polypeptides according to the invention and pharmaceutical compositions comprising any one of ABCA5, ABCA6, ABCA9, and ABCA10 polypeptides according to the invention intended for the prevention and/or treatment of diseases linked to a deficiency in the transport of cholesterol or inflammatory lipid substances.

The present invention also relates to a new therapeutic approach for the treatment of pathologies linked to the transport of lipophilic substances, comprising transferring and expressing *in vivo* nucleic acids encoding at least one of ABCA5, ABCA6, ABCA9, and ABCA10 proteins according to the invention.

Thus, the present invention offers a new approach for the treatment and/or the prevention of pathologies linked to the abnormalities of the transport of lipophilic substances.

Consequently, the invention also relates to a pharmaceutical composition intended for the prevention of or treatment of subjects affected by, a dysfunction in lipophilic substances, comprising a nucleic acid encoding at least one of the ABCA5, ABCA6, ABCA9, and ABCA10 proteins, in combination with one or more physiologically compatible vehicle and/or excipient.

According to a specific embodiment of the invention, a composition is provided for the *in vivo* production of at least one of the ABCA5, ABCA6, ABCA9, and ABCA10 proteins. This composition comprises a nucleic acid encoding any one of the ABCA5, ABCA6, ABCA9, and ABCA10 polypeptides placed under the control of appropriate regulatory sequences, in solution in a physiologically acceptable vehicle and/or excipient.

Therefore, the present invention also relates to a composition comprising a nucleic acid encoding a polypeptide comprising an amino acid sequence of SEQ ID NOs: 5-8, wherein the nucleic acid is placed under the control of appropriate regulatory elements.

Preferably, such a composition will comprise a nucleic acid comprising a nucleotide sequence of SEQ ID NOs:1-4, placed under the control of appropriate regulatory elements.

According to another aspect, the subject of the invention is also a preventive and/or curative therapeutic method of treating diseases caused by a deficiency in the transport of lipophilic substances, such a method comprising a step in which there is administered to a patient a nucleic acid encoding any one of the ABCA5, ABCA6, ABCA9, and ABCA10 polypeptides according to the invention in said patient, said nucleic acid being, where appropriate, combined with one or more physiologically compatible vehicles and/or excipients.

The invention also relates to a pharmaceutical composition intended for the prevention of or treatment of subjects affected by, a dysfunction in the transport of lipophilic substances, comprising a recombinant vector according to the invention, in combination with one or more physiologically compatible excipients.

According to a specific embodiment, a method of introducing a nucleic acid according to the invention into a host cell, in particular a host cell obtained from a mammal, *in vivo*, comprises a step during which a preparation comprising a pharmaceutically compatible vector and a "naked" nucleic acid according to the invention, placed under the control of appropriate regulatory sequences, is introduced by local injection at the level of the chosen tissue, for example a smooth muscle tissue, the "naked" nucleic acid being absorbed by the cells of this tissue.

The invention also relates to the use of a nucleic acid according to the invention, encoding any one of the ABCA5, ABCA6, ABCA9, and ABCA10 proteins, for the manufacture of a medicament intended for the prevention and/or treatment in various forms or more particularly for the treatment of subjects affected by a dysfunction in the transport of lipophilic substances.

The invention also relates to the use of a recombinant vector according to the invention, comprising a nucleic acid encoding any one of the ABCA5, ABCA6, ABCA9, and ABCA10 proteins, for the manufacture of a medicament intended for the prevention and/or treatment of subjects affected by a dysfunction in the transport of lipophilic substances.

As indicated above, the present invention also relates to the use of a defective recombinant virus according to the invention for the preparation of a pharmaceutical composition for the treatment and/or prevention of pathologies linked to the transport of lipophilic substances.

The invention relates to the use of such a defective recombinant virus for the preparation of a pharmaceutical composition intended for the treatment and/or prevention of a deficiency associated with the transport of lipophilic substances. Thus, the present invention also relates to a pharmaceutical composition comprising one or more defective recombinant viruses according to the invention.

The present invention also relates to the use of cells genetically modified *ex vivo* with a virus according to the invention, or of producing cells such as viruses, implanted in the body, allowing a prolonged and effective expression *in vivo* of at least one biologically active ABCA5, ABCA6, ABCA9, and ABCA10 proteins.

The present invention shows that it is possible to incorporate a nucleic acid encoding any one of the ABCA5, ABCA6, ABCA9, and ABCA10 polypeptides into a viral vector, and that these vectors make it possible to effectively express a biologically active, mature form. More particularly, the invention shows that the *in vivo* expression of any one of ABCA5, ABCA6, ABCA9, and ABCA10 genes may be obtained by direct administration of an adenovirus or by implantation of a producing cell or of a cell genetically modified by an adenovirus or by a retrovirus incorporating such a DNA.

Preferably, the pharmaceutical compositions of the invention comprise a pharmaceutically acceptable vehicle or physiologically compatible excipient for an injectable formulation, in particular for an intravenous injection, such as for example into the patient's portal vein. These may relate in particular to isotonic sterile solutions or dry, in particular, freeze-dried, compositions which, upon addition depending on the case of sterilized water or physiological saline, allow the preparation of injectable solutions. Direct injection into the patient's portal vein is preferred because it makes it possible to target the infection at the level of the liver and thus to concentrate the therapeutic effect at the level of this organ.

A "pharmaceutically acceptable vehicle or excipient" includes diluents and fillers which are pharmaceutically acceptable for method of administration, are sterile, and may be aqueous or oleaginous suspensions formulated using suitable dispersing or wetting agents and suspending agents. The particular pharmaceutically acceptable carrier and the ratio of active compound to carrier are determined by the solubility and chemical properties of the composition, the particular mode of administration, and standard pharmaceutical practice.

Any nucleic acid, polypeptide, vector, or host cell of the invention will preferably be introduced *in vivo* in a pharmaceutically acceptable vehicle or excipient. The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "excipient" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as excipients, particularly for injectable solutions. Suitable pharmaceutical excipients are described in "Remington's Pharmaceutical Sciences" by E.W. Martin.

The pharmaceutical compositions according to the invention may be equally well administered by the oral, rectal, parenteral, intravenous, subcutaneous or intradermal route.

According to another aspect, the subject of the invention is also a preventive and/or curative therapeutic method of treating diseases caused by a deficiency in the transport of cholesterol or inflammatory lipid substances, comprising administering to a patient or subject a nucleic acid encoding any one of ABCA5, ABCA6, ABCA9, and ABCA10 polypeptides, said nucleic acid being combined with one or more physiologically compatible vehicles and/or excipients.

In another embodiment, the nucleic acids, , recombinant vectors, and compositions according to the invention can be delivered in a vesicle, in particular a liposome (see Langer, 1990, Science, 249:1527-1533; Treat et al., 1989, *Liposomes in the Therapy of Infectious Disease and Cancer*, Lopez-Berestein and Fidler (eds.), Liss: New York, pp. 353-365; and Lopez-Berestein, 1989, In: *Liposomes in the Therapy of Infectious Disease and Cancer,* Lopez-Berestein and Fidler (eds.), Liss: New York, pp. 317-327).

In a further aspect, recombinant cells that have been transformed with a nucleic acid according to the invention and that express high levels of any one of ABCA5, ABCA6, ABCA9, and ABCA10 polypeptides according to the invention can be transplanted in a subject in need of any one of ABCA5, ABCA6, ABCA9, and ABCA10 polypeptides. Preferably autologous cells transformed with an any one of ABCA5, ABCA6, ABCA9, and ABCA10 encoding nucleic acids according to the invention are transplanted to avoid rejection; alternatively, technology is available to shield non-autologous cells that produce soluble factors within a polymer matrix that prevents immune recognition and rejection.

A subject in whom administration of the nucleic acids, polypeptides, recombinant vectors, recombinant host cells, and compositions according to the invention is performed is preferably a human, but can be any animal. Thus, as can be readily appreciated by one of ordinary skill in the art, the methods and pharmaceutical compositions of the present invention are particularly suited to administration to any animal, particularly a mammal, and including, but by no means limited to, domestic animals, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., avian species, such as chickens, turkeys, songbirds, etc., *i.e.*, for veterinary medical use.

Preferably, a pharmaceutical composition comprising a nucleic acid, a recombinant vector, or a recombinant host cell, as defined above, will be administered to the patient or subject.

### METHODS OF SCREENING AN AGONIST OR ANTAGONIST COMPOUND FOR THE ABCA5, ABCA6, ABCA9, and ABCA10 POLYPEPTIDES

According to another aspect, the invention also relates to various methods of screening compounds or small molecules for therapeutic use which are useful in the treatment of diseases due to a deficiency in the transport of cholesterol or inflammatory lipid substances.

The invention therefore also relates to the use of any one of ABCA5, ABCA6, ABCA9, and ABCA10 polypeptides, or of cells expressing any one of ABCA5, ABCA6, ABCA9, and ABCA10 polypeptides, for screening active ingredients for the prevention and/or treatment of diseases resulting from a dysfunction in ABCA5, ABCA6, ABCA9, or ABCA10 defiencies . The catalytic sites and oligopeptide or immunogenic fragments of any one of ABCA5, ABCA6, ABCA9, and ABCA10 polypeptides can serve for screening product libraries by a whole range of existing techniques. The polypeptide fragment used in this type of screening may be free in solution, bound to a solid support, at the cell surface or in the cell. The formation of the binding complexes between any one of the ABCA5, ABCA6, ABCA9, and ABCA10 polypeptide fragments and the tested agent can then be measured.

Another product screening technique which may be used in high-flux screenings giving access to products having affinity for the protein of interest is described in application WO84/03564. In this method, applied to any one of ABCA5, ABCA6, ABCA9, and ABCA10 proteins, various products are synthesized on a solid surface. These products react with corresponding ABCA5, ABCA6, ABCA9, and ABCA10 proteins or fragments thereof and the complex is washed. The products binding any one of the ABCA5, ABCA6, ABCA9, and ABCA10 proteins are then detected by methods known to persons skilled in the art. Non-neutralizing antibodies can also be used to capture a peptide and immobilize it on a support.

Another possibility is to perform a product screening method using any one of the ABCA5, ABCA6, ABCA9, and ABCA10 neutralizing competition antibodies, at least one of ABCA5, ABCA6, ABCA9, and ABCA10 proteins and a product potentially binding any one of the ABCA5, ABCA6, ABCA9, and ABCA10 proteins. In this manner, the antibodies may be used to detect the presence of a peptide having a common antigenic unit with any one of ABCA5, ABCA6, ABCA9, and ABCA10 polypeptides or proteins.

Of the products to be evaluated for their ability to increase activity of any one of ABCA5, ABCA6, ABCA9, and ABCA10, there may be mentioned in particular kinase-specific ATP homologs involved in the activation of the molecules, as well as phosphatases, which may be able to avoid the dephosphorylation resulting from said kinases. There may be mentioned in particular inhibitors of the phosphodiesterase (PDE) theophylline and 3-isobutyl-1-methylxanthine type or the adenylcyclase forskolin activators.

Accordingly, this invention relates to the use of any method of screening products, *i.e.*, compounds, small molecules, and the like, based on the method of translocation of cholesterol or lipophilic substances between the membranes or vesicles, this being in all synthetic or cellular types, that is to say of mammals, insects, bacteria, or yeasts expressing constitutively or having incorporated any one of human ABCA5, ABCA6, ABCA9, and ABCA10 encoding nucleic acids. To this effect, labeled lipophilic substances analogs may be used.
Furthermore, knowing that the disruption of numerous transporters have been described (van den Hazel et al., 1999, J. Biol Chem, 274: 1934-41), it is possible to think of using cellular mutants having a characteristic phenotype and to complement the function thereof with at least one of ABCA5, ABCA6, ABCA9, and ABCA10 proteins and to use the whole for screening purposes.

The invention also relates to a method of screening a compound or small molecule active on the transport of lipophilic substances, an agonist or antagonist of any one of ABCA5, ABCA6, ABCA9, and ABCA10 polypeptides, said method comprising the following steps:
a) preparing a membrane vesicle comprising at least one of the ABCA5, ABCA6, ABCA9, and ABCA10 polypeptides and a lipid substrate comprising a detectable marker;
b) incubating the vesicle obtained in step a) with an agonist or antagonist candidate compound;
c) qualitatively and/or quantitatively measuring release of the lipid substrate comprising a detectable marker; and
d) comparing the release measurement obtained in step b) with a measurement of release of labeled lipophilic substrate by a vesicle that has not been previously incubated with the agonist or antagonist candidate compound.

ABCA5, ABCA6, ABCA9, and ABCA10 polypeptides comprise an amino acid sequence selected from SEQ ID NOs: 5-8.

According to a first aspect of the above screening method, the membrane vesicle is a synthetic lipid vesicle, which may be prepared according to techniques well known to a person skilled in the art. According to this particular aspect, ABCA5, ABCA6, ABCA9, and ABCA10 proteins may be recombinant proteins.

According to a second aspect, the membrane vesicle is a vesicle of a plasma membrane derived from cells expressing at least one of ABCA5, ABCA6, ABCA9, and ABCA10 polypeptides. These may be cells naturally expressing any one of ABCA5, ABCA6, ABCA9, and ABCA10 polypeptides or cells transfected with a nucleic acid encoding at least one ABCA5, ABCA6, ABCA9, and ABCA10 polypeptides or recombinant vector comprising a nucleic acid encoding any one of ABCA5, ABCA6, ABCA9, and ABCA10 polypeptides.

According to a third aspect of the above screening method, the lipid substrate is chosen from prostaglandins or prostacyclins.

According to a fourth aspect of the above screening method, the lipid substrate is chosen from cholesterol or phosphatidylcholine.

According to a fifth aspect, the lipid substrate is radioactively labelled, for example with an isotope chosen from ³H or ¹²⁵I.

According to a sixth aspect, the lipid substrate is labelled with a fluorescent compound, such as NBD or pyrene.

According to a seventh aspect, the membrane vesicle comprising the labelled lipophilic substances and any one of the ABCA5, ABCA6, ABCA9, and ABCA10 polypeptides is immobilized at the surface of a solid support prior to step b).

According to a eighth aspect, the measurement of the fluorescence or of the radioactivity released by the vesicle is the direct reflection of the activity of lipid substrate transport by any one of the ABCA5, ABCA6, ABCA9, and ABCA10 polypeptides.

The invention also relates to a method of screening a compound or small molecule active on the transport of cholesterol or lipid substances, an agonist or antagonist of any one of ABCA5, ABCA6, ABCA9, and ABCA10 polypeptides, said method comprising the following steps:
a) obtaining cells, for example a cell line, that, either naturally or after transfecting the cell with any one of ABCA5, ABCA6, ABCA9, AND ABCA10 encoding nucleic acids, expresses any one of ABCA5, ABCA6, ABCA9, AND ABCA10 polypeptides;
b) incubating the cells of step a) in the presence of an anion labelled with a detectable marker;
c) washing the cells of step b) in order to remove the excess of the labelled anion which has not penetrated into these cells;
d) incubating the cells obtained in step c) with an agonist or antagonist candidate compound for any one of ABCA5, ABCA6, ABCA9, and ABCA10 polypeptides;
e) measuring efflux of the labelled anion; and
f) comparing the value of efflux of the labelled anion determined in step e) with a value of the efflux of a labelled anion measured with cells that have not been previously incubated in the presence of the agonist or antagonist candidate compound of any one of ABCA5, ABCA6, ABCA9, and ABCA10 polypeptides.

In a first specific embodiment, any one of the ABCA5, ABCA6, ABCA9, and ABCA10 polypeptides comprise an amino acid sequence of SEQ ID NOs: 5-8.

According to a second aspect, the cells used in the screening method described above may be cells not naturally expressing, or alternatively expressing at a low level, any one of the ABCA5, ABCA6, ABCA9, and ABCA10 polypeptides, said cells being transfected with a recombinant vector according to the invention capable of directing the expression of a nucleic acid encoding any one of the ABCA5, ABCA6, ABCA9, and ABCA10 polypeptides.

According to a third aspect, the cells may be cells having a natural deficiency in anion transport, or cells pretreated with one or more anion channel inhibitors such as Verapamil™ or tetraethylammonium.

According to a fourth aspect of said screening method, the anion is a radioactively labelled iodide, such as the salts K¹²⁵I or Na¹²⁵I.

According to a fifth aspect, the measurement of efflux of the labelled anion is determined periodically over time during the experiment, thus making it possible to also establish a kinetic measurement of this efflux.

According to a sixth aspect, the value of efflux of the labelled anion is determined by measuring the quantity of labelled anion present at a given time in the cell culture supernatant.

According to a seventh aspect, the value of efflux of the labelled anion is determined as the proportion of radioactivity found in the cell culture supernatant relative to the total radioactivity corresponding to the sum of the radioactivity found in the cell lysate and the radioactivity found in the cell culture supernatant.

The subject of the invention is also a method of screening a compound or small molecule active on the metabolism of lipophilic substances, an agonist or antagonist of any one of ABCA5, ABCA6, ABCA9, and ABCA10 polypeptides, said method comprising the following steps:
a) culturing cells of a human myocyte line in an appropriate culture medium, in the presence of purified human albumin;
b) incubating the cells of step a) simultaneously in the presence of a compound stimulating the production of interleukine and of an agonist or antagonist candidate compound;
c) incubating the cells obtained in step b) in the presence of an appropriate concentration of ATP;
d) measuring interleukinereleased into the cell culture supernatant; and
e) comparing the value of the release of the interleukineobtained in step d) with the value of the interleukinereleased into the culture supernatant of cells which have not been previously incubated in the presence of the agonist or antagonist candidate compound.

According to a first aspect of the screening method described above, the cells used belong to the human or mousemyocytes.

According to a second aspect of the screening method, the compound stimulating the production of interleukineis a lipopolysaccharide.

According to a third aspect of said method, the production of all interleukinesand TNF alpha by these cells is also qualitatively and/or quantitatively determined.

According to a fourth aspect, the level of expression of the messenger RNA encoding interleukineis also determined.

The following examples are intended to further illustrate the present invention but do not limit the invention.

### EXAMPLES

### EXAMPLE 1 : Search of human ABCA5, ABCA6, ABCA9, AND ABCA10 genes in genomic database

Expressed sequence tags (EST) of ABCA1-like genes as described by Allikmets et al. (*Hum Mol Genet*, 1996, 5, 1649-1655) were used to search Genbank and UniGene nucleotide sequence databases using BLAST2 (Altschul et al, 1997, Nucleic Acids Res., 25:3389-3402). The main protein sequences databases screened were Swissprot, TrEMBL, Genpept and PIR.

The genomic DNA analysis was performed by combination of several gene-finding programs such as GENSCAN (Burge and Karlin, 1997, *J Mol Biol*., 268(1):78-94), FGENEH/FEXH (Solovyev and Salamov, 1997, *Ismb*; 5:294-302), and XPOUND (Thomas and Skolnick, 1994, *J Math Appl Med Biol*.;1(1):1-16). The combination of different tools lead to increase sensitivity and specificity. The second step in the genomic DNA analysis is the homology searching in the EST and protein databases. Combination of software performing database searching and software for exon/intron prediction give the best sensitive and specific results. Sequence assembly and analysis were performed using the Genetics Computer Group (GCG) sequence analysis software package.

Multiple alignments were generated by GAP software from GCG package and the Dialign2 program (Morgenstern et al, 1996, Proc Natl Acad Sci U S A.; 93(22):12098-103), the FASTA3 package (Pearson and Lipman, 1988, Proc Natl Acad Sci U S A.; 85(8):2444-8) and SIM4 (Florea et al, 1998, Genome Res. 1998 Sep;8(9):967-74). The specific ABCA motifs used in our process were the TMN, TMC, NBD1 and NBD2 described in the literature (Broccardo et al, 1999). This corresponds in ABCA1 to residues 630-846 for the N terminal (TMN = exon 14-16) and from 1647-1877 for the C terminal set of membrane spanners (TMC = exon 36-40). The NBD corresponds to the extended nucleotide binding domain, i.e., in ABCA1 it spans from amino acids 885-1152 for the N-terminal one (NBD1 = exon 18-22) and 1918-2132 for the C-terminal one (NBD2 = exon 42-47).

Sequence comparison between candidate ABCA ESTs with two overlapping BAC clones containing the microsatellite marker D17S940 (GenBank accession # AC005495, AC005922, revealed surprisingly that all these ESTs are located within this 325,000 bp. An electronic intron/exon prediction was performed by using the AC005495 and AC005922 BAC sequences, and provided potential transcript sequences which were predictied to correspond to the full coding sequence (CDS) of ABCA6 and ABCA9. ABCA5 gene sequences were found to be partially contain in the contig of BACs as 3' and 5' ends, respectively. Moreover, the analysis of the sequence revealed the ABCA10 gene, for which full CDS was also predicted.

Additional sequence information for ABCA5 was obtained by using the working draft BACs that overlap with the above described BAC contig (AC005495 and AC005922) on both 3' and 5' ends. A supplemental BAC working draft, e.g., GenBank Accession number AC007763, was then identified on the 5' end. A parallel database mining approach based on the specific motifs search in the different Genbank subdivisions and UniGene Homo sapiens led to identification of two of these sequences (one contains a TMC motif, one contains a NBD1 domain) which matched with two fragments of the BAC # AC007763.

Using exon-intron sequence of these genes, we compared the sequence of the cDNAs with the genomic sequence of the BACs (AC005495, AC005922 and AC007763) and established the approximate genomic size and respective orientation of the ABCA5, ABCA6, ABCA9, and ABCA10 genes.

### EXAMPLE 2 : 5' Extension of the human ABCA5, ABCA6, ABCA9, AND ABCA10 cDNA.

This Example describes the isolation and identification of cDNA molecules encoding the full length human ABCA5, ABCA6, ABCA9, and ABCA10 protein. 5' extension of the partial ABCA5, ABCA6, ABCA9, and ABCA10 cDNA sequence was performed by using a combination of 5' RACE and RT-PCR on liver, heart, or testis.

Oligonucleotide primers allowing to distinguish novel ABCA5-6 and 9-10 genes from other family members, were designed taking advantage of the exonic/intronic prediction of the genomic sequence and used to identify specific cDNA transcript by RT-PCR on RNA from various human tissues. With the exception of ABCA10 that required an additional cloning step, all RT-PCR products were directly sequenced. In the case of ABCA6, ABCA9, and ABCA10 a 5' RACE step was also performed in order to confirm the initiator ATG codon. The identification of the full CDS of ABCA5 was obtained by linking the 3 potential fragments of the transcript by RT-PCR and direct sequencing. Finally, full ORF sequences of these new genes that belong to the same chromosome 17 cluster were determined.

### Reverse transcription

In a total volume of 11.5 µl, 500 ng of mRNA poly(A)+ (Clontech) mixed with 500 ng of oligodT are denaturated at 70°C for 10 min and then chilled on ice. After addition of 10 units of RNAsin, 10 mM DTT, 0.5 mM dNTP, Superscript first strand buffer and 200 units of Superscript II (Life Technologies), the reaction is incubated for 45 min at 42°C. We used poly(A) mRNA from liver, heart, brain and lung for ABCA9, from testis for ABCA5, from testis and heart for ABCA10.

### PCR

Each polymerase chain reaction contained 400 µM each dNTP, 2 units of *Thermus aquaticus* (Taq) DNA polymerase (Ampli Taq Gold; Perkin Elmer), 0.5 µM each primer, 2.5 mM MgCl₂, PCR buffer and 50 ng of DNA, or about 25 ng of cDNA, or 1/50è of primary PCR mixture. Reactions were carried out for 30 cycles in a Perkin Elmer 9700 thermal cycler in 96-well microtiter plates. After an initial denaturation at 94°C for 10 min, each cycle consisted of: a denaturation step of 30 s (94°C), a hybridization step of 30 s (64°C for 2 cycles, 61°C for 2 cycles, 58°C for 2 cycles and 55°C for 28 cycles), and an elongation step of 1 min/kb (72°C). PCR ended with a final 72°C extension of 7 min. In case of RT-PCR, control reactions without reverse transcriptase and reactions containing water instead of cDNA were performed for every sample.

### DNA Sequencing

PCR products are analyzed and quantified by agarose gel electrophoresis, purified with a P100 column. Purified PCR products were sequenced using ABI Prism BigDye terminator cycle sequencing kit (Perkin Elmer Applied Biosystems). The sequence reaction mixture was purified using Microcon-100 microconcentrators (Amicon, Inc., Beverly). Sequencing reactions were resolved on an ABI 377 DNA sequencer (Perkin Elmer Applied Biosystems) according to manufacturer's protocol (Applied Biosystems, Perkin Elmer).

### 5' Rapid amplification of cDNA Ends (RACE)

5' RACE analysis was performed using the SMART RACE cDNA amplification kit (Clontech, Palo Alto, CA). Human testis, liver and heart polyA+ RNA (Clontech) were used as template to generate the 5' SMART cDNA library according to the manufacturer's instructions. First-amplification primers and nested primers were designed from the cDNA sequence. Amplimers of the nested PCR were cloned. Insert of specific clones are amplified by PCR with universal primers (Rev and -21) and sequenced on both strands. Primers ABC-A6_L1, L2, ABC-A9_L1, L2, and ABC-A10_L1, L2 were used to identify 5' ends of ABC-A6, ABC-A9, and ABC-A10 respectively.

### Primers

Oligonucleotides were selected using Prime from GCG package or Oligo 4 (National Biosciences, Inc.) softwares. Primers were ordered from Life Technologies, Ltd and used without further purification (Table 15).

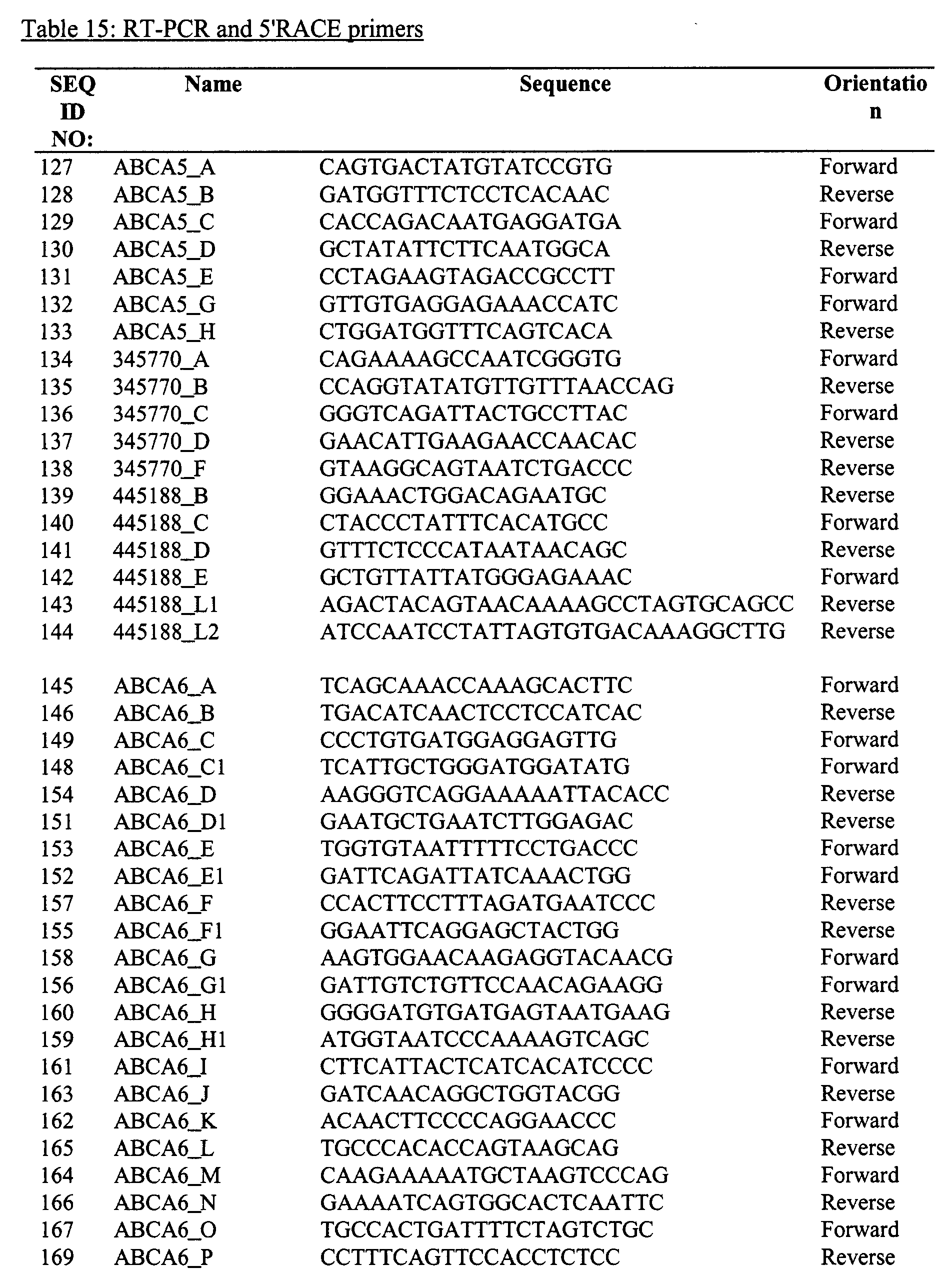

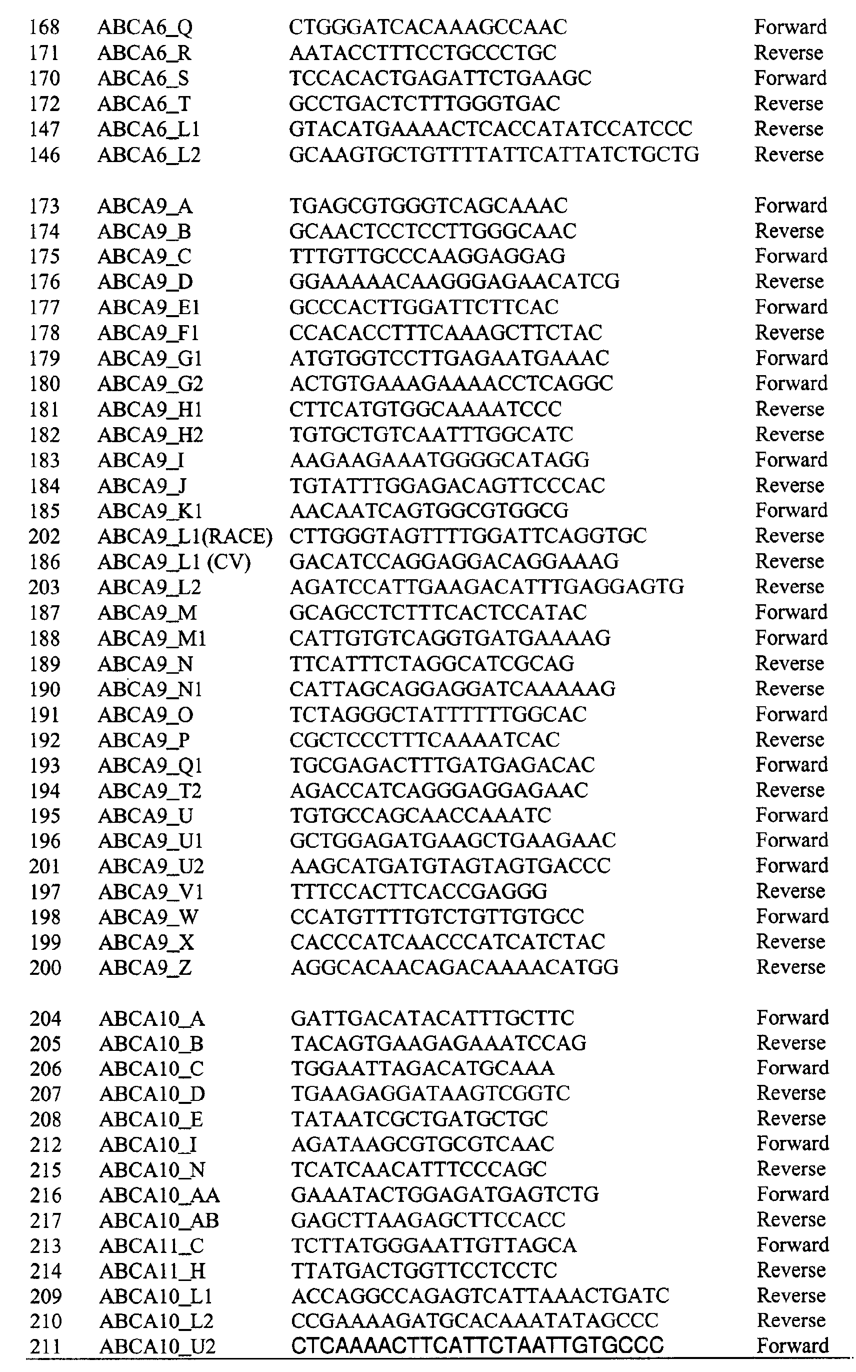

### EXAMPLE 3: Tissue distribution of the transcripts of the ABCA5, ABCA6, ABCA9, and ABCA10 genes according to the invention.

The profile of expression of the polynucleotides according to the present invention is determined according to the protocols for PCR-coupled reverse transcription and Northern blot analysis described in particular by Sambrook et al. (1989, *Molecular cloning: a laboratory manual.* 2ed. Cold Spring Harbor Laboratory, Cold spring Harbor, New York).

For example, in the case of an analysis by reverse transcription, a pair of primers as described above may be synthesized from a cDNA of the human ABCA5, ABCA6, ABCA9, and ABCA10 genes. This primer pair may be used to detect the corresponding ABCA5, ABCA6, ABCA9, and ABCA10 cDNAs.

The polymerase chain reaction (PCR) is carried out on cDNA templates corresponding to retrotranscribed polyA⁺ mRNAs (Clontech). The reverse transcription to cDNA is carried out with the enzyme SUPERSCRIPT II (GibcoBRL, Life Technologies) according to the conditions described by the manufacturer. The polymerase chain reaction is carried out according to standard conditions, in 20 µl of reaction mixture with 25 ng of cDNA preparation. The reaction mixture is composed of 400 µM of each of the dNTPs, 2 units of Thermus aquaticus (Taq) DNA polymerase (Ampli Taq Gold; Perkin Elmer), 0.5 µM of each primer, 2.5 mM MgCl2, and PCR buffer. Thirty four PCR cycles [denaturing 30 seconds at 94°C, annealing of 30 seconds divided up as follows during the 34 cycles: 64°C (2 cycles), 61°C (2 cycles), 58°C (2 cycles), and 55°C (28 cycles), and an extension of one minute per kilobase at 72°C] are carried out after a first step of denaturing at 94°C for 10 minutes using a Perkin Elmer 9700 thermocycler. The PCR reactions are visualized on agarose gel by electrophoresis. The cDNA fragments obtained may be used as probes for a Northern blot analysis and may also be used for the exact determination of the nucleotide sequence.

### Northern Blot Analysis

To study mRNA expression of the ABCA 5, ABCA6, ABCA9, and ABCA10 genes, human MTN (Multiple Tissue Northern) blots (Human II 7759-1, Human 7760-1, and human fetal II 7756-1, Clontech) were hybridized with specific pools of probes consisting in two amplimers: ABCA5_A-ABCA5_B and 445188_C-445188_D for ABC-A5, ABCA6_A-ABCA6_B and ABCA6_S-ABCA6_T for ABC-A6, ABCA9_A-ABCA9-B and ABCA9_M1-ABCA9_N1 for ABC-A9. A unique RT-PCR product obtained between ABCA10_I-ABCA10_B was used for ABC-A10 (Table 15).

### Preparation of the probe

PCR products were gel-purified using Qiaquick® column (Qiagen). 10-20 ng of purified PCR product were radiolabelled with [α³²P]dCTP (Amersham; 6000 Ci/mmol, 10 mCi/ml) by the random priming method (Rediprime kit; Amersham) according to the manufacturer's protocol. Unincorporated radioactive nucleotides were separated from the labelled probe by filtration on a G50 microcolumn (Pharmacia). Probe was competed with 50 µg of denatured human Cot1 DNA during 2 hours at 65°C.

### Hybridization

Prehybridization of Northern blot (6 hours at 42°C) with hybridization solution (5x SSPE, 5x Denhardt's, 2.5% Dextran, 0.5% SDS, 50% formamide, 100 µg/ml denaturated salmon sperm DNA, 40 µg denatured human DNA) was followed by hybridization with radiolabelled probe (2.10⁶ cpm/ml hybridization solution) and 40 µg of denatured human DNA. Filters were washed in 2x SSC for 30 min at room temperature, twice in 2x SSC-0.1% SDS for 10 min at 65°C and twice in 1x SSC-0.1% SDS for 10 min at 65°C. Northern blot were analyzed after overnight exposure on the Storm (Molecular Dynamics, Sunnyvale, CA). The human transferrin probe was used to control the amount of RNA in each lane of the membrane.

### EXAMPLE 4 : Construction of the expression vector containing the complete cDNA of ABCA5, ABCA6, ABCA9, or ABCA10 in mammalian cells

The ABCA5, ABCA6, ABCA9, or ABCA10 genes may be expressed in mammalian cells. A typical eukaryotic expression vector contains a promoter which allows the initiation of the transcription of the mRNA, a sequence encoding the protein, and the signals required for the termination of the transcription and for the polyadenylation of the transcript. It also contains additional signals such as enhancers, the Kozak sequence and sequences necessary for the splicing of the mRNA. An effective transcription is obtained with the early and late elements of the SV40 virus promoters, the retroviral LTRs or the CMV virus early promoter. However, cellular elements such as the actin promoter may also be used. Many expression vectors may be used to carry out the present invention, an example of such a vector is pcDNA3 (Invitrogen).

### EXAMPLE 5 : Production of normal and mutated ABCA5, ABCA6, ABCA9, or ABCA10 polypeptides.

The normal ABCA5, ABCA6, ABCA9, or ABCA10 polypeptides encoded by complete corresponding cDNAs whose isolation is described in Example 2, or the mutated ABCA5, ABCA6, ABCA9, or ABCA10 polypeptides whose complete cDNA may also be obtained according to the techniques described in Example 2, may be easily produced in a bacterial or insect cell expression system using the baculovirus vectors or in mammalian cells with or without the vaccinia virus vectors. All the methods are now widely described and are known to persons skilled in the art. A detailed description thereof will be found for example in F. Ausubel et al. (1989, Current Protocols in Molecular Biology, Green Publishing Associates and Wiley Interscience, N.Y).

### EXAMPLE 6 : Production of an antibody directed against one of the mutated ABCA5, ABCA6, ABCA9, or ABCA10 polypeptides.

The antibodies in the present invention may be prepared by various methods (Current Protocols In Molecular Biology Volume 1 edited by Frederick M. Ausubel, Roger Brent, Robert E. Kingston, David D. Moore, J.G. Seidman, John A. Smith, Kevin Struhl - Massachusetts General Hospital Harvard Medical School, chapter 11, 1989). For example, the cells expressing a polypeptide of the present invention are injected into an animal in order to induce the production of serum containing the antibodies. In one of the methods described, the proteins are prepared and purified so as to avoid contaminations. Such a preparation is then introduced into the animal with the aim of producing polyclonal antisera having a higher activity.

In the preferred method, the antibodies of the present invention are monoclonal antibodies. Such monoclonal antibodies may be prepared using the hybridoma technique (Köhler et al, 1975, Nature, 256:495 ; Köhler et al, 1976, Eur. J. Immunol. 6:292; Köhler et al, 1976, Eur. J. Immunol., 6:511; Hammeling et al., 1981, Monoclonal Antibodies and T-Cell Hybridomas, Elsevier, N.Y., pp. 563-681). In general, such methods involve immunizing the animal (preferably a mouse) with a polypeptide or better still with a cell expressing the polypeptide. These cells may be cultured in a suitable tissue culture medium. However, it is preferable to culture the cells in an Eagle medium (modified Earle) supplemented with 10% fetal bovine serum (inactivated at 56°C) and supplemented with about 10 g/l of nonessential amino acids, 1000 U/ml of penicillin and about 100 µg/ml of streptomycin.

The splenocytes of these mice are extracted and fused with a suitable myeloma cell line. However, it is preferable to use the parental myeloma cell line (SP2O) available from the ATCC. After fusion, the resulting hybridoma cells are selectively maintained in HAT medium and then cloned by limiting dilution as described by Wands et al. (1981, Gastroenterology, 80:225-232). The hybridoma cells obtained after such a selection are tested in order to identify the clones secreting antibodies capable of binding to the polypeptide.

Moreover, other antibodies capable of binding to the polypeptide may be produced according to a 2-stage procedure using anti-idiotype antibodies such a method is based on the fact that the antibodies are themselves antigens and consequently it is possible to obtain an antibody recognizing another antibody. According to this method, the antibodies specific for the protein are used to immunize an animal, preferably a mouse. The splenocytes of this animal are then used to produce hybridoma cells, and the latter are screened in order to identify the clones which produce an antibody whose capacity to bind to the specific antibody-protein complex may be blocked by the polypeptide. These antibodies may be used to immunize an animal in order to induce the formation of antibodies specific for the protein in a large quantity.

It is preferable to use Fab and F(ab')2 and the other fragments of the antibodies of the present invention according to the methods described here. Such fragments are typically produced by proteolytic cleavage with the aid of enzymes such as Papain (in order to produce the Fab fragments) or Pepsin (in order to produce the F(ab')2 fragments). Otherwise, the secreted fragments recognizing the protein may be produced by applying the recombinant DNA or synthetic chemistry technology.

For the *in vivo* use of antibodies in humans, it would be preferable to use "humanized" chimeric monoclonal antibodies. Such antibodies may be produced using genetic constructs derived from hybridoma cells producing the monoclonal antibodies described above. The methods for producing the chimeric antibodies are known to persons skilled in the art (for a review, see : Morrison (1985. Science 229:1202); Oi et al., (1986, Biotechnique, 4:214); Cabilly et al., US patent No. 4,816,567 ; Taniguchi et al., EP 171496; Morrison et al., EP 173494; Neuberger et al., WO 8601533 ; Robinson et al., WO 8702671; Boulianne et al ; (1984, Nature, 312:643); and Neuberger et al., (1985, Nature, 314:268).

### EXAMPLE 7 : Determination of polymorphisms/mutations in the ABCA5, ABCA6, ABCA9, or ABCA10 genes.

The detection of polymorphisms or of mutations in the sequences of the transcripts or in the genomic sequence of the ABCA5, ABCA6, ABCA9, or ABCA10 genes may be carried out according to various protocols. The preferred method is direct sequencing.

For patients from whom it is possible to obtain an mRNA preparation, the preferred method consists in preparing the cDNAs and sequencing them directly. For patients for whom only DNA is available, and in the case of a transcript where the structure of the corresponding gene is unknown or partially known, it is necessary to precisely determine its intron-exon structure as well as the genomic sequence of the corresponding gene. This therefore involves, in a first instance, isolating the genomic DNA BAC or cosmid clone(s) corresponding to the transcript studied, sequencing the insert of the corresponding clone(s) and detemrining the intron-exon structure by comparing the cDNA sequence to that of the genomic DNA obtained.

The technique of detection of mutations by direct sequencing consists in comparing the genomic sequences of the ABCA5, ABCA6, ABCA9, or ABCA10 gene obtained from homozygotes for the disease or from at least 8 individuals (4 individuals affected by the pathology studied and 4 individuals not affected) or from at least 32 unrelated individuals from the studied population. The sequence divergences constitute polymorphisms. All those modifying the amino acid sequence of the wild-type protein may be mutations capable of affecting the function of said protein which it is preferred to consider more particularly for the study of cosegregation of the mutation and of the disease (denoted genotype-phenotype correlation) in the pedigree, or of a pharmacological response to a therapeutic molecule in the pharmacogenomic studies, or in the studies of case/control association for the analysis of the sporadic cases.

### EXAMPLE 8 : Identification of a causal gene for a disease linked to a deficiency in the transport of cholesterol and inflammatory lipid substances by causal mutation or a transcriptional difference

Among the mutations identified according to the method described in Example 7, all those associated with the disease phenotype are capable of being causal. Validation of these results is made by sequencing the gene in all the affected individuals and their relations (whose DNA is available).

Moreover, Northern blot or RT-PCR analysis, according to the methods described in Example 2, using RNA specific to affected or nonaffected individuals makes it possible to detect notable variations in the level of expression of the gene studied, in particular in the absence of transcription of the gene.

### EXAMPLE 9: Construction of recombinant vectors comprising a nucleic acid encoding any one of ABCA5, ABCA6, ABCA9, and ABCA10 proteins

### Synthesis of a nucleic acid encoding any one of human ABCA5, ABCA6, ABCA9, or ABCA10 proteins:

Total RNA (500 ng) isolated from a human cell ( for example, placental tissue, Clontech, Palo Alto, CA, USA, or THP1 cells) may be used as source for the synthesis of the cDNA of the human ABCA5, ABCA6, ABCA9, and ABCA10 genes. Methods to reverse transcribe mRNA to cDNA are well known in the art. For example, one may use the system "Superscript one step RT-PCR (Life Technologies, Gaithersburg, MD, USA).

Oligonucleotide primers specific for ABCA5, ABCA6, ABCA9, and ABCA10 cDNAs may be used for this purpose, containing sequences as set forth in any of SEQ ID NO: 127-217. These oligonucleotide primers may be synthesized by the phosphoramidite method on a DNA synthesizer of the ABI 394 type (Applied Biosystems, Foster City, CA, USA).

Sites recognized by the restriction enzyme NotI may be incorporated into the amplified ABCA5, ABCA6, ABCA9, and ABCA10 cDNAs to flank the cDNA region desired for insertion into the recombinant vector by a second amplification step using 50 ng of human ABCA5, ABCA6, ABCA9, and ABCA10 cDNAs as template, and 0.25 µM of the ABCA5, ABCA6, ABCA9, and ABCA10 specific oligonucleotide primers used above containing, at their 5' end, the site recognized by the restriction enzyme NotI (5'-GCGGCCGC-3'), in the presence of 200 µM of each of said dideoxynucleotides dATP, dCTP, dTTP and dGTP as well as the *Pyrococcus furiosus* DNA polymerase (Stratagene, Inc. La Jolla, CA, USA).

The PCR reaction may be carried out over 30 cycles each comprising a step of denaturation at 95°C for one minute, a step of renaturation at 50°C for one minute and a step of extension at 72°C for two minutes, in a thermocycler apparatus for PCR (Cetus Perkin Elmer Norwalk, CT, USA).

### Cloning of the cDNA of the human ABCA5, ABCA6, ABCA9, and ABCA10 genes into an expression vector:

The human ABCA5, ABCA6, ABCA9, and ABCA10 cDNA inserts may then be cloned into the NotI restriction site of an expression vector, for example, the pCMV vector containing a cytomegalovirus (CMV) early promoter and an enhancer sequence as well as the SV40 polyadenylation signal (Beg et al., 1990, PNAS, 87:3473; Applebaum-Boden, 1996, JCI 97), in order to produce an expression vector designated pABCA5, pABCA6, pABCA9, and pABCA10.

The sequence of the cloned cDNA can be confirmed by sequencing on the two strands using the reaction set "ABI Prism Big Dye Terminator Cycle Sequencing ready" (marketed by Applied Biosystems, Foster City, CA, USA) in a capillary sequencer of the ABI 310 type (Applied Biosystems, Foster City, CA, USA).

### Construction of a recombinant adenoviral vector containing the cDNA of the human ABCA5, ABCA6, ABCA9, and ABCA10 genes:

### Modification of the expression vector pCMV-β:

The β-galactosidase cDNA of the expression vector pCMV-β (Clontech, Palo Alto, CA, USA, Gene Bank Accession No. UO2451) may be deleted by digestion with the restriction endonuclease NotI and replaced with a multiple cloning site containing, from the 5' end to the 3' end, the following sites: NotI, AscI, RsrII, AvrII, SwaI, and NotI, cloned at the region of the NotI restriction site. The sequence of this multiple cloning site is:
5'-CGGCCGCGGCGCGCCCGGACCGCCTAGGATTTAAATCGCGGCCCGCG-3'.

The DNA fragment between the EcoRI and SanI sites of the modified expression vector pCMV may be isolated and cloned into the modified XbaI site of the shuttle vector pXCXII (McKinnon et al., 1982, Gene, 19:33; McGrory et al., 1988, Virology, 163:614).

### Modification of the shuttle vector pXCXII:

A multiple cloning site comprising, from the 5' end to the 3 end the XbaI, EcoRI, SfiI, PmeI, NheI, SrfI, PacI, Sail and XbaI restriction sites having the sequence:
5'CTCTAGAATTCGGCCTCCGTGGCCGTTTAAACGCTAGCGCCCGGGCTTAATT AAGTCGACTCTAGAGC-3', may be inserted at the level of the XbaI site (nucleotide at position 3329) of the vector pXCXII (McKinnon et al., 1982, Gene 19:33; McGrory et al., 1988, Virology, 163:614).

The EcoRI-SalI DNA fragment isolated from the modified vector pCMV-β containing the CMV promoter/enhancer, the donor and acceptor splicing sites of FV40 and the polyadenylation signal of FV40 may then be cloned into the EcoRI-SalI site of the modified shuttle vector pXCX, designated pCMV-11.

### Preparation of the shuttle vector pAD12-ABCA:

The human ABCA5, ABCA6, ABCA9, and ABCA10 cDNAs are obtained by an RT-PCR reaction, as described above, and cloned at the level of the NotI site into the vector pCMV-12, resulting in the obtaining of the vector pCMV-ABCA5, pCMV-ABCA6, pCMV-ABCA9, and pCMV-ABCA10.

### Construction of the ABC1 recombinant adenovirus:

The recombinant adenovirus containing the human ABCA5, ABCA6, ABCA9, and ABCA10 cDNAs may be constructed according to the technique described by McGrory et al. (1988, Virology, 163:614).

Briefly, the vector pAD12-ABCA is cotransfected with the vector tGM17 according to the technique of Chen and Okayama (1987, Mol Cell Biol., 7:2745-2752).

Likewise, the vector pAD12-Luciferase was constructed and cotransfected with the vector pJM17.

The recombinant adenoviruses are identified by PCR amplification and subjected to two purification cycles before a large-scale amplification in the human embryonic kidney cell line HEK 293 (American Type Culture Collection, Rockville, MD, USA).

The infected cells are collected 48 to 72 hours after their infection with the adenoviral vectors and subjected to five freeze-thaw lysing cycles.

The crude lysates are extracted with the aid of Freon (Halocarbone 113, Matheson Product, Scaucus, N.J. USA), sedimented twice in cesium chloride supplemented with 0.2% murine albumine (Sigma Chemical Co., St Louis, MO, USA) and dialysed extensively against buffer composed of 150 nM NaCl, 10 mM Hepes (pH 7,4), 5 mM KCl, 1 mM MgCl₂, and 1 mM CaCl₂.

The recombinant adenoviruses are stored at -70°C and titrated before their administration to animals or their incubation with cells in culture.

The absence of wild-type contaminating adenovirus is confirmed by screening with the aid of PCR amplification using oligonucleotide primers located in the structural portion of the deleted region.

### Validation of the expression of the human ABCA5, ABCA6, ABCA9, and ABCA10 cDNAs:

Polyclonal antibodies specific for a human ABCA5, ABCA6, ABCA9, and ABCA10 polypeptides may be prepared as described above in rabbits and chicks by injecting a synthetic polypeptide fragment derived from an ABC1 protein, comprising all or part of an amino acid sequence as described in SEQ ID NO:5-8. These polyclonal antibodies are used to detect and/or quantify the expression of the human ABCA5, ABCA6, ABCA9, and ABCA10 genes in cells and animal models by immunoblotting and/or immunodetection.

The biological activity of ABCA5-6, 9-10 may be monitored by quantifying the cholesterol fluxes induced by apoA-I using cells transfected with the vector pCMV-ABCI which have been loaded with cholesterol (Remaley et al., 1997, ATVB, 17:1813).

### Expression in vitro of the human ABCA5, ABCA6, ABCA9, and ABCA10 cDNAs in cells:

Cells of the HEK293 line and of the COS-7 line (American Tissue Culture Collection, Bethesda, MD, USA), as well as fibroblasts in primary culture derived from Tangier patients or from patients suffering from hypo-alphalipoproteinemia are transfected with the expression vector pCMV-ABCA5, pCMV-ABCA6, pCMV-ABCA9, and pCMV-ABCA10 (5-25 µg) using Lipofectamine (BRL, Gaithersburg, MD, USA) or by coprecipitation with the aid of calcium chloride (Chen et al., 1987, Mol Cell Biol., 7:2745-2752).

These cells may also be infected with the vector pABCA5-AdV, pABCA6-AdV, pABCA9-AdV, and pABCA10-AdV (Index of infection, MOI=10).

The expression of human ABCA5-6, 9-10 may be monitored by immunoblotting as well as by quantification of the efflux of cholesterol induced by apoA-1 using transfected and/or infected cells.

### Expression in vivo of the ABCA5, ABCA6, ABCA9, and ABCA10 genes in various animal models:

An appropriate volume (100 to 300 µl) of a medium containing the purified recombinant adenovirus (pABCA-AdV or pLucif-AdV) containing from 10⁸ to 10⁹ lysis plaque-forming units (pfu) are infused into the Saphenous vein of mice (C57BL/6, both control mice and models of transgenic or knock-out mice) on day 0 of the experiment.

The evaluation of the physiological role of the ABCA5, ABCA6, ABCA9, and ABCA10 proteins in the transport of cholesterol or inflammatory lipid substances is carried out by determining the total quantity of cholesterol or appropriate inflammatory lipid substances before (day zero) and after (days 2, 4, 7, 10, 14) the administration of the adenovirus.

Kinetic studies with the aid of radioactively labelled products are carried out on day 5 after the administration of the vectors rLucif-AdV and rABCA-AdV in order to evaluate the effect of the expression of ABCA5, ABCA6, ABCA9, and ABCA10 on the transport of cholesterol and inflammatory lipid substances.

Furthermore, transgenic mice and rabbits overexpressing the ABCA5, ABCA6, ABCA9, and ABCA10 genes may be produced, in accordance with the teaching of Vaisman (1995) and Hoeg (1996) using constructs containing the human ABCA5, ABCA6, ABCA9, and ABCA10 cDNAs under the control of endogenous promoters such as ABCA5, ABCA6, ABCA9, and ABCA10, or CMV or apoE.

The evaluation of the long-term effect of the expression of ABCA5, ABCA6, ABCA9, and ABCA10 on the kinetics of the lipids involved in the mediation of the inflammation may be carried out as described above.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

## Claims

1. An isolated nucleic acid comprising any one of SEQ ID NOs: 1-4 and 9-126, or of a complementary nucleotide sequence.

2. An isolated nucleic acid comprising at least eight consecutive nucleotides of a nucleotide sequence of any one of SEQ ID NOs: 1-4 and 9-126, or of a complementary nucleotide sequence.

3. An isolated nucleic acid comprising at least 80% nucleotide identity with a nucleic acid comprising any one of SEQ ID NOs: 1-4 and 9-126, or of a complementary nucleotide sequence.

4. The isolated nucleic acid according to claim 3, wherein the nucleic acid comprises an 85%, 90%, 95%, or 98% nucleotide identity with the nucleic acid comprising any one of SEQ ID NOs: 1-4 and 9-126, or of a complementary nucleotide sequence.

5. An isolated nucleic acid that hybridizes under high stringency conditions with a nucleic acid comprising a) any one of SEQ ID NOs: 1-4 and 9-126, or of a complementary nucleotide sequence.

6. An isolated nucleic acid comprising a nucleotide sequence as depicted in any one of SEQ ID NOs: 1-4 and 9-126, or of a complementary nucleotide sequence.

7. A nucleotide probe or primer specific for any one of ABCA5, ABCA6, ABCA9, and ABCA10 genes, wherein the nucleotide probe or primer comprises at least 15 consecutive nucleotides of a nucleotide sequence of any one of SEQ ID NOs: 1-4 and 9-126, or of a complementary nucleotide sequence.

8. A nucleotide probe or primer specific for an ABCA5 gene, wherein the nucleotide probe or primer comprises a nucleotide sequence of any one of SEQ ID NO:127-144, or a complementary nucleotide sequence.

9. A nucleotide probe or primer specific for an ABCA6 gene, wherein the nucleotide probe or primer comprises a nucleotide sequence of any one of SEQ ID NOs: 145-172, or of a complementary nucleotide sequence.

10. A nucleotide probe or primer specific for an ABCA9 gene, wherein the nucleotide probe or primer comprises a nucleotide sequence of any one of SEQ ID NOs: 173-203, or of a complementary nucleotide sequence.

11. A nucleotide probe or primer specific for an ABCA10 gene, wherein the nucleotide probe or primer comprises a nucleotide sequence of any one of SEQ ID NOs: 204-217, or of a complementary nucleotide sequence.

12. A method of amplifying a region of the nucleic acid according to claim 1, wherein the method comprises:
a) contacting the nucleic acid with two nucleotide primers, wherein the first nucleotide primer hybridizes at a position 5' of the region of the nucleic acid, and the second nucleotide primer hybridizes at a position 3' of the region of the nucleic acid, in the presence of reagents necessary for an amplification reaction; and
b) detecting the amplified nucleic acid region.

13. A method of amplifying a region of the nucleic acid according to claim 12, wherein the two nucleotide primers are selected from the group consisting of
a) a nucleotide primer comprising at least 15 consecutive nucleotides of a nucleotide sequence of any one of SEQ ID NOs: 1-4 and 9-126, or of a complementary nucleotide sequence,
b) a nucleotide primer as in any one of claims 7-11,
c) a nucleotide primer comprising a nucleotide sequence of any one of SEQ ID NOs: 127-217, or a nucleic acid having a complementary sequence.

14. A kit for amplifying the nucleic acid according to claim 1, wherein the kit comprises:
a) two nucleotide primers whose hybridization position is located respectively 5' and 3' of the region of the nucleic acid; and optionally,
b) reagents necessary for an amplification reaction.

15. The kit according to claim 14, wherein the two nucleotide primers are selected from the group consisting of
a) a nucleotide primer comprising at least 15 consecutive nucleotides of a nucleotide sequence of any one of SEQ ID NOs: 1-4 and 9-126, or of a complementary nucleotide sequence,
b) a nucleotide primer as in any one of claims 7-11,
c) a nucleotide primer comprising a nucleotide sequence of any one of SEQ ID NOs: 127-217, or a nucleic acid having a complementary sequence.

16. The nucleotide probe or primer according to any of claim 7-11, wherein the nucleotide probe or primer comprises a marker compound.

17. A method of detecting a nucleic acid according to claim 1, wherein the method comprises:
a) contacting the nucleic acid with a nucleotide probe selected from the group consisting of
1) a nucleotide probe comprising at least 15 consecutive nucleotides of a nucleotide sequence of any one of SEQ ID NOs: 1-4 and 9-126, or of a complementary nucleotide sequence,
2) a nucleotide primer as in any one of claims 7-11,
3) a nucleotide probe comprising a nucleotide sequence of any one of SEQ ID NOs: 127-217, or of a complementary nucleotide sequence, and
b) detecting a complex formed between the nucleic acid and the probe.

18. The method of detection according to claim 17, wherein the probe is immobilized on a support.

19. A kit for detecting the nucleic acid according to claim 1, wherein the kit comprises
a) a nucleotide probe selected from the group consisting of
1) a nucleotide probe comprising at least 15 consecutive nucleotides of a nucleotide sequence of any one of SEQ ID NOs: 1-4 and 9-126, or of a complementary nucleotide sequence,
2) a nucleotide primer as in any one of claims 7-11,
3) a nucleotide probe comprising a nucleotide sequence of any one of SEQ ID NOs: 127-217, or of a complementary nucleotide sequence, and optionally,
b) reagents necessary for a hybridization reaction.

20. The kit according to claim 19, wherein the probe is immobilized on a support.

21. A recombinant vector comprising the nucleic acid according claim 1.

22. The vector according to claim 21, wherein the vector is an adenovirus.

23. A recombinant host cell comprising the recombinant vector according to claim 21.

24. A recombinant host cell comprising the nucleic acid according claim 1.

25. An isolated nucleic acid encoding a polypeptide comprising an amino acid sequence of any one of SEQ ID NO: 5-8.

26. A recombinant vector comprising the nucleic acid according to claim 25.

27. A recombinant host cell comprising the nucleic acid according to claim 25.

28. A recombinant host cell comprising the recombinant vector according to claim 26.

29. An isolated polypeptide selected from the group consisting of
a) a polypeptide comprising an amino acid sequence of any one of SEQ ID NOs: 5-8,
b) a polypeptide fragment or variant of a polypeptide comprising an amino acid sequence of any one of SEQ ID NOs: 5-8, and
c) a polypeptide homologous to a polypeptide comprising amino acid sequence of any one of SEQ ID NO: 5-8.

30. An antibody directed against the isolated polypeptide according to claim 29.

31. The antibody according to claim 30, wherein the antibody comprises a detectable compound.

32. A method of detecting a polypeptide, wherein the method comprises
a) contacting the polypeptide with an antibody according to claim 31; and
b) detecting an antigen/antibody complex formed between the polypeptide and the antibody.

33. A diagnostic kit for detecting a polypeptide, wherein the kit comprises
a) the antibody according to claim 31; and
b) a reagent allowing detection of an antigen/antibody complex formed between the polypeptide and the antibody.

34. A pharmaceutical composition comprising the nucleic acid according to claim 1 and a physiologically compatible excipient.

35. A pharmaceutical composition comprising the recombinant vector according to claim 21 and a physiologically compatible excipient.

36. Use of the nucleic acid according to claim 1 for the manufacture of a medicament intended for the prevention and/or treatment of a subject affected by a dysfunction in the reverse transport of cholesterol.

37. Use of a recombinant vector according to claim 21 for the manufacture of a medicament for the prevention and/or treatment of subjects affected by a dysfunction in the lipophilic subtance transport.

38. Use of any one of isolated ABCA5, ABCA6, ABCA9, and ABCA10 polypeptides comprising an amino acid sequence of SEQ ID NO: 5-8 for the manufacture of a medicament intended for the prevention and/or treatment of subjects affected by a dysfunction in the lipophilic subtance transport.

39. A pharmaceutical composition comprising a polypeptide comprising an amino acid sequence of any one of SEQ ID NOs: 5-8, and a physiologically compatible excipient.

40. Use of any one of isolated ABCA5, ABCA6, ABCA9, and ABCA10 polypeptides comprising an amino acid sequence of any one of SEQ ID NOs: 5-8 for screening an active ingredient for the prevention or treatment of a disease resulting from a dysfunction in the lipophilic subtance transport..

41. Use of a recombinant host cell expressing any one of the ABCA5, ABCA6, ABCA9, and ABCA10 polypeptides comprising an amino acid sequence of SEQ ID NOs: 5-8 for screening an active ingredient for the prevention or treatment of a disease resulting from a dysfunction in the lipophilic subtance transport..

42. A method of screening a compound active on cholesterol metabolism, an agonist, or an antagonist of any one of the ABCA5, ABCA6, ABCA9, and ABCA10 polypeptides, wherein the method comprises
a) preparing a membrane vesicle comprising at least one of the ABCA5, ABCA6, ABCA9, and ABCA10 polypeptides and a lipid substrate comprising a detectable marker;
b) incubating the vesicle obtained in step a) with an agonist or antagonist candidate compound;
c) qualitatively and/or quantitatively measuring a release of the lipid substrate comprising the detectable marker; and
d) comparing the release of the lipid substrate measured in step b) with a measurement of a release of a labeled lipid substrate by a membrane vesicle that has not been previously incubated with the agonist or antagonist candidate compound.

43. A method of screening a compound active on cholesterol metabolism, an agonist, or an antagonist of any one of ABCA5, ABCA6, ABCA9, and ABCA10 polypeptides, wherein the method comprises
a) incubating a cell that expresses at least one of the ABCA5, ABCA6, ABCA9, and ABCA10 polypeptides with an anion labeled with a detectable marker;
b) washing the cell of step a) whereby excess labeled anion that has not penetrated into the cell is removed;
c) incubating the cell obtained in step b) with an agonist or antagonist candidate compound for any one of the ABCA5, ABCA6, ABCA9, and ABCA10 polypeptide;
d) measuring efflux of the labeled anion from the cell; and
e) comparing the efflux of the labeled anion determined in step d) with efflux of a labeled anion measured with a cell that has not been previously incubated with the agonist or antagonist candidate compound..

44. An implant comprising the recombinant host cell according to claim 23.


LISTE DE SEQUENCES

<110> AVENTIS PHARMA

<120> NUCLEIC ACIDS OF THE HUMAN ABCA5, ABCA6, ABCA9, ABCA10 GENES,
VECTORS CONTAINING SUCH NUCLEIC ACIDS AND USES THEREOF

<130> ABCA5, 6, 9, 10

<140> ST00034
<141> 2000-12-06

<160> 217

<170> PatentIn Ver. 2.1

<210> 1
<211> 6525
<212> ADN
<213> Homo sapiens

<400> 1
aaaatgttga tattttctct tagcaggctg tcaaccaggt taggttcagg tcataagttt 60
ctacccacat tctttgaact gtagttgtca ttttagttta tttttcaaaa acttttgcag 120
tacctttttg gtctgtcttg tgtgtgcctt gcagtgaaca gtctggattt ggacagtggt 180
ctgtctgtta gttcagtttc tcaagccttt gtcacactaa taggattgga tttatgtatg 240
tccagcttgg gaattattac aggaattaaa aacaactttt tagagtgctt tcctgagctc 300
tctttctatt tgttccccct tctacttttt gcttccctgt ggctgctgtt tctatcctcc 360
agccagagag ctagtgttta ttttctccat tgtgttacac acttgtgcag ctgcaaccac 420
catatccagg gcccaatggt aggaggtaga gaagaaaagc aaaagggatt ggcctcatcc 480
tcttacaacg atagttccat tgaatagaga gaaaggtttt cctgcctcag agtgttggct 540
gcactaggct tttgttactg tagtctggcc ctgttaccat gggattgctt gcatgtgggg 600
atacaggaga attcagaaaa gaaaaaaaga tttgctattt ctacattctc cctgagcatt 660
aagacctccc ttgcccattc ctcaattcaa agctaaggct tcttctggag ctgcctctgt 720
gggcggttcg ggagatacca aaggagaaaa agtaccactg ttgatatggt ggtatttcaa 780
attctggtct accctatttc acatgccttg tttacttttc agagctgaca gattgctgct 840
ccatgcattc tgtccagttt cctaagagag acagcttgga gtatgcttaa tccatcttac 900
ctgggactga aacagctgct tattttgccg ttaaaaatta catgcagttt actgcgtggc 960
tccgggtttg tttgtttgtt tttcctcttt aataggttta ttcagaaaac atgtccactg 1020
caattaggga ggtaggagtt tggagacaga ccagaacact tctactgaag aattacttaa 1080
ttaaatgcag aaccaaaaag agtagtgttc aggaaattct ttttccacta ttttttttat 1140
tttggttaat attaattagc atgatgcatc caaataagaa atatgaagaa gtgcctaata 1200
tagaactcaa tcctatggac aagtttactc tttctaatct aattcttgga tatactccag 1260
tgactaatat tacaagcagc atcatgcaga aagtgtctac tgatcatcta cctgatgtca 1320
taattactga agaatataca aatgaaaaag aaatgttaac atccagtctc tctaagccga 1380
gcaactttgt aggtgtggtt ttcaaagact ccatgtccta tgaacttcgt ttttttcctg 1440
atatgattcc agtatcttct atttatatgg attcaagagc tggctgttca aaatcatgtg 1500
aggctgctca gtactggtcc tcaggtttca cagttttaca agcatccata gatgctgcca 1560
ttatacagtt gaagaccaat gtttctcttt ggaaggagct ggagtcaact aaagctgtta 1620
ttatgggaga aactgctgtt gtagaaatag atacctttcc ccgaggagta attttaatat 1680
acctagttat agcattttca ccttttggat actttttggc aattcatatc gtagcagaaa 1740
aagaaaaaaa aataaaagaa tttttaaaga taatgggact tcatgatact gccttttggc 1800
tttcctgggt tcttctatat acaagtttaa tttttcttat gtcccttctt atggcagtca 1860
ttgcgacagc ttctttgtta tttcctcaaa gtagcagcat tgtgatattt ctgctttttt 1920
tcctttatgg attatcatct gtattttttg ctttaatgct gacacctctt tttaaaaaat 1980
caaaacatgt gggaatagtt gaattttttg ttactgtggc ttttggattt attggcctta 2040
tgataatcct catagaaagt tttcccaaat cgttagtgtg gcttttcagt cctttctgtc 2100
actgtacttt tgtgattggt attgcacagg tcatgcattt agaagatttt aatgaaggtg 2160
cttcattttc aaatttgact gcaggcccat atcctctaat tattacaatt atcatgctca 2220
cacttaatag tatattctat gtcctcttgg ctgtctatct tgatcaagtc attccagggg 2280
aatttggctt acggagatca tctttatatt ttctgaagcc ttcatattgg tcaaagagta 2340
aaagaaatta tgaggagtta tcagagggca atgttaatgg aaatattagt tttagtgaaa 2400
ttattgagcc agtttcttca gaatttgtag gaaaagaagc cataagaatt agtggtattc 2460
agaagacata cagaaagaag ggtgaaaatg tggaggcttt gagaaatttg tcatttgaca 2520
tatatgaggg tcagattact gccttacttg gccacagtgg aacaggaaag agtacattga 2580
tgaatattct ttgtggactc tgcccacctt ctgatgggtt tgcatctata tatggacaca 2640
gagtctcaga aatagatgaa atgtttgaag caagaaaaat gattggcatt tgtccacagt 2700
tagatataca ctttgatgtt ttgacagtag aagaaaattt atcaattttg gcttcaatca 2760
aagggatacc agccaacaat ataatacaag aagtgcagaa ggttttacta gatttagaca 2820
tgcagactat caaagataac caagctaaaa aattaagtgg tggtcaaaaa agaaagctgt 2880

cattaggaat tgctgttctt gggaacccaa agatactgct gctagatgaa ccaacagctg 2940
gaatggaccc ctgttctcga catattgtat ggaatctttt aaaatacaga aaagccaatc 3000
gggtgacagt gttcagtact catttcatgg atgaagctga cattcttgca gataggaaag 3060
ctgtgatatc acaaggaatg ctgaaatgtg ttggttcttc aatgttcctc aaaagtaaat 3120
gggggatcgg ctaccgcctg agcatgtaca tagacaaata ttgtgccaca gaatctcttt 3180
cttcactggt taaacaacat atacctggag ctactttatt acaacagaat gaccaacaac 3240
ttgtgtatag cttgcctttc aaggacatgg acaaattttc aggtttgttt tctgccctag 3300
acagtcattc aaatttgggt gtcatttctt atggtgtttc catgacgact ttggaagacg 3360
tatttttaaa gctagaagtt gaagcagaaa ttgaccaagc agattatagt gtatttactc 3420
agcagccact ggaggaagaa atggattcaa aatcttttga tgaaatggaa cagagcttac 3480
ttattctttc tgaaaccaag gcttctctag tgagcaccat gagcctttgg aaacaacaga 3540
tgtatacaat agcaaagttt catttcttta ccttgaaacg tgaaagtaaa tcagtgagat 3600
cagtgttgct tctgctttta atttttttca cagttcagat tttatgtttt ttggttcatc 3660
actcttttaa aaatgctgtg gttcccatca aacttgttcc agacttatat tttctaaaac 3720
ctggagacaa accacataaa tacaaaacaa gtctgcttct tcaaaattct gctgactcag 3780
atatcagtga tcttattagc tttttcacaa gccagaacat aatggtgacg atgattaatg 3840
acagtgacta tgtatccgtg gctccccata gtgcggcttt aaatgtgatg cattcagaaa 3900
aggactatgt ttttgcagct gttttcaaca gtactatggt ttattcttta cctatattag 3960
tgaatatcat tagtaactac tatctttatc atttaaatgt gactgaaacc atccagatct 4020
ggagtacccc attctttcaa gaaattactg atatagtttt taaaattgag ctgtattttc 4080
aagcagcttt gcttggaatc attgttactg caatgccacc ttactttgcc atggaaaatg 4140
cagagaatca taagatcaaa gcttatactc aacttaaact ttcaggtctt ttgccatctg 4200
catattggat tggacaagct gttgttgata tccccttatt ttttatcatt cttattttga 4260
tgctaggaag cttactggca tttcattatg gattatattt ttatactgta aagttccttg 4320
ctgtggtttt ttgccttatt ggttatgttc catcagttat tctgttcact tatattgctt 4380
ctttcacctt taagaaaatt ttaaatacca aagaattttg gtcatttatc tattctgtgg 4440
cagcgttgnc ttgtattgca atcactgaaa taactttctt tatgggatac acaattgcaa 4500
ctattcttca ttatgccttt tgtatcatca ttccaatcta tccacttcta ggttgcctga 4560
tttctttcat aaagatttct tggaagaatg tacgaaaaaa tgtggacacc tataatccat 4620
gggataggct ttcagtagct gttatatcgc cttacctgca gtgtgtactg tggattttcc 4680
tcttacaata ctatgagaaa aaatatggag gcagatcaat aagaaaagat ccctttttca 4740
gaaacctttc aacgaagtct aaaaatagga agcttccaga accaccagac aatgaggatg 4800
aagatgaaga tgtcaaagct gaaagactaa aggtcaaaga gctgatgggt tgccagtgtt 4860
gtgaggagaa accatccatt atggtcagca atttgcataa agaatatgat gacaagaaag 4920
attttcttct ttcaagaaaa gtaaagaaag tggcaactaa atacatctct ttctgtgtga 4980
aaaaaggaga gatcttagga ctattgggtc caaatggtgc tggcaaaagc acaattatta 5040
atattctggt tggtgatatt gaaccaactt caggccaggt atttttagga gattattctt 5100
cagagacaag tgaagatgat gattcactga agtgtatggg ttactgtcct cagataaacc 5160
ctttgtggcc agatactaca ttgcaggaac attttgaaat ttatggagct gtcaaaggaa 5220
tgagtgcaag tgacatgaaa gaagtcataa gtcgaataac acatgcactt gatttaaaag 5280
aacatcttca gaagactgta aagaaactac ctgcaggaat caaacgaaag ttgtgttttg 5340
ctctaagtat gctagggaat cctcagatta ctttgctaga tgaaccatct acaggtatgg 5400
atcccaaagc caaacagcac atgtggcgag caattcgaac tgcatttaaa aacagaaagc 5460
gggctgctat tctgaccact cactatatgg aggaggcaga ggctgtctgt gatcgagtag 5520
ctatcatggt gtctgggcag ttaagatgta tcggaacagt acaacatcta aagagtaaat 5580
ttggaaaagg ctactttttg gaaattaaat tgaaggactg gatagaaaac ctagaagtag 5640
accgccttca aagagaaatt cagtatattt tcccaaatgc aagccgtcag gaaagttttt 5700
cttctatttt ggcttataaa attcctaagg aagatgttca gtccctttca caatcttttt 5760
ttaagctgga agaagctaaa catgcttttg ccattgaaga atatagcttt tctcaagcaa 5820
cattggaaca ggtttttgta gaactcacta aagaacaaga ggaggaagat aatagttgtg 5880
gaactttaaa cagcacactt tggtgggaac gaacacaaga agatagagta gtattttgaa 5940
tttgtattgt tcggtctgct tactgggact tctttctttt tcacttaatt ttaactttgg 6000
tttaaaaagt tttttattgg aatggtaact ggagaaccaa gaacgcactt gaaatttttc 6060
taagctcctt aattgaaatg ctgtggttgt gtgttttgct tttctttaaa taaaacgtat 6120
gtataattaa gtgaagctgc atgtttgtat tgaagtatat tgaactatat agtttgtatg 6180
tcatcttttt caccattcag aaacagtgct tctgaatttg tgatttaaag gaattgtaat 6240
agaatagttt tatttttaag ttatctttaa gtttatgcca tcttcttaaa taagtacgta 6300
atgttccaat ctaaataaaa aactaataca taactaatgc atagaaaaga tacataaagc 6360
aatgtgaaag tttcttgctt ctccttttta atttctaaaa aagccacttt gaatggaagt 6420
tgtcatccgt aaaagctgaa gtgtaagcac taggaaatct caatatagag atttgaggaa 6480
agttatatcc actaggtggc agtcattgat cataataagt gaaat 6525

<210> 2
<211> 5296
<212> ADN
<213> Homo sapiens

<400> 2
ctgctggagt aggcacccat ttaaagaaaa aatgaagaag cagcaataaa gaagttgtaa 60

tcgttaccta gacaaacaga gaactggttt tgacagtgtt tctagagtgc tttttattat 120
tttcctgaca gttgtgttcc accatgatta ctttctcctt cagcgaatag gctaaatgaa 180
tatgaaacag aaaagcgtgt atcagcaaac caaagcactt ctgtgcaaga attttcttaa 240
gaaatggagg atgaaaagag agagcttatt ggaatggggc ctctcaatac ttctaggact 300
gtgtattgct ctgttttcca gttccatgag aaatgtccag tttcctggaa tggctcctca 360
gaatctggga agggtagata aatttaatag ctcttcttta atggttgtgt atacaccaat 420
atctaattta acccagcaga taatgaataa aacagcactt gctcctcttt tgaaaggaac 480
aagtgtcatt ggggcaccaa ataaaacaca catggacgaa atacttctgg aaaatttacc 540
atatgctatg ggaatcatct ttaatgaaac tttctcttat aagttaatat ttttccaggg 600
atataacagt ccactttgga aagaagattt ctcagctcat tgctgggatg gatatggtga 660
gttttcatgt acattgacca aatactggaa tagaggattt gtggctttac aaacagctat 720
taatactgcc attatagaaa tcacaaccaa tcaccctgtg atggaggagt tgatgtcagt 780
tactgctata actatgaaga cattaccttt cataactaaa aatcttcttc acaatgagat 840
gtttatttta ttcttcttgc ttcatttctc cccacttgta tattttatat cactcaatgt 900
aacaaaagag agaaaaaagt ctaagaattt gatgaaaatg atgggtctcc aagattcagc 960
attctggctc tcctggggtc taatctatgc tggcttcatc tttattattt ccatattcat 1020
tacaattatc ataacattca cccaaattat agtcatgact ggcttcatgg tcatatttat 1080
actctttttt ttatatggct tatctttggt agctttggtg ttcctgatga gtgtgctgtt 1140
aaagaaagct gtcctcacca atttggttgt gtttctcctt accctctttt ggggatgtct 1200
gggattcact gtattttatg aacaacttcc ttcatctctg gagtggattt tgaatatttg 1260
tagccctttt gcctttacta ctggaatgat tcagattatc aaactggatt ataacttgaa 1320
tggtgtaatt tttcctgacc cttcaggaga ctcatataca atgatagcaa ctttttctat 1380
gttgcttttg gatggtctca tctacttgct attggcatta tactttgaca aaattttacc 1440
ctatggagat gagcgccatt attctccttt atttttcttg aattcatcat cttgtttcca 1500
acaccaaagg actaatgcta aggttattga gaaagaaatc gatgctgagc atccctctga 1560
tgattatttt gaaccagtag ctcctgaatt ccaaggaaaa gaagccatca gaatcagaaa 1620
tgttaagaag gaatataaag gaaaatctgg aaaagtggaa gcattgaaag gcttgctctt 1680
tgacatatat gaaggtcaaa tcacggcaat cctgggtcac agtggagctg gcaaatcttc 1740
actgctaaat attcttaatg gattgtctgt tccaacagaa ggatcagtta ccatctataa 1800
taaaaatctc tctgaaatgc aagacttgga ggaaatcaga aagataactg gcgtctgtcc 1860
tcaattcaat gttcaatttg acatactcac cgtgaaggaa aacctcagcc tgtttgctaa 1920
aataaaaggg attcatctaa aggaagtgga acaagaggta caacgaatat tattggaatt 1980
ggacatgcaa aacattcaag ataaccttgc taaacattta agtgaaggac agaaaagaaa 2040
gctgactttt gggattacca ttttaggaga tcctcaaatt ttgcttttag atgaaccaac 2100
tactggattg gatccctttt ccagagatca agtgtggagc ctcctgagag agcgtagagc 2160
agatcatgtg atccttttca gtacccagtc catggatgag gctgacatcc tggctgatag 2220
aaaagtgatc atgtccaatg ggagactgaa gtgtgcaggt tcttctatgt ttttgaaaag 2280
aaggtggggt cttggatatc acctaagttt acataggaat gaaatatgta acccagaaca 2340
aataacatcc ttcattactc atcacatccc cgatgctaaa ttaaaaacag aaaacaaaga 2400
aaagcttgta tatactttgc cactggaaag gacaaataca tttccagatc ttttcagtga 2460
tctggataag tgttctgacc agggagtgac aggttatgac atttccatgt caactctaaa 2520
tgaagtcttt atgaaactgg aaggacagtc aactatcgaa caagatttcg aacaagtgga 2580
gatgataaga gactcagaaa gcctcaatga aatggagctg gctcactctt ccttctctga 2640
aatgcagaca gctgtgagtg acatgggcct ctggagaatg caagtctttg ccatggcacg 2700
gctccgtttc ttaaagttaa aacgtcaaac taaagtgtta ttgaccctat tattggtatt 2760
tggaatcgca atattccctt tgattgttga aaatataata tatgctatgt taaatgaaaa 2820
gatcgattgg gaatttaaaa acgaattgta ttttctctct cctggacaac ttccccagga 2880
accccgtacc agcctgttga tcatcaataa cacagaatca aatattgaag attttataaa 2940
atcactgaag catcaaaata tacttttgga agtagatgac tttgaaaaca gaaatggtac 3000
tgatggcctc tcatacaatg gagctatcat agtttctggt aaacaaaagg attatagatt 3060
ttcagttgtg tgtaatacca agagattgca ctgttttcca attcttatga atattatcag 3120
caatgggcta cttcaaatgt ttaatcacac acaacatatt cgaattgagt caagcccatt 3180
tcctcttagc cacataggac tctggactgg gttgccggat ggttcctttt tcttattttt 3240
ggttctatgt agcatttctc cttatatcac catgggcagc atcagtgatt acaagaaaaa 3300
tgctaagtcc cagctatgga tttcaggcct ctacacttct gcttactggt gtgggcaggc 3360
actagtggac gtcagcttct tcattttaat tctcctttta atgtatttaa ttttctacat 3420
agaaaacatg cagtaccttc ttattacaag ccaaattgtg tttgctttgg ttatagttac 3480
tcctggttat gcagcttctc ttgtcttctt catatatatg atatcattta tttttcgcaa 3540
aaggagaaaa aacagtggcc tttggtcatt ttacttcttt tttgcctcca ccatcatgtt 3600
ttccatcact ttaatcaatc attttgacct aagtatattg attaccacca tggtattggt 3660
tccttcatat accttgcttg gatttaaaac ttttttggaa gtgagagacc aggagcacta 3720
cagagaattt ccagaggcaa attttgaatt gagtgccact gattttctag tctgcttcat 3780
accctacttt cagactttgc tattcgtttt tgttctaaga tgcatggaac taaaatgtgg 3840
aaagaaaaga atgcgaaaag atcctgtttt cagaatttcc ccccaaagta gagatgctaa 3900
gccaaatcca gaagaaccca tagatgaaga tgaagatatt caaacagaaa gaataagaac 3960
agccactgct ctgaccactt caatcttaga tgagaaacct gttataattg ccagctgtct 4020
acacaaagaa tatgcaggcc agaagaaaag ttgcttttca aagaggaaga agaaaatagc 4080
agcaagaaat atctctttct gtgttcaaga aggtgaaatt ttgggattgc taggacccag 4140
tggtgctgga aaaagttcat ctattagaat gatatctggg atcacaaagc caactgctgg 4200
agaggtggaa ctgaaaggct gcagttcagt tttgggccac ctggggtact gccctcaaga 4260

gaacgtgctg tggcccatgc tgacgttgag ggaacacctg gaggtgtatg ctgccgtcaa 4320
ggggctcagg aaagcggacg cgaggctcgc catcgcaaga ttagtgagtg ctttcaaact 4380
gcatgagcag ctgaatgttc ctgtgcagaa attaacagca ggaatcacga gaaagttgtg 4440
ttttgtgctg agcctcctgg gaaactcacc tgtcttgctc ctggatgaac catctacggg 4500
catagacccc acagggcagc agcaaatgtg gcaggcaatc caggcagtcg ttaaaaacac 4560
agagagaggt gtcctcctga ccacccataa cctggctgag gcggaagcct tgtgtgaccg 4620
tgtggccatc atggtgtctg gaaggcttag atgcattggc tccatccaac acctgaaaaa 4680
caaacttggc aaggattaca ttctagagct aaaagtgaag gaaacgtctc aagtgacttt 4740
ggtccacact gagattctga agcttttccc acaggctgca gggcaggaaa ggtattcctc 4800
tttgttaacc tataagctgc ccgtggcaga cgtttaccct ctatcacaga cctttcacaa 4860
attagaagca gtgaagcata actttaacct ggaagaatac agcctttctc agtgcacact 4920
ggagaaggta ttcttagagc tttctaaaga acaggaagta ggaaattttg atgaagaaat 4980
tgatacaaca atgagatgga aactcctccc tcattcagat gaaccttaaa acctcaaacc 5040
tagtaatttt ttgttgatct cctataaact tatgttttat gtaataatta atagtatgtt 5100
taattttaaa gatcatttaa aattaacatc aggtatattt tgtaaattta gttaacaaat 5160
acataaattt taaaattatt cttcctctca aacatagggg tgatagcaaa cctgtgataa 5220
aggcaataca aaatattagt aaagtcaccc aaagagtcag gcactgggta ttgtggaaat 5280
aaaactatat aaactt 5296

<210> 3
<211> 5981
<212> ADN
<213> Homo sapiens

<400> 3
   attcacaatg aatgtgaaat taaaagcatg atgtagtagt gacccaaaag gaatgtgaat 60
   tctcctccag aacatgcaga gacccatgga tgaactgtgt ttctagattt ttcctccagc 120
   tttcctgaga gaaacaggtc aaaatgagca agagacgcat gagcgtgggt cagcaaacat 180
   gggctcttct ctgcaagaac tgtctcaaaa aatggagaat gaaaagacag accttgttgg 240
   aatggctctt ttcatttctt ctggtactgt ttctgtacct atttttctcc aatttacatc 300
   aagttcatga cactcctcaa atgtcttcaa tggatctggg acgtgtagat agttttaatg 360
   atactaatta tgttattgca tttgcacctg aatccaaaac tacccaagag ataatgaaca 420
   aagtggcttc agccccattc ctaaaggaaa gaacaatcat ggggtggcct gatgaaaaaa 480
   gcatggatga attggatttg aactattcaa tagacgcagt gagagtcatc tttactgata 540
   ccttctccta ccatttgaag ttttcttggg gacatagaat ccccatgatg aaagagcaca 600
   gagaccattc agctcactgt caagcagtga atgaaaaaat gaagtgtgaa ggttcagagt 660
   tctgggagaa aggctttgta gcttttcaag ctgccattaa tgctgctatc atagaaatcg 720
   caacaaatca ttcagtgatg gaacagctga tgtcagttac tggtgtacat atgaagatat 780
   taccttttgt tgcccaagga ggagttgcaa ctgatttttt catttctttt tgcattattt 840
   ctttttctac atttatatac tatgtatcag tcaatgttac acaagaaaga caatacatta 900
   cgtcattgat gacaatgatg ggactccgag agtcagcatt ctggctttcc tggggtttga 960
   tgtatgctgg cttcatcctt atcatggcca ctttaatggc tcttattgta aaatctgcac 1020
   aaattgtcgt cctgactggt tttgtgatgg tcttcaccct ctttctcctc tatggcctgt 1080
   ctttgataac tttagctttc ctgatgagtg tgttgataaa gaaacctttc cttacgggct 1140
   tggttgtgtt tctccttatt gtcttttggg ggatcctggg attcccagca ttgtatacac 1200
   atcttcctgc atttttggaa tggactttgt gtcttcttag cccctttgcc ttcactgttg 1260
   ggatggccca gcttatacat ttggactatg atgtgaattc taatgcccac ttggattctt 1320
   cacaaaatcc atacctcata atagctactc ttttcatgtt ggtttttgac acccttctgt 1380
   atttggtatt gacattatat tttgacaaaa ttttgcccgc tgaatatgga catcgatgtt 1440
   ctcccttgtt tttcctgaaa tcctgttttt ggtttcaaca cggaagggct aatcatgtgg 1500
   tccttgagaa tgaaacagat tctgatccta cacctaatga ctgttttgaa ccagtgtctc 1560
   cagaattctg tgggaaggaa gccatcagaa tcaaaaatct taaaaaagaa tatgcaggga 1620
   agtgtgagag agtagaagct ttgaaaggtg tggtgtttga catatatgaa ggccagatca 1680
   ctgccctcct tggtcacagt ggagctggaa aaactaccct gttaaacata cttagtgggt 1740
   tgtcagttcc aacatcaggt tcagtcactg tctataatca cacactttca agaatggctg 1800
   atatagaaaa tatcagcaag ttcactggat tttgtccaca atccaatgtg caatttggat 1860
   ttctcactgt gaaagaaaac ctcaggctgt ttgctaaaat aaaagggatt ttgccacatg 1920
   aagtggagaa agaggtacaa cgagttgtac aggaattaga aatggaaaat attcaagaca 1980
   tccttgctca aaacttaagt ggtggacaaa ataggaaact aacttttggg attgccattt 2040
   taggagatcc tcaagttttg ctattggatg aaccgactgc tggattggat cctctttcaa 2100
   ggcaccgaat atggaatctc ctgaaagagg ggaaatcaga cagagtaatt ctcttcagca 2160
   cccagtttat agatgaggct gacattctgg cggacaggaa ggtgttcata tccaatggga 2220
   agctgaagtg tgcaggctct tctctgttcc ttaagaagaa atggggcata ggctaccatt 2280
   taagtttgca tctgaatgaa aggtgtgatc cagagagtat aacatcactg gttaagcagc 2340
   acatctctga tgccaaattg acagcacaaa gtgaagaaaa acttgtatat attttgcctt 2400
   tggaaaggac aaacaaattt ccagaacttt acaggatctt tgatagatgt tctaaccaag 2460
   gcattgagga ttatggtgtt tccataacaa ctttgaatga ggtgtttctg aaattagaag 2520
   gaaaatcaac tattgatgaa tcagatattg gaatttgggg acaattacaa actgatgggg 2580
   caaaagatat aggaagcctt gttgagctgg aacaagtttt gtcttccttc cacgaaacaa 2640
   ggaaaacaat cagtggcgtg gcgctctgga ggcagcaggt ctgtgcaata gcaaaagttc 2700
   gcttcctaaa gttaaagaaa gaaagaaaaa gcctgtggac tatattattg ctttttggta 2760
   ttagctttat ccctcaactt ttggaacatc tattctacga gtcatatcag aaaagttacc 2820
   cgtgggaact gtctccaaat acatacttcc tctcaccagg acaacaacca caggatcctc 2880
   tgacccattt actggtcatc aataagacag ggtcaaccat tgataacttt ttacattcac 2940
   tgaggcgaca gaacatagct atagaagtgg atgcctttgg aactagaaat ggcacagatg 3000
   acccatctta caatggtgct atcattgtgt caggtgatga aaaggatcac agattttcaa 3060
   tagcatgtaa tacaaaacgg ctgaattgct ttcctgtcct cctggatgtc attagcaatg 3120
   gactacttgg aatttttaat tcgtcagaac acattcagac tgacagaagc acattttttg 3180
   aagagcatat ggattatgag tatgggtacc gaagtaacac cttcttctgg ataccgatgg 3240
   cagcctcttt cactccatac attgcaatga gcagcattgg tgactacaaa aaaaaagctc 3300
   attcccagct acggatttca ggcctctacc cttctgcata ctggtttggc caagcactgg 3360
   tggatgtttc cctgtacttt ttgatcctcc tgctaatgca aataatggat tatattttta 3420
   gcccagagga gattatattt ataattcaaa acctgttaat tcaaatcctg tgtagtattg 3480
   gctatgtctc atctcttgtt ttcttgacat atgtgatttc attcattttt cgcaatggga 3540
   gaaaaaatag tggcatttgg tcatttttct tcttaattgt ggtcatcttc tcgatagttg 3600
   ctactgatct aaatgaatat ggatttctag ggctattttt tggcaccatg ttaatacctc 3660
   ccttcacatt gattggctct ctattcattt tttctgagat ttctcctgat tccatggatt 3720
   acttaggagc ttcagaatct gaaattgtat acctggcact gctaatacct taccttcatt 3780
   ttctcatttt tcttttcatt ctgcgatgcc tagaaatgaa ctgcaggaag aaactaatga 3840
   gaaaggatcc tgtgttcaga atttctccaa gaagcaacgc tatttttcca aacccagaag 3900
   agcctgaagg agaggaggaa gatatccaga tggaaagaat gagaacagtg aatgctatgg 3960
   ctgtgcgaga ctttgatgag acacccgtca tcattgccag ctgtctacgg aaggaatatg 4020
   caggcaaaaa gaaaaattgc ttttctaaaa ggaagaaaac aattgccaca agaaatgtct 4080
   ctttttgtgt taaaaaaggt gaagttatag gactgttagg acacaatgga gctggtaaaa 4140
   gtacaactat taagatgata actggagaca caaaaccaac tgcaggacag gtgattttga 4200
   aagggagcgg tggaggggaa cccctgggct tcctggggta ctgccctcag gagaatgcgc 4260
   tgtggcccaa cctgacagtg aggcagcacc tggaggtgta cgctgccgtg aaaggtctca 4320
   ggaaagggga cgcaatgatc gccatcacac ggttagtgga tgcgctcaag ctgcaggacc 4380
   agctgaaggc tcccgtgaag accttgtcag agggaataaa gcgaaagctg cgctttgtgc 4440
   tgagcatcct ggggaacccg tcagtggtgc ttctggatga gccgtcgacc gggatggacc 4500
   ccgaggggca gcagcaaatg tggcaggtga ttcggccacc ctttagaaac acggagaggg 4560
   gcgccctcct gaccacccac tacatggcag aggctgaggc ggtgtgtgac cgagtggcca 4620
   tcatggtgtc aggaaggctg agatgtattg gttccatcca acacctgaaa agcaaatttg 4680
   gcaaagacta cctgctggag atgaagctga agaacctggc acaaatggag cccctccatg 4740
   cagagatcct gaggcttttc ccccaggctg ctcagcagga aaggttctcc tccctgatgg 4800
   tctataagtt gcctgttgag gatgtgcgac ctttatcaca ggctttcttc aaattagaga 4860
   tagttaaaca gagtttcgac ctggaggagt acagcctctc acagtctacc ctggagcagg 4920
   ttttcctgga gctctccaag gagcaggagc tgggtgatct tgaagaggac tttgatccct 4980
   cggtgaagtg gaaactcctc ctgcaggaag agccttaaag ctccaaatac cctatatctt 5040
   tctttaatcc tgtgactctt ttaaagataa tattttatag ccttaatatg ccttatatca 5100
   gaggtggtac aaaatgcatt tgaaactcat gcaataatta tcctcagtag tatttcttac 5160
   agtgagacaa caggcaatgt cagtgagggc gatcgtaggg cataagccta agccatacca 5220
   tgcagccttt gtgccagcaa ccaaatccca tgtttcctac tgtgttaagt ttaaaaatgc 5280
   atttattata gaattgtcta catttctgag gatgtcatgg agaatgctta attttctttc 5340
   tctgaacttc aaaatattaa atattttctt atttttttga ttaaagtata aattaagaca 5400
   ccctattgac ttccgggtaa ggggagtcaa ttgattaccc agcagcacag tatttgcttt 5460
   ttataattcc ctttttaaat acttgttctt aattgactgg ttttcctttt ctgtcatttt 5520
   tcagagttta gattgtgagt ccatgttttg tctgttgtgc ctataaagga aatttgaaat 5580
   ctgtatcatt ctactataaa gacacatgca cacgtatgtt tattgcagca ctgtttacaa 5640
   tagcaaagac ttggaaccaa ccaaaatacc cacaaatgat agaccggata aagaaaacgt 5700
   gacacatata caccatggaa tactatgcag ccatagaaaa ggatgagttc atattcttca 5760
   cagggacatg gatgaagctg gaaaccatca tcctcagcaa actaacacag gaacagaaaa 5820
   ccaaacaccg catgttctca ctcataagtg ggaattgaac aatgagaata catggacaca 5880
   gggaggggaa caccacaccc tggggcctgt tggggggatg ggggctaggg gagggatagc 5940
   attaggagaa atacctgatg tagatgatgg gttgatgggt g 5981

<210> 4
<211> 6181
<212> ADN
<213> Homo sapiens

<400> 4
aattaatttt acttaggata agtgttgtta ttattgtttt tattgttgtt ctgttagtta 60
ctcaaaactt cattctaatt gtgccctgag tttgttaaaa taccatactg tatttttgtg 120
taacatgtaa ataggcatta atttttgaga aatagaaatg tttatcctta atgtattttt 180
aatttgctaa cattgatttt ttattttctt tcctgaaata gcttatttcc taaaatgaaa 240
gaatttattc tcagatgaat aatttttata tcagctattc ttatcagagc aataaacaaa 300
taccaatgat gcgctcagcc aacaattcat tacactctct gaagagtaac tggacaagga 360

gaaaaacata gggaaaaaac caacagaatt tgttggcatg ttctacacac agaccatggc 420
ttttcagaag ccaagctgaa taaaaacagt tttaaaagag gcaaccattt gtagaggagt 480
ccttgaagga ttcttcattg ttttcttgga caaaaagaga ccagtggatc caagtgcttc 540
aaatacttct ctcttatttt cttaactcta ttgctctgca atatttactt taccctgtta 600
atgaacagga caaaatggtt aaaaaagaga taagcgtgcg tcaacaaatt caggctcttc 660
tgtacaagaa ttttcttaaa aaatggagaa taaaaagaga gtttattgga atggacaata 720
acattgtttc tagggctata tttgtgcatc ttttcggaac acttcagagc tacccgtttt 780
cctgaacaac ctcctaaagt cctgggaagc gtggatcagt ttaatgactc tggcctggta 840
gtggcatata caccagtcag taacataaca caaaggataa tgaataagat ggccttggct 900
tcctttatga aaggaagaac agtcattggg acaccagatg aagagaccat ggatatagaa 960
cttccaaaaa aataccatga aatggtggga gttatattta gtgatacttt ctcatatcgc 1020
ctgaagttta attggggata tagaatccca gttataaagg agcactctga atacacagaa 1080
cactgttggg ccatgcatgg tgaaattttt tgttacttgg caaagtactg gctaaaaggg 1140
tttgtagctt ttcaagctgc aattaatgct gcaattatag aagtcacaac aaatcattct 1200
gtaatggagg agttgacatc agttattgga ataaatatga agataccacc tttcatttct 1260
aagggagaaa ttatgaatga atggtttcat tttacttgct tagtttcttt ctcttctttt 1320
atatactttg catcattaaa tgttgcaagg gaaagaggaa aatttaagaa actgatgaca 1380
gtaatgggtc tccgagagtc agcattctgg ctctcctggn gattgacata catttgcttc 1440
atcttcatta tgtccatttt tatggctctg gtcataacat caatctcaat tgtatttcat 1500
actggcttca tggtgatatt cacactctat agcttatatg gcctttcttt gatagcattg 1560
gctttcctca tgagtgtttt aataaggaaa cctatgctcg ctggtttggc tggatttctc 1620
ttcactgtat tttggggatg tctgggattc actgtgttat acagacaact tcctttatct 1680
ttgggatggg tattaagtct tcttagccct tttgccttca ctgctggaat ggcccaggtt 1740
acacacctgg ataattactt aagtggtgtt atttttcctg atccctctgg ggattcatac 1800
aaaatgatag ccactttttt cattttggca tttgatactc ttttctattt gatattcaca 1860
ttatattttg agcgagtttt acctgataaa gatggccatg gggattctcc attatttttc 1920
cttaagtcct cattttggtc caaacatcaa aatactcatc atgaaatctt tgagaatgaa 1980
ataaatcctg agcattcctc tgatgattct tttgaaccgg tgtctccaga attccatgga 2040
aaagaagcca taagaatcag aaatgttata aaagaatata atggaaagac tggaaaagta 2100
gaagcattgc aaggcatatt ttttgacata tatgaaggac agatcactgc aatacttggg 2160
cataatggag ctggtaaatc aacactgcta aacattctta gtggattgtc tgtttctaca 2220
gaaggatcag ccactattta taatactcaa ctctctgaaa taactgacat ggaagaaatt 2280
agaaagaata ttggattttg tccacagttc aattttcaat ttgacttcct cactgtgaga 2340
gaaaacctca gggtatttgc taaaataaaa gggattcagc caaaggaagt ggaacaagag 2400
gtaaaaagaa ttataatgga attagacatg caaagcattc aagacattat tgctaaaaaa 2460
ttaagtggtg ggcagaagag aaaactaaca ctagggattg ccatcttagg agatcctcag 2520
gttttgctgc tagatgaacc aactgctgga ttggatccct tttcaagaca ccgagtgtgg 2580
agcctcctga aggagcataa agtagaccga cttatcctct tcagtaccca attcatggat 2640
gaggctgaca tcttggctga taggaaagta tttctgtcta atgggaagtt gaaatgtgca 2700
ggatcatctt tgtttctgaa gcgaaagtgg ggtattggat atcatttaag tttacacagg 2760
aatgaaatgt gtgacacaga aaaaatcaca tcccttatta agcagcacat tcctgatgcc 2820
aagttaacaa cagaaagtga agaaaaactt gtatatagtt tgcctttgga aaaaacgaac 2880
aaatttccag atctttacag tgaccttgat aagtgttctg accagggcat aaggaattat 2940
gctgtttcag tgacatctct gaatgaagta ttcttgaacc tagaaggaaa atcagcaatt 3000
gatgaaccag attttgacat tgggaaacaa gagaaaatac atgtgacaag aaatactgga 3060
gatgagtctg aaatggaaca ggttctttgt tctcttcctg aaacaagaaa ggctgtcagt 3120
agtgcagctc tctggagacg acaaatctat gcagtggcaa cacttcgctt cttaaagtta 3180
aggcgtgaaa ggagagctct tttgtgtttg ttactagtac ttggaattgc ttttatcccc 3240
atcattctag agaagataat gtataaagta actcgtgaaa ctcattgttg ggagttttca 3300
cccagtatgt atttcctttc tctggaacaa atcccgaaga cgcctcttac cagcctgtta 3360
atcgttaata atacaggatc aaatattgaa gacctcgtgc attcactgaa gtgtcaggat 3420
atagttttgg aaatagatga ctttagaaac agaaatggct cagatgatcc ctcctacaat 3480
ggagccatca tagtgtctgg tgaccagaag gattacagat tttctgttgc gtgtaatacc 3540
aagaaattga attgttttcc tgttcttatg ggaattgtta gcaatgccct tatgggaatt 3600
tttaacttca cggagcttat tcaaacggag agcacttcat tttctcgtga tgacatagtg 3660
ctggatcttg gttttataga tgggtccata tttttgttgt tgatcacaaa ctgcgtttct 3720
ccttttatcg gcatgagcag catcagcgat tataaaaaaa atgttcaatc ccagttatgg 3780
atttcaggcc tctggccttc agcatactgg tgtggacagg ctctggtgga cattccatta 3840
tacttcttga ttctcttttc aatacattta atttactact tcatatttct gggattccag 3900
ctttcatggg aactcatgtt tgttttggtg gtatgcataa ttggttgtgc agtttctctt 3960
atattcctca catatgtgct ttcattcatc tttcgcaagt ggagaaaaaa taatggcttt 4020
tggtcttttg gcttttttat tatcttaata tgtgtatcca caattatggt atcaactcaa 4080
tatgaaaaac tcaacttaat tttgtgcatg attttcatac cttccttcac tttgctgggg 4140
tatgtcatgt tattgatcca gctcgacttt atgagaaact tggacagtct ggacaataga 4200
ataaatgaag tcaataaaac cattctttta acaaccttaa taccatacct tcagagtgtt 4260
attttccttt ttgtcataag gtgtctggaa atgaagtatg gaaatgaaat aatgaataaa 4320
gacccagttt tcagaatctc tccacggagt agagaaactc atcccaatcc ggaagagccc 4380
gaagaagaag atgaagatgt tcaagctgaa agagtccaag cagcaaatgc actcactgct 4440
ccaaacttgg aggaggaacc agtcataact gcaagctgtt tacacaagga atattatgag 4500
acaaagaaaa gttgcttttc aacaagaaag aagaaaatag ccatcagaaa tgtttccttt 4560

tgtgttaaaa aaggtgaagt tttgggatta ctaggacaca atggagctgg taaaagtact 4620
tccattaaaa tgataactgg gtgcacaaag ccaactgcag gagtggtggt gttacaaggc 4680
agcagagcat cagtaaggca acagcatgac aacagcctca agttcttggg gtactgccct 4740
caggagaact cactgtggcc caagcttaca atgaaagagc acttggagtt gtatgcagct 4800
gtgaaaggac tgggcaaaga agatgctgct ctcagtattt cacgattggt ggaagctctt 4860
aagctccagg aacaacttaa ggctcctgtg aaaactctat cagagggaat aaagagaaag 4920
ctgtgctttg tgctgagcat cctggggaac ccatcagtgg tgcttctaga tgagccgttc 4980
accgggatgg accccgaggg gcagcagcaa atgtggcaga tacttcaggc taccgttaaa 5040
aacaaggaga ggggcaccct cttgaccacc cattacatgt cagaggctga ggctgtgtgt 5100
gaccgtatgg ccatgatggt gtcaggaacg ctaaggtgta ttggttccat tcaacatctg 5160
aaaaacaagt ttggtagaga ttatttacta gaaataaaaa tgaaagaacc tacccaggtg 5220
gaagctctcc acacagagat tttgaagctt ttcccacagg ctgcttggca ggaaagatat 5280
tcctctttaa tggcgtataa gttacctgtg gaggatgtcc accctctatc tcggcctttt 5340
ttcaagttag aggcgatgaa acagaccttc aacctggagg aatacagcct ctctcaggct 5400
accttggagc aggtattctt agaactctgt aaagagcagg agctgggaaa tgttgatgat 5460
aaaattgata caacagttga atggaaactt ctcccacagg aagaccctta aaatgaagaa 5520
cctcctaaca ttcaatttta ggtcctacta cattgttagt ttccataatt ctacaagaat 5580
gtttcctttt acttcagtta acaaaagaaa acatttaata aacattcaat aatgattaca 5640
gttttcattt ttaaaaattt aggatgaagg aaacaaggaa atatagggaa aagtagtaga 5700
caaaattaac aaaatcagac atgttattca tccccaacat gggtctattt tgtgcttaaa 5760
aataatttaa aaatcataca atattaggtt ggttttcggt tattatcaat aaagctaaca 5820
ctgagaacat tttacaaata aaaatatgag ttttttagcc tgaacttcaa atgtatcagc 5880
tatttttaaa cattatttac tcggattcta atttaatgtg acattgacta taagaaggtc 5940
tgataaactg atgaaatggc acagcataac atttaattat aatgacattc tgattataaa 6000
ataaatgcat gtgaatttta gtacatattg aagttatatg gaagaagata gccataatct 6060
gtaagaaagt accgcagtta atattttctt tagccaactt atattcaatg tattttttat 6120
ggatcctttt tcaaaggtag tatcagtagg catagtcatt ttctgtatct tttcacctca 6180
c 6181

<210> 5
<211> 1642
<212> PRT
<213> Homo sapiens

<400> 5
Met Ser Thr Ala Ile Arg Glu Val Gly Val Trp Arg Gln Thr Arg Thr
1               5                   10                  15

Leu Leu Leu Lys Asn Tyr Leu Ile Lys Cys Arg Thr Lys Lys Ser Ser
            20                  25                  30

Val Gln Glu Ile Leu Phe Pro Leu Phe Phe Leu Phe Trp Leu Ile Leu
        35                  40                  45

Ile Ser Met Met His Pro Asn Lys Lys Tyr Glu Glu Val Pro Asn Ile
    50                  55                  60

Glu Leu Asn Pro Met Asp Lys Phe Thr Leu Ser Asn Leu Ile Leu Gly
65                  70                  75                  80

Tyr Thr Pro Val Thr Asn Ile Thr Ser Ser Ile Met Gln Lys Val Ser
                85                  90                  95

Thr Asp His Leu Pro Asp Val Ile Ile Thr Glu Glu Tyr Thr Asn Glu
            100                 105                 110

Lys Glu Met Leu Thr Ser Ser Leu Ser Lys Pro Ser Asn Phe Val Gly
        115                 120                 125

Val Val Phe Lys Asp Ser Met Ser Tyr Glu Leu Arg Phe Phe Pro Asp
    130                 135                 140

Met Ile Pro Val Ser Ser Ile Tyr Met Asp Ser Arg Ala Gly Cys Ser
145                 150                 155                 160

Lys Ser Cys Glu Ala Ala Gln Tyr Trp Ser Ser Gly Phe Thr Val Leu
                165                 170                 175

Gln Ala Ser Ile Asp Ala Ala Ile Ile Gln Leu Lys Thr Asn Val Ser

            180                 185                 190

Leu Trp Lys Glu Leu Glu Ser Thr Lys Ala Val Ile Met Gly Glu Thr
        195                 200                 205

Ala Val Val Glu Ile Asp Thr Phe Pro Arg Gly Val Ile Leu Ile Tyr
    210                 215                 220

Leu Val Ile Ala Phe Ser Pro Phe Gly Tyr Phe Leu Ala Ile His Ile
225                 230                 235                 240

Val Ala Glu Lys Glu Lys Lys Ile Lys Glu Phe Leu Lys Ile Met Gly
                245                 250                 255

Leu His Asp Thr Ala Phe Trp Leu Ser Trp Val Leu Leu Tyr Thr Ser
            260                 265                 270

Leu Ile Phe Leu Met Ser Leu Leu Met Ala Val Ile Ala Thr Ala Ser
        275                 280                 285

Leu Leu Phe Pro Gln Ser Ser Ser Ile Val Ile Phe Leu Leu Phe Phe
    290                 295                 300

Leu Tyr Gly Leu Ser Ser Val Phe Phe Ala Leu Met Leu Thr Pro Leu
305                 310                 315                 320

Phe Lys Lys Ser Lys His Val Gly Ile Val Glu Phe Phe Val Thr Val
                325                 330                 335

Ala Phe Gly Phe Ile Gly Leu Met Ile Ile Leu Ile Glu Ser Phe Pro
            340                 345                 350

Lys Ser Leu Val Trp Leu Phe Ser Pro Phe Cys His Cys Thr Phe Val
        355                 360                 365

Ile Gly Ile Ala Gln Val Met His Leu Glu Asp Phe Asn Glu Gly Ala
    370                 375                 380

Ser Phe Ser Asn Leu Thr Ala Gly Pro Tyr Pro Leu Ile Ile Thr Ile
385                 390                 395                 400

Ile Met Leu Thr Leu Asn Ser Ile Phe Tyr Val Leu Leu Ala Val Tyr
                405                 410                 415

Leu Asp Gln Val Ile Pro Gly Glu Phe Gly Leu Arg Arg Ser Ser Leu
            420                 425                 430

Tyr Phe Leu Lys Pro Ser Tyr Trp Ser Lys Ser Lys Arg Asn Tyr Glu
        435                 440                 445

Glu Leu Ser Glu Gly Asn Val Asn Gly Asn Ile Ser Phe Ser Glu Ile
    450                 455                 460

Ile Glu Pro Val Ser Ser Glu Phe Val Gly Lys Glu Ala Ile Arg Ile
465                 470                 475                 480

Ser Gly Ile Gln Lys Thr Tyr Arg Lys Lys Gly Glu Asn Val Glu Ala
                485                 490                 495

Leu Arg Asn Leu Ser Phe Asp Ile Tyr Glu Gly Gln Ile Thr Ala Leu
            500                 505                 510

Leu Gly His Ser Gly Thr Gly Lys Ser Thr Leu Met Asn Ile Leu Cys
        515                 520                 525

Gly Leu Cys Pro Pro Ser Asp Gly Phe Ala Ser Ile Tyr Gly His Arg
    530                 535                 540

Val Ser Glu Ile Asp Glu Met Phe Glu Ala Arg Lys Met Ile Gly Ile
545                 550                 555                 560

Cys Pro Gln Leu Asp Ile His Phe Asp Val Leu Thr Val Glu Glu Asn
                565                 570                 575

Leu Ser Ile Leu Ala Ser Ile Lys Gly Ile Pro Ala Asn Asn Ile Ile
            580                 585                 590

Gln Glu Val Gln Lys Val Leu Leu Asp Leu Asp Met Gln Thr Ile Lys
        595                 600                 605

Asp Asn Gln Ala Lys Lys Leu Ser Gly Gly Gln Lys Arg Lys Leu Ser
    610                 615                 620

Leu Gly Ile Ala Val Leu Gly Asn Pro Lys Ile Leu Leu Leu Asp Glu
625                 630                 635                 640

Pro Thr Ala Gly Met Asp Pro Cys Ser Arg His Ile Val Trp Asn Leu
                645                 650                 655

Leu Lys Tyr Arg Lys Ala Asn Arg Val Thr Val Phe Ser Thr His Phe
            660                 665                 670

Met Asp Glu Ala Asp Ile Leu Ala Asp Arg Lys Ala Val Ile Ser Gln
        675                 680                 685

Gly Met Leu Lys Cys Val Gly Ser Ser Met Phe Leu Lys Ser Lys Trp
    690                 695                 700

Gly Ile Gly Tyr Arg Leu Ser Met Tyr Ile Asp Lys Tyr Cys Ala Thr
705                 710                 715                 720

Glu Ser Leu Ser Ser Leu Val Lys Gln His Ile Pro Gly Ala Thr Leu
                725                 730                 735

Leu Gln Gln Asn Asp Gln Gln Leu Val Tyr Ser Leu Pro Phe Lys Asp
            740                 745                 750

Met Asp Lys Phe Ser Gly Leu Phe Ser Ala Leu Asp Ser His Ser Asn
        755                 760                 765

Leu Gly Val Ile Ser Tyr Gly Val Ser Met Thr Thr Leu Glu Asp Val
    770                 775                 780

Phe Leu Lys Leu Glu Val Glu Ala Glu Ile Asp Gln Ala Asp Tyr Ser
785                 790                 795                 800

Val Phe Thr Gln Gln Pro Leu Glu Glu Glu Met Asp Ser Lys Ser Phe
                805                 810                 815

Asp Glu Met Glu Gln Ser Leu Leu Ile Leu Ser Glu Thr Lys Ala Ser
            820                 825                 830

Leu Val Ser Thr Met Ser Leu Trp Lys Gln Gln Met Tyr Thr Ile Ala
        835                 840                 845

Lys Phe His Phe Phe Thr Leu Lys Arg Glu Ser Lys Ser Val Arg Ser
    850                 855                 860

Val Leu Leu Leu Leu Leu Ile Phe Phe Thr Val Gln Ile Phe Met Phe
865                 870                 875                 880

Leu Val His His Ser Phe Lys Asn Ala Val Val Pro Ile Lys Leu Val
                885                 890                 895

Pro Asp Leu Tyr Phe Leu Lys Pro Gly Asp Lys Pro His Lys Tyr Lys
            900                 905                 910

Thr Ser Leu Leu Leu Gln Asn Ser Ala Asp Ser Asp Ile Ser Asp Leu
        915                 920                 925

Ile Ser Phe Phe Thr Ser Gln Asn Ile Met Val Thr Met Ile Asn Asp
    930                 935                 940

Ser Asp Tyr Val Ser Val Ala Pro His Ser Ala Ala Leu Asn Val Met
945                 950                 955                 960

His Ser Glu Lys Asp Tyr Val Phe Ala Ala Val Phe Asn Ser Thr Met
                965                 970                 975

Val Tyr Ser Leu Pro Ile Leu Val Asn Ile Ile Ser Asn Tyr Tyr Leu
            980                 985                 990

Tyr His Leu Asn Val Thr Glu Thr Ile Gln Ile Trp Ser Thr Pro Phe
        995                 1000                1005

Phe Gln Glu Ile Thr Asp Ile Val Phe Lys Ile Glu Leu Tyr Phe Gln
    1010                1015                1020

Ala Ala Leu Leu Gly Ile Ile Val Thr Ala Met Pro Pro Tyr Phe Ala
1025                1030                1035                1040

Met Glu Asn Ala Glu Asn His Lys Ile Lys Ala Tyr Thr Gln Leu Lys
                1045                1050                1055

Leu Ser Gly Leu Leu Pro Ser Ala Tyr Trp Ile Gly Gln Ala Val Val
            1060                1065                1070

Asp Ile Pro Leu Phe Phe Ile Ile Leu Ile Leu Met Leu Gly Ser Leu
        1075                1080                1085

Leu Ala Phe His Tyr Gly Leu Tyr Phe Tyr Thr Val Lys Phe Leu Ala
    1090                1095                1100

Val Val Phe Cys Leu Ile Gly Tyr Val Pro Ser Val Ile Leu Phe Thr
1105                1110                1115                1120

Tyr Ile Ala Ser Phe Thr Phe Lys Lys Ile Leu Asn Thr Lys Glu Phe
                1125                1130                1135

Trp Ser Phe Ile Tyr Ser Val Ala Ala Leu Xaa Cys Ile Ala Ile Thr
            1140                1145                1150

Glu Ile Thr Phe Phe Met Gly Tyr Thr Ile Ala Thr Ile Leu His Tyr
        1155                1160                1165

Ala Phe Cys Ile Ile Ile Pro Ile Tyr Pro Leu Leu Gly Cys Leu Ile
    1170                1175                1180

Ser Phe Ile Lys Ile Ser Trp Lys Asn Val Arg Lys Asn Val Asp Thr
1185                1190                1195                1200

Tyr Asn Pro Trp Asp Arg Leu Ser Val Ala Val Ile Ser Pro Tyr Leu
                1205                1210                1215

Gln Cys Val Leu Trp Ile Phe Leu Leu Gln Tyr Tyr Glu Lys Lys Tyr
            1220                1225                1230

Gly Gly Arg Ser Ile Arg Lys Asp Pro Phe Phe Arg Asn Leu Ser Thr
        1235                1240                1245

Lys Ser Lys Asn Arg Lys Leu Pro Glu Pro Pro Asp Asn Glu Asp Glu
    1250                1255                1260

Asp Glu Asp Val Lys Ala Glu Arg Leu Lys Val Lys Glu Leu Met Gly
1265                1270                1275                1280

Cys Gln Cys Cys Glu Glu Lys Pro Ser Ile Met Val Ser Asn Leu His
                1285                1290                1295

Lys Glu Tyr Asp Asp Lys Lys Asp Phe Leu Leu Ser Arg Lys Val Lys

1300                1305                1310

Lys Val Ala Thr Lys Tyr Ile Ser Phe Cys Val Lys Lys Gly Glu Ile
    1315                1320                1325

Leu Gly Leu Leu Gly Pro Asn Gly Ala Gly Lys Ser Thr Ile Ile Asn
    1330                1335                1340

Ile Leu Val Gly Asp Ile Glu Pro Thr Ser Gly Gln Val Phe Leu Gly
1345                1350                1355                1360

Asp Tyr Ser Ser Glu Thr Ser Glu Asp Asp Asp Ser Leu Lys Cys Met
                1365                1370                1375

Gly Tyr Cys Pro Gln Ile Asn Pro Leu Trp Pro Asp Thr Thr Leu Gln
            1380                1385                1390

Glu His Phe Glu Ile Tyr Gly Ala Val Lys Gly Met Ser Ala Ser Asp
        1395                1400                1405

Met Lys Glu Val Ile Ser Arg Ile Thr His Ala Leu Asp Leu Lys Glu
    1410                1415                1420

His Leu Gln Lys Thr Val Lys Lys Leu Pro Ala Gly Ile Lys Arg Lys
1425                1430                1435                1440

Leu Cys Phe Ala Leu Ser Met Leu Gly Asn Pro Gln Ile Thr Leu Leu
                1445                1450                1455

Asp Glu Pro Ser Thr Gly Met Asp Pro Lys Ala Lys Gln His Met Trp
            1460                1465                1470

Arg Ala Ile Arg Thr Ala Phe Lys Asn Arg Lys Arg Ala Ala Ile Leu
        1475                1480                1485

Thr Thr His Tyr Met Glu Glu Ala Glu Ala Val Cys Asp Arg Val Ala
    1490                1495                1500

Ile Met Val Ser Gly Gln Leu Arg Cys Ile Gly Thr Val Gln His Leu
1505                1510                1515                1520

Lys Ser Lys Phe Gly Lys Gly Tyr Phe Leu Glu Ile Lys Leu Lys Asp
                1525                1530                1535

Trp Ile Glu Asn Leu Glu Val Asp Arg Leu Gln Arg Glu Ile Gln Tyr
            1540                1545                1550

Ile Phe Pro Asn Ala Ser Arg Gln Glu Ser Phe Ser Ser Ile Leu Ala
        1555                1560                1565

Tyr Lys Ile Pro Lys Glu Asp Val Gln Ser Leu Ser Gln Ser Phe Phe
    1570                1575                1580

Lys Leu Glu Glu Ala Lys His Ala Phe Ala Ile Glu Glu Tyr Ser Phe
1585                1590                1595                1600

Ser Gln Ala Thr Leu Glu Gln Val Phe Val Glu Leu Thr Lys Glu Gln
                1605                1610                1615

Glu Glu Glu Asp Asn Ser Cys Gly Thr Leu Asn Ser Thr Leu Trp Trp
            1620                1625                1630

Glu Arg Thr Gln Glu Asp Arg Val Val Phe
        1635                1640

<210> 6
<211> 1617
<212> PRT

<213> Homo sapiens

<400> 6

<210> 7
<211> 1624
<212> PRT
<213> Homo sapiens

<400> 7

<210> 8
<211> 1543
<212> PRT
<213> Homo sapiens

<400> 8
Met Asn Lys Met Ala Leu Ala Ser Phe Met Lys Gly Arg Thr Val Ile
1               5                   10                  15

Gly Thr Pro Asp Glu Glu Thr Met Asp Ile Glu Leu Pro Lys Lys Tyr
            20                  25                  30

His Glu Met Val Gly Val Ile Phe Ser Asp Thr Phe Ser Tyr Arg Leu
        35                  40                  45

Lys Phe Asn Trp Gly Tyr Arg Ile Pro Val Ile Lys Glu His Ser Glu
    50                  55                  60

Tyr Thr Glu His Cys Trp Ala Met His Gly Glu Ile Phe Cys Tyr Leu
65                  70                  75                  80

Ala Lys Tyr Trp Leu Lys Gly Phe Val Ala Phe Gln Ala Ala Ile Asn
                85                  90                  95

Ala Ala Ile Ile Glu Val Thr Thr Asn His Ser Val Met Glu Glu Leu
            100                 105                 110

Thr Ser Val Ile Gly Ile Asn Met Lys Ile Pro Pro Phe Ile Ser Lys
        115                 120                 125

Gly Glu Ile Met Asn Glu Trp Phe His Phe Thr Cys Leu Val Ser Phe
    130                 135                 140

Ser Ser Phe Ile Tyr Phe Ala Ser Leu Asn Val Ala Arg Glu Arg Gly
145                 150                 155                 160

Lys Phe Lys Lys Leu Met Thr Val Met Gly Leu Arg Glu Ser Ala Phe
                165                 170                 175

Trp Leu Ser Trp Xaa Leu Thr Tyr Ile Cys Phe Ile Phe Ile Met Ser
            180                 185                 190

Ile Phe Met Ala Leu Val Ile Thr Ser Ile Ser Ile Val Phe His Thr
        195                 200                 205

Gly Phe Met Val Ile Phe Thr Leu Tyr Ser Leu Tyr Gly Leu Ser Leu
    210                 215                 220

Ile Ala Leu Ala Phe Leu Met Ser Val Leu Ile Arg Lys Pro Met Leu
225                 230                 235                 240

Ala Gly Leu Ala Gly Phe Leu Phe Thr Val Phe Trp Gly Cys Leu Gly
                245                 250                 255

Phe Thr Val Leu Tyr Arg Gln Leu Pro Leu Ser Leu Gly Trp Val Leu
            260                 265                 270

Ser Leu Leu Ser Pro Phe Ala Phe Thr Ala Gly Met Ala Gln Val Thr
        275                 280                 285

His Leu Asp Asn Tyr Leu Ser Gly Val Ile Phe Pro Asp Pro Ser Gly
    290                 295                 300

Asp Ser Tyr Lys Met Ile Ala Thr Phe Phe Ile Leu Ala Phe Asp Thr
305                 310                 315                 320

Leu Phe Tyr Leu Ile Phe Thr Leu Tyr Phe Glu Arg Val Leu Pro Asp
                325                 330                 335

Lys Asp Gly His Gly Asp Ser Pro Leu Phe Phe Leu Lys Ser Ser Phe
            340                 345                 350

Trp Ser Lys His Gln Asn Thr His His Glu Ile Phe Glu Asn Glu Ile
        355                 360                 365

Asn Pro Glu His Ser Ser Asp Asp Ser Phe Glu Pro Val Ser Pro Glu
    370                 375                 380

Phe His Gly Lys Glu Ala Ile Arg Ile Arg Asn Val Ile Lys Glu Tyr
385                 390                 395                 400

Asn Gly Lys Thr Gly Lys Val Glu Ala Leu Gln Gly Ile Phe Phe Asp
                405                 410                 415

Ile Tyr Glu Gly Gln Ile Thr Ala Ile Leu Gly His Asn Gly Ala Gly
            420                 425                 430

Lys Ser Thr Leu Leu Asn Ile Leu Ser Gly Leu Ser Val Ser Thr Glu
        435                 440                 445

Gly Ser Ala Thr Ile Tyr Asn Thr Gln Leu Ser Glu Ile Thr Asp Met
    450                 455                 460

Glu Glu Ile Arg Lys Asn Ile Gly Phe Cys Pro Gln Phe Asn Phe Gln
465                 470                 475                 480

Phe Asp Phe Leu Thr Val Arg Glu Asn Leu Arg Val Phe Ala Lys Ile
                485                 490                 495

Lys Gly Ile Gln Pro Lys Glu Val Glu Gln Glu Val Lys Arg Ile Ile
            500                 505                 510

Met Glu Leu Asp Met Gln Ser Ile Gln Asp Ile Ile Ala Lys Lys Leu
        515                 520                 525

Ser Gly Gly Gln Lys Arg Lys Leu Thr Leu Gly Ile Ala Ile Leu Gly
    530                 535                 540

Asp Pro Gln Val Leu Leu Leu Asp Glu Pro Thr Ala Gly Leu Asp Pro
545                 550                 555                 560

Phe Ser Arg His Arg Val Trp Ser Leu Leu Lys Glu His Lys Val Asp
                565                 570                 575

Arg Leu Ile Leu Phe Ser Thr Gln Phe Met Asp Glu Ala Asp Ile Leu
            580                 585                 590

Ala Asp Arg Lys Val Phe Leu Ser Asn Gly Lys Leu Lys Cys Ala Gly
        595                 600                 605

Ser Ser Leu Phe Leu Lys Arg Lys Trp Gly Ile Gly Tyr His Leu Ser
    610                 615                 620

Leu His Arg Asn Glu Met Cys Asp Thr Glu Lys Ile Thr Ser Leu Ile
625                 630                 635                 640

Lys Gln His Ile Pro Asp Ala Lys Leu Thr Thr Glu Ser Glu Glu Lys
                645                 650                 655

Leu Val Tyr Ser Leu Pro Leu Glu Lys Thr Asn Lys Phe Pro Asp Leu
            660                 665                 670

Tyr Ser Asp Leu Asp Lys Cys Ser Asp Gln Gly Ile Arg Asn Tyr Ala
        675                 680                 685

Val Ser Val Thr Ser Leu Asn Glu Val Phe Leu Asn Leu Glu Gly Lys
    690                 695                 700

Ser Ala Ile Asp Glu Pro Asp Phe Asp Ile Gly Lys Gln Glu Lys Ile
705                 710                 715                 720

His Val Thr Arg Asn Thr Gly Asp Glu Ser Glu Met Glu Gln Val Leu
                725                 730                 735

Cys Ser Leu Pro Glu Thr Arg Lys Ala Val Ser Ser Ala Ala Leu Trp

            740                 745                 750

Arg Arg Gln Ile Tyr Ala Val Ala Thr Leu Arg Phe Leu Lys Leu Arg
        755                 760                 765

Arg Glu Arg Arg Ala Leu Leu Cys Leu Leu Leu Val Leu Gly Ile Ala
    770                 775                 780

Phe Ile Pro Ile Ile Leu Glu Lys Ile Met Tyr Lys Val Thr Arg Glu
785                 790                 795                 800

Thr His Cys Trp Glu Phe Ser Pro Ser Met Tyr Phe Leu Ser Leu Glu
                805                 810                 815

Gln Ile Pro Lys Thr Pro Leu Thr Ser Leu Leu Ile Val Asn Asn Thr
            820                 825                 830

Gly Ser Asn Ile Glu Asp Leu Val His Ser Leu Lys Cys Gln Asp Ile
        835                 840                 845

Val Leu Glu Ile Asp Asp Phe Arg Asn Arg Asn Gly Ser Asp Asp Pro
    850                 855                 860

Ser Tyr Asn Gly Ala Ile Ile Val Ser Gly Asp Gln Lys Asp Tyr Arg
865                 870                 875                 880

Phe Ser Val Ala Cys Asn Thr Lys Lys Leu Asn Cys Phe Pro Val Leu
                885                 890                 895

Met Gly Ile Val Ser Asn Ala Leu Met Gly Ile Phe Asn Phe Thr Glu
            900                 905                 910

Leu Ile Gln Thr Glu Ser Thr Ser Phe Ser Arg Asp Asp Ile Val Leu
        915                 920                 925

Asp Leu Gly Phe Ile Asp Gly Ser Ile Phe Leu Leu Leu Ile Thr Asn
    930                 935                 940

Cys Val Ser Pro Phe Ile Gly Met Ser Ser Ile Ser Asp Tyr Lys Lys
945                 950                 955                 960

Asn Val Gln Ser Gln Leu Trp Ile Ser Gly Leu Trp Pro Ser Ala Tyr
                965                 970                 975

Trp Cys Gly Gln Ala Leu Val Asp Ile Pro Leu Tyr Phe Leu Ile Leu
            980                 985                 990

Phe Ser Ile His Leu Ile Tyr Tyr Phe Ile Phe Leu Gly Phe Gln Leu
        995                 1000                1005

Ser Trp Glu Leu Met Phe Val Leu Val Val Cys Ile Ile Gly Cys Ala
    1010                1015                1020

Val Ser Leu Ile Phe Leu Thr Tyr Val Leu Ser Phe Ile Phe Arg Lys
1025                1030                1035                1040

Trp Arg Lys Asn Asn Gly Phe Trp Ser Phe Gly Phe Phe Ile Ile Leu
                1045                1050                1055

Ile Cys Val Ser Thr Ile Met Val Ser Thr Gln Tyr Glu Lys Leu Asn
            1060                1065                1070

Leu Ile Leu Cys Met Ile Phe Ile Pro Ser Phe Thr Leu Leu Gly Tyr
        1075                1080                1085

Val Met Leu Leu Ile Gln Leu Asp Phe Met Arg Asn Leu Asp Ser Leu
    1090                1095                1100

Asp Asn Arg Ile Asn Glu Val Asn Lys Thr Ile Leu Leu Thr Thr Leu
1105                1110                1115                1120

Ile Pro Tyr Leu Gln Ser Val Ile Phe Leu Phe Val Ile Arg Cys Leu
                1125                1130                1135

Glu Met Lys Tyr Gly Asn Glu Ile Met Asn Lys Asp Pro Val Phe Arg
            1140                1145                1150

Ile Ser Pro Arg Ser Arg Glu Thr His Pro Asn Pro Glu Glu Pro Glu
        1155                1160                1165

Glu Glu Asp Glu Asp Val Gln Ala Glu Arg Val Gln Ala Ala Asn Ala
    1170                1175                1180

Leu Thr Ala Pro Asn Leu Glu Glu Glu Pro Val Ile Thr Ala Ser Cys
1185                1190                1195                1200

Leu His Lys Glu Tyr Tyr Glu Thr Lys Lys Ser Cys Phe Ser Thr Arg
                1205                1210                1215

Lys Lys Lys Ile Ala Ile Arg Asn Val Ser Phe Cys Val Lys Lys Gly
            1220                1225                1230

Glu Val Leu Gly Leu Leu Gly His Asn Gly Ala Gly Lys Ser Thr Ser
        1235                1240                1245

Ile Lys Met Ile Thr Gly Cys Thr Lys Pro Thr Ala Gly Val Val Val
    1250                1255                1260

Leu Gln Gly Ser Arg Ala Ser Val Arg Gln Gln His Asp Asn Ser Leu
1265                1270                1275                1280

Lys Phe Leu Gly Tyr Cys Pro Gln Glu Asn Ser Leu Trp Pro Lys Leu
                1285                1290                1295

Thr Met Lys Glu His Leu Glu Leu Tyr Ala Ala Val Lys Gly Leu Gly
            1300                1305                1310

Lys Glu Asp Ala Ala Leu Ser Ile Ser Arg Leu Val Glu Ala Leu Lys
        1315                1320                1325

Leu Gln Glu Gln Leu Lys Ala Pro Val Lys Thr Leu Ser Glu Gly Ile
    1330                1335                1340

Lys Arg Lys Leu Cys Phe Val Leu Ser Ile Leu Gly Asn Pro Ser Val
1345                1350                1355                1360

Val Leu Leu Asp Glu Pro Phe Thr Gly Met Asp Pro Glu Gly Gln Gln
                1365                1370                1375

Gln Met Trp Gln Ile Leu Gln Ala Thr Val Lys Asn Lys Glu Arg Gly
            1380                1385                1390

Thr Leu Leu Thr Thr His Tyr Met Ser Glu Ala Glu Ala Val Cys Asp
        1395                1400                1405

Arg Met Ala Met Met Val Ser Gly Thr Leu Arg Cys Ile Gly Ser Ile
    1410                1415                1420

Gln His Leu Lys Asn Lys Phe Gly Arg Asp Tyr Leu Leu Glu Ile Lys
1425                1430                1435                1440

Met Lys Glu Pro Thr Gln Val Glu Ala Leu His Thr Glu Ile Leu Lys
                1445                1450                1455

Leu Phe Pro Gln Ala Ala Trp Gln Glu Arg Tyr Ser Ser Leu Met Ala
            1460                1465                1470

Tyr Lys Leu Pro Val Glu Asp Val His Pro Leu Ser Arg Ala Phe Phe
        1475                1480                1485

Lys Leu Glu Ala Met Lys Gln Thr Phe Asn Leu Glu Glu Tyr Ser Leu
1490                1495                1500

Ser Gln Ala Thr Leu Glu Gln Val Phe Leu Glu Leu Cys Lys Glu Gln
1505                1510                1515                1520

Glu Leu Gly Asn Val Asp Asp Lys Ile Asp Thr Thr Val Glu Trp Lys
            1525                1530                1535

Leu Leu Pro Gln Glu Asp Pro
        1540

<210> 9
<211> 130
<212> ADN
<213> Homo sapiens

<400> 9
ctgctggagt aggcacccat ttaaagaaaa aatgaagaag cagcaataaa gaagttgtaa 60
tcgttaccta gacaaacaga gaactggttt tgacagtgtt tctagagtgc tttttattat 120
tttcctgaca                                                        130

<210> 10
<211> 141
<212> ADN
<213> Homo sapiens

<400> 10
gttgtgttcc accatgatta ctttctcctt cagcgaatag gctaaatgaa tatgaaacag 60
aaaagcgtgt atcagcaaac caaagcactt ctgtgcaaga attttcttaa gaaatggagg 120
atgaaaagag agagcttatt g                                           141

<210> 11
<211> 205
<212> ADN
<213> Homo sapiens

<400> 11
gaatggggcc tctcaatact tctaggactg tgtattgctc tgttttccag ttccatgaga 60
aatgtccagt ttcctggaat ggctcctcag aatctgggaa gggtagataa atttaatagc 120
tcttctttaa tggttgtgta tacaccaata tctaatttaa cccagcagat aatgaataaa 180
acagcacttg ctcctctttt gaaag                                       205

<210> 12
<211> 159
<212> ADN
<213> Homo sapiens

<400> 12
gaacaagtgt cattggggca ccaaataaaa cacacatgga cgaaatactt ctggaaaatt 60
taccatatgc tatgggaatc atctttaatg aaactttctc ttataagtta atatttttcc 120
agggatataa cagtccactt tggaaagaag atttctcag                        159

<210> 13
<211> 104
<212> ADN
<213> Homo sapiens

<400> 13
ctcattgctg ggatggatat ggtgagtttt catgtacatt gaccaaatac tggaatagag 60
gatttgtggc tttacaaaca gctattaata ctgccattat agaa                  104

<210> 14

<211> 227
<212> ADN
<213> Homo sapiens

<400> 14
atcacaacca atcaccctgt gatggaggag ttgatgtcag ttactgctat aactatgaag 60
acattacctt tcataactaa aaatcttctt cacaatgaga tgtttatttt attcttcttg 120
cttcatttct ccccacttgt atattttata tcactcaatg taacaaaaga gagaaaaaag 180
tctaagaatt tgatgaaaat gatgggtctc caagattcag cattctg 227

<210> 15
<211> 142
<212> ADN
<213> Homo sapiens

<400> 15
gctctcctgg ggtctaatct atgctggctt catctttatt atttccatat tcattacaat 60
tatcataaca ttcacccaaa ttatagtcat gactggcttc atggtcatat ttatactctt 120
ttttttatat ggcttatctt tg 142

<210> 16
<211> 186
<212> ADN
<213> Homo sapiens

<400> 16
gtagctttgg tgttcctgat gagtgtgctg ttaaagaaag ctgtcctcac caatttggtt 60
gtgtttctcc ttaccctctt ttggggatgt ctgggattca ctgtatttta tgaacaactt 120
ccttcatctc tggagtggat tttgaatatt tgtagccctt ttgcctttac tactggaatg 180
attcag 186

<210> 17
<211> 148
<212> ADN
<213> Homo sapiens

<400> 17
attatcaaac tggattataa cttgaatggt gtaatttttc ctgacccttc aggagactca 60
tatacaatga tagcaacttt ttctatgttg cttttggatg gtctcatcta cttgctattg 120
gcattatact ttgacaaaat tttaccct 148

<210> 18
<211> 169
<212> ADN
<213> Homo sapiens

<400> 18
atggagatga gcgccattat tctcctttat ttttcttgaa ttcatcatct tgtttccaac 60
accaaaggac taatgctaag gttattgaga aagaaatcga tgctgagcat ccctctgatg 120
attattttga accagtagct cctgaattcc aaggaaaaga agccatcag 169

<210> 19
<211> 59
<212> ADN
<213> Homo sapiens

<400> 19
aatcagaaat gttaagaagg aatataaagg aaaatctgga aaagtggaag cattgaaag 59

<210> 20
<211> 111
<212> ADN
<213> Homo sapiens

<400> 20
gcttgctctt tgacatatat gaaggtcaaa tcacggcaat cctgggtcac agtggagctg 60
gcaaatcttc actgctaaat attcttaatg gattgtctgt tccaacagaa g 111

<210> 21
<211> 176
<212> ADN
<213> Homo sapiens

<400> 21
gatcagttac catctataat aaaaatctct ctgaaatgca agacttggag gaaatcagaa 60
agataactgg cgtctgtcct caattcaatg ttcaatttga catactcacc gtgaaggaaa 120
acctcagcct gtttgctaaa ataaaaggga ttcatctaaa ggaagtggaa caagag 176

<210> 22
<211> 120
<212> ADN
<213> Homo sapiens

<400> 22
gtacaacgaa tattattgga attggacatg caaaacattc aagataacct tgctaaacat 60
ttaagtgaag gacagaaaag aaagctgact tttgggatta ccattttagg agatcctcaa 120

<210> 23
<211> 139
<212> ADN
<213> Homo sapiens

<400> 23
attttgcttt tagatgaacc aactactgga ttggatccct tttccagaga tcaagtgtgg 60
agcctcctga gagagcgtag agcagatcat gtgatccttt tcagtaccca gtccatggat 120
gaggctgaca tcctggctg 139

<210> 24
<211> 91
<212> ADN
<213> Homo sapiens

<400> 24
atagaaaagt gatcatgtcc aatgggagac tgaagtgtgc aggttcttct atgtttttga 60
aaagaaggtg ggtcttggga tatcacctaa g 91

<210> 25
<211> 140
<212> ADN
<213> Homo sapiens

<400> 25
tttacatagg aatgaaatat gtaacccaga acaaataaca tccttcatta ctcatcacat 60
ccccgatgct aaattaaaaa cagaaaacaa agaaaagctt gtatatactt tgccactgga 120
aaggacaaat acatttccag 140

<210> 26
<211> 117
<212> ADN
<213> Homo sapiens

<400> 26
atcttttcag tgatctggat aagtgttctg accagggagt gacaggttat gacatttcca 60
tgtcaactct aaatgaagtc tttatgaaac tggaaggaca gtcaactatc gaacaag 117

<210> 27
<211> 184

<212> ADN
<213> Homo sapiens

<400> 27
atttcgaaca agtggagatg ataagagact cagaaagcct caatgaaatg gagctggctc 60
actcttcctt ctctgaaatg cagacagctg tgagtgacat gggcctctgg agaatgcaag 120
tctttgccat ggcacggctc cgtttcttaa agttaaaacg tcaaactaaa gtgttattga 180
ccct 184

<210> 28
<211> 167
<212> ADN
<213> Homo sapiens

<400> 28
attattggta tttggaatcg caatattccc tttgattgtt gaaaatataa tatatgctat 60
gttaaatgaa aagatcgatt gggaatttaa aaacgaattg tattttctct ctcctggaca 120
acttccccag gaaccccgta ccagcctgtt gatcatcaat aacacag 167

<210> 29
<211> 134
<212> ADN
<213> Homo sapiens

<400> 29
aatcaaatat tgaagatttt ataaaatcac tgaagcatca aaatatactt ttggaagtag 60
atgactttga aaacagaaat ggtactgatg gcctctcata caatggagct atcatagttt 120
ctggtaaaca aaag 134

<210> 30
<211> 138
<212> ADN
<213> Homo sapiens

<400> 30
gattatagat tttcagttgt gtgtaatacc aagagattgc actgttttcc aattcttatg 60
aatattatca gcaatgggct acttcaaatg tttaatcaca cacaacatat tcgaattgag 120
tcaagcccat ttcctctt 138

<210> 31
<211> 108
<212> ADN
<213> Homo sapiens

<400> 31
agccacatag gactctggac tgggttgccg gatggttcct ttttcttatt tttggttcta 60
tgtagcattt ctccttatat caccatgggc agcatcagtg attacaag 108

<210> 32
<211> 174
<212> ADN
<213> Homo sapiens

<400> 32
aaaaatgcta agtcccagct atggatttca ggcctctaca cttctgctta ctggtgtggg 60
caggcactag tggacgtcag cttcttcatt ttaattctcc ttttaatgta tttaattttc 120
tacatagaaa acatgcagta ccttcttatt acaagccaaa ttgtgtttgc tttg 174

<210> 33
<211> 114
<212> ADN
<213> Homo sapiens

<400> 33

gttatagtta ctcctggtta tgcagcttct cttgtcttct tcatatatat gatatcattt 60
atttttcgca aaaggagaaa aaacagtggc ctttggtcat tttacttctt tttt 114

<210> 34
<211> 120
<212> ADN
<213> Homo sapiens

<400> 34
gcctccacca tcatgttttc catcacttta atcaatcatt ttgacctaag tatattgatt 60
accaccatgg tattggttcc ttcatatacc ttgcttggat ttaaaacttt tttggaagtg 120

<210> 35
<211> 78
<212> ADN
<213> Homo sapiens

<400> 35
agagaccagg agcactacag agaatttcca gaggcaaatt ttgaattgag tgccactgat 60
tttctagtct gcttcata 78

<210> 36
<211> 92
<212> ADN
<213> Homo sapiens

<400> 36
ccctactttc agactttgct attcgttttt gttctaagat gcatggaact aaaatgtgga 60
aagaaaagaa tgcgaaaaga tcctgttttc ag 92

<210> 37
<211> 121
<212> ADN
<213> Homo sapiens

<400> 37
aatttccccc caaagtagag atgctaagcc aaatccagaa gaacccatag atgaagatga 60
agatattcaa acagaaagaa taagaacagc cactgctctg accacttcaa tcttagatga 120
g 121

<210> 38
<211> 118
<212> ADN
<213> Homo sapiens

<400> 38
aaacctgtta taattgccag ctgtctacac aaagaatatg caggccagaa gaaaagttgc 60
ttttcaaaga ggaagaagaa aatagcagca agaaatatct ctttctgtgt tcaagaag 118

<210> 39
<211> 92
<212> ADN
<213> Homo sapiens

<400> 39
gtgaaatttt gggattgcta ggacccagtg gtgctggaaa aagttcatct attagaatga 60
tatctgggat cacaaagcca actgctggag ag 92

<210> 40
<211> 155
<212> ADN
<213> Homo sapiens

<400> 40
gtggaactga aaggctgcag ttcagttttg ggccacctgg ggtactgccc tcaagagaac 60
gtgctgtggc ccatgctgac gttgagggaa cacctggagg tgtatgctgc cgtcaagggg 120
ctcaggaaag cggacgcgag gctcgccatc gcaag 155

<210> 41
<211> 76
<212> ADN
<213> Homo sapiens

<400> 41
attagtgagt gctttcaaac tgcatgagca gctgaatgtt cctgtgcaga aattaacagc 60
aggaatcacg agaaag 76

<210> 42
<211> 95
<212> ADN
<213> Homo sapiens

<400> 42
ttgtgttttg tgctgagcct cctgggaaac tcacctgtct tgctcctgga tgaaccatct 60
acgggcatag accccacagg gcagcagcaa atgtg 95

<210> 43
<211> 120
<212> ADN
<213> Homo sapiens

<400> 43
gcaggcaatc caggcagtcg ttaaaaacac agagagaggt gtcctcctga ccacccataa 60
cctggctgag gcggaagcct tgtgtgaccg tgtggccatc atggtgtctg gaaggcttag 120

<210> 44
<211> 141
<212> ADN
<213> Homo sapiens

<400> 44
atgcattggc tccatccaac acctgaaaaa caaacttggc aaggattaca ttctagagct 60
aaaagtgaag gaaacgtctc aagtgacttt ggtccacact gagattctga agcttttccc 120
acaggctgca gggcaggaaa g 141

<210> 45
<211> 80
<212> ADN
<213> Homo sapiens

<400> 45
gtattcctct ttgttaacct ataagctgcc cgtggcagac gtttaccctc tatcacagac 60
ctttcacaaa ttagaagcag 80

<210> 46
<211> 56
<212> ADN
<213> Homo sapiens

<400> 46
tgaagcataa ctttaacctg gaagaataca gcctttctca gtgcacactg gagaag 56

<210> 47
<211> 369
<212> ADN
<213> Homo sapiens

<400> 47
gtattcttag agctttctaa agaacaggaa gtaggaaatt ttgatgaaga aattgataca 60
acaatgagat ggaaactcct ccctcattca gatgaacctt aaaacctcaa acctagtaat 120
tttttgttga tctcctataa acttatgttt tatgtaataa ttaatagtat gtttaatttt 180
aaagatcatt taaaattaac atcaggtata ttttgtaaat ttagttaaca aatacataaa 240
ttttaaaatt attcttcctc tcaaacatag gggtgatagc aaacctgtga taaaggcaat 300
acaaaatatt agtaaagtca cccaaagagt caggcactgg gtattgtgga aataaaacta 360
tataaactt 369

<210> 48
<211> 130
<212> ADN
<213> Homo sapiens

<400> 48
attcacaatg aatgtgaaat taaaagcatg atgtagtagt gacccaaaag gaatgtgaat 60
tctcctccag aacatgcaga gacccatgga tgaactgtgt ttctagattt ttcctccagc 120
tttcctgaga 130

<210> 49
<211> 109
<212> ADN
<213> Homo sapiens

<400> 49
gaaacaggtc aaaatgagca agagacgcat gagcgtgggt cagcaaacat gggctcttct 60
ctgcaagaac tgtctcaaaa aatggagaat gaaaagacag accttgttg 109

<210> 50
<211> 208
<212> ADN
<213> Homo sapiens

<400> 50
gaatggctct tttcatttct tctggtactg tttctgtacc tatttttctc caatttacat 60
caagttcatg acactcctca aatgtcttca atggatctgg gacgtgtaga tagttttaat 120
gatactaatt atgttattgc atttgcacct gaatccaaaa ctacccaaga gataatgaac 180
aaagtggctt cagccccatt cctaaaag 208

<210> 51
<211> 165
<212> ADN
<213> Homo sapiens

<400> 51
gaagaacaat catggggtgg cctgatgaaa aaagcatgga tgaattggat ttgaactatt 60
caatagacgc agtgagagtc atctttactg ataccttctc ctaccatttg aagttttctt 120
ggggacatag aatccccatg atgaaagagc acagagacca ttcag 165

<210> 52
<211> 104
<212> ADN
<213> Homo sapiens

<400> 52
ctcactgtca agcagtgaat gaaaaaatga agtgtgaagg ttcagagttc tgggagaaag 60
gctttgtagc ttttcaagct gccattaatg ctgctatcat agaa 104

<210> 53
<211> 227
<212> ADN
<213> Homo sapiens

<400> 53
atcgcaacaa atcattcagt gatggaacag ctgatgtcag ttactggtgt acatatgaag 60
atattacctt ttgttgccca aggaggagtt gcaactgatt ttttcatttt cttttgcatt 120
atttcttttt ctacatttat atactatgta tcagtcaatg ttacacaaga aagacaatac 180
attacgtcat tgatgacaat gatgggactc cgagagtcag cattctg 227

<210> 54
<211> 142
<212> ADN
<213> Homo sapiens

<400> 54
gctttcctgg ggtttgatgt atgctggctt catccttatc atggccactt taatggctct 60
tattgtaaaa tctgcacaaa ttgtcgtcct gactggtttt gtgatggtct tcaccctctt 120
tctcctctat ggcctgtctt tg 142

<210> 55
<211> 186
<212> ADN
<213> Homo sapiens

<400> 55
ataactttag ctttcctgat gagtgtgttg ataaagaaac ctttccttac gggcttggtt 60
gtgtttctcc ttattgtctt ttgggggatc ctgggattcc cagcattgta tacacatctt 120
cctgcatttt tggaatggac tttgtgtctt cttagcccct ttgccttcac tgttgggatg 180
gcccag 186

<210> 56
<211> 148
<212> ADN
<213> Homo sapiens

<400> 56
cttatacatt tggactatga tgtgaattct aatgcccact tggattcttc acaaaatcca 60
tacctcataa tagctactct tttcatgttg gtttttgaca cccttctgta tttggtattg 120
acattatatt ttgacaaaat tttgcccg 148

<210> 57
<211> 169
<212> ADN
<213> Homo sapiens

<400> 57
ctgaatatgg acatcgatgt tctcccttgt ttttcctgaa atcctgtttt tggtttcaac 60
acggaagggc taatcatgtg gtccttgaga atgaaacaga ttctgatcct acacctaatg 120
actgttttga accagtgtct ccagaattct gtgggaagga agccatcag 169

<210> 58
<211> 59
<212> ADN
<213> Homo sapiens

<400> 58
aatcaaaaat cttaaaaaag aatatgcagg gaagtgtgag agagtagaag ctttgaaag 59

<210> 59
<211> 111
<212> ADN
<213> Homo sapiens

<400> 59
gtgtggtgtt tgacatatat gaaggccaga tcactgccct ccttggtcac agtggagctg 60
gaaaaactac cctgttaaac atacttagtg ggttgtcagt tccaacatca g 111

<210> 60
<211> 176
<212> ADN
<213> Homo sapiens

<400> 60
gttcagtcac tgtctataat cacacacttt caagaatggc tgatatagaa aatatcagca 60
agttcactgg attttgtcca caatccaatg tgcaatttgg atttctcact gtgaaagaaa 120
acctcaggct gtttgctaaa ataaaaggga ttttgccaca tgaagtggag aaagag 176

<210> 61
<211> 120
<212> ADN
<213> Homo sapiens

<400> 61
gtacaacgag ttgtacagga attagaaatg gaaaatattc aagacatcct tgctcaaaac 60
ttaagtggtg gacaaaatag gaaactaact tttgggattg ccattttagg agatcctcaa 120

<210> 62
<211> 139
<212> ADN
<213> Homo sapiens

<400> 62
gttttgctat tggatgaacc gactgctgga ttggatcctc tttcaaggca ccgaatatgg 60
aatctcctga aagaggggaa atcagacaga gtaattctct tcagcaccca gtttatagat 120
gaggctgaca ttctggcgg 139

<210> 63
<211> 91
<212> ADN
<213> Homo sapiens

<400> 63
acaggaaggt gttcatatcc aatgggaagc tgaagtgtgc aggctcttct ctgttcctta 60
agaagaaatg gggcataggc taccatttaa g 91

<210> 64
<211> 140
<212> ADN
<213> Homo sapiens

<400> 64
tttgcatctg aatgaaaggt gtgatccaga gagtataaca tcactggtta agcagcacat 60
ctctgatgcc aaattgacag cacaaagtga agaaaaactt gtatatattt tgcctttgga 120
aaggacaaac aaatttccag 140

<210> 65
<211> 120
<212> ADN
<213> Homo sapiens

<400> 65
aactttacag ggatcttgat agatgttcta accaaggcat tgaggattat ggtgtttcca 60
taacaacttt gaatgaggtg tttctgaaat tagaaggaaa atcaactatt gatgaatcag 120

<210> 66
<211> 199
<212> ADN
<213> Homo sapiens

<400> 66

atattggaat ttggggacaa ttacaaactg atggggcaaa agatatagga agccttgttg 60
agctggaaca agttttgtct tccttccacg aaacaaggaa aacaatcagt ggcgtggcgc 120
tctggaggca gcaggtctgt gcaatagcaa aagttcgctt cctaaagtta aagaaagaaa 180
gaaaaagcct gtggactat 199

<210> 67
<211> 167
<212> ADN
<213> Homo sapiens

<400> 67
attattgctt tttggtatta gctttatccc tcaacttttg gaacatctat tctacgagtc 60
atatcagaaa agttacccgt gggaactgtc tccaaataca tacttcctct caccaggaca 120
acaaccacag gatcctctga cccatttact ggtcatcaat aagacag 167

<210> 68
<211> 134
<212> ADN
<213> Homo sapiens

<400> 68
ggtcaaccat tgataacttt ttacattcac tgaggcgaca gaacatagct atagaagtgg 60
atgcctttgg aactagaaat ggcacagatg acccatctta caatggtgct atcattgtgt 120
caggtgatga aaag 134

<210> 69
<211> 138
<212> ADN
<213> Homo sapiens

<400> 69
gatcacagat tttcaatagc atgtaataca aaacggctga attgctttcc tgtcctcctg 60
gatgtcatta gcaatggact acttggaatt tttaattcgt cagaacacat tcagactgac 120
agaagcacat tttttgaa 138

<210> 70
<211> 108
<212> ADN
<213> Homo sapiens

<400> 70
gagcatatgg attatgagta tgggtaccga agtaacacct tcttctggat accgatggca 60
gcctctttca ctccatacat tgcaatgagc agcattggtg actacaaa 108

<210> 71
<211> 174
<212> ADN
<213> Homo sapiens

<400> 71
aaaaaagctc attcccagct acggatttca ggcctctacc cttctgcata ctggtttggc 60
caagcactgg tggatgtttc cctgtacttt ttgatcctcc tgctaatgca aataatggat 120
tatattttta gcccagagga gattatattt ataattcaaa acctgttaat tcaa 174

<210> 72
<211> 114
<212> ADN
<213> Homo sapiens

<400> 72
atcctgtgta gtattggcta tgtctcatct cttgttttct tgacatatgt gatttcattc 60
atttttcgca atgggagaaa aaatagtggc atttggtcat ttttcttctt aatt 114

<210> 73
<211> 120
<212> ADN
<213> Homo sapiens

<400> 73
gtggtcatct tctcgatagt tgctactgat ctaaatgaat atggatttct agggctattt 60
tttggcacca tgttaatacc tcccttcaca ttgattggct ctctattcat tttttctgag 120

<210> 74
<211> 69
<212> ADN
<213> Homo sapiens

<400> 74
atttctcctg attccatgga ttacttagga gcttcagaat ctgaaattgt atacctggca 60
ctgctaata 69

<210> 75
<211> 92
<212> ADN
<213> Homo sapiens

<400> 75
ccttaccttc attttctcat ttttcttttc attctgcgat gcctagaaat gaactgcagg 60
aagaaactaa tgagaaagga tcctgtgttc ag 92

<210> 76
<211> 121
<212> ADN
<213> Homo sapiens

<400> 76
aatttctcca agaagcaacg ctatttttcc aaacccagaa gagcctgaag gagaggagga 60
agatatccag atggaaagaa tgagaacagt gaatgctatg gctgtgcgag actttgatga 120
g 121

<210> 77
<211> 118
<212> ADN
<213> Homo sapiens

<400> 77
acacccgtca tcattgccag ctgtctacgg aaggaatatg caggcaaaaa gaaaaattgc 60
ttttctaaaa ggaagaaaac aattgccaca agaaatgtct ctttttgtgt taaaaaag 118

<210> 78
<211> 92
<212> ADN
<213> Homo sapiens

<400> 78
gtgaagttat aggactgtta ggacacaatg gagctggtaa aagtacaact attaagatga 60
taactggaga cacaaaacca actgcaggac ag 92

<210> 79
<211> 161
<212> ADN
<213> Homo sapiens

<400> 79
gtgattttga aagggagcgg tggaggggaa cccctgggct tcctggggta ctgccctcag 60
gagaatgcgc tgtggcccaa cctgacagtg aggcagcacc tggaggtgta cgctgccgtg 120
aaaggtctca ggaaagggga cgcaatgatc gccatcacac g 161

<210> 80
<211> 76
<212> ADN
<213> Homo sapiens

<400> 80
gttagtggat gcgctcaagc tgcaggacca gctgaaggct cccgtgaaga ccttgtcaga 60
gggaataaag cgaaag 76

<210> 81
<211> 95
<212> ADN
<213> Homo sapiens

<400> 81
ctgcgctttg tgctgagcat cctggggaac ccgtcagtgg tgcttctgga tgagccgtcg 60
accgggatgg accccgaggg gcagcagcaa atgtg 95

<210> 82
<211> 120
<212> ADN
<213> Homo sapiens

<400> 82
gcaggtgatt cgggccacct ttagaaacac ggagaggggc gccctcctga ccacccacta 60
catggcagag gctgaggcgg tgtgtgaccg agtggccatc atggtgtcag gaaggctgag 120

<210> 83
<211> 141
<212> ADN
<213> Homo sapiens

<400> 83
atgtattggt tccatccaac acctgaaaag caaatttggc aaagactacc tgctggagat 60
gaagctgaag aacctggcac aaatggagcc cctccatgca gagatcctga ggcttttccc 120
ccaggctgct cagcaggaaa g 141

<210> 84
<211> 80
<212> ADN
<213> Homo sapiens

<400> 84
gttctcctcc ctgatggtct ataagttgcc tgttgaggat gtgcgacctt tatcacaggc 60
tttcttcaaa ttagagatag 80

<210> 85
<211> 56
<212> ADN
<213> Homo sapiens

<400> 85
ttaaacagag tttcgacctg gaggagtaca gcctctcaca gtctaccctg gagcag 56

<210> 86
<211> 1062
<212> ADN
<213> Homo sapiens

<400> 86
gttttcctgg agctctccaa ggagcaggag ctgggtgatc ttgaagagga ctttgatccc 60
tcggtgaagt ggaaactcct cctgcaggaa gagccttaaa gctccaaata ccctatatct 120

ttctttaatc ctgtgactct tttaaagata atattttata gccttaatat gccttatatc 180
agaggtggta caaaatgcat ttgaaactca tgcaataatt atcctcagta gtatttctta 240
cagtgagaca acaggcaatg tcagtgaggg cgatcgtagg gcataagcct aagccatacc 300
atgcagcctt tgtgccagca accaaatccc atgtttccta ctgtgttaag tttaaaaatg 360
catttattat agaattgtct acatttctga ggatgtcatg gagaatgctt aattttcttt 420
ctctgaactt caaaatatta aatattttct tatttttttg attaaagtat aaattaagac 480
accctattga cttccgggta aggggagtca attgattacc cagcagcaca gtatttgctt 540
tttataattc cctttttaaa tacttgttct taattgactg gttttccttt tctgtcattt 600
ttcagagttt agattgtgag tccatgtttt gtctgttgtg cctataaagg aaatttgaaa 660
tctgtatcat tctactataa agacacatgc acacgtatgt ttattgcagc actgtttaca 720
atagcaaaga cttggaacca accaaaatac ccacaaatga tagaccggat aaagaaaacg 780
tgacacatat acaccatgga atactatgca gccatagaaa aggatgagtt catattcttc 840
acagggacat ggatgaagct ggaaaccatc atcctcagca aactaacaca ggaacagaaa 900
accaaacacc gcatgttctc actcataagt gggaattgaa caatgagaat acatggacac 960
agggagggga acaccacacc ctggggcctg ttggggggat gggggctagg ggagggatag 1020
cattaggaga aatacctgat gtagatgatg ggttgatggg tg 1062

<210> 87
<211> 287
<212> ADN
<213> Homo sapiens

<400> 87
aattaatttt acttaggata agtgttgtta ttattgtttt tattgttgtt ctgttagtta 60
ctcaaaactt cattctaatt gtgccctgag tttgttaaaa taccatactg tatttttgtg 120
taacatgtaa ataggcatta atttttgaga aatagaaatg tttatcctta atgtattttt 180
aatttgctaa cattgatttt ttattttctt tcctgaaata gcttatttcc taaaatgaaa 240
gaatttattc tcagatgaat aatttttata tcagctattc ttatcag 287

<210> 88
<211> 280
<212> ADN
<213> Homo sapiens

<400> 88
agcaataaac aaataccaat gatgcgctca gccaacaatt cattacactc tctgaagagt 60
aactggacaa ggagaaaaac atagggaaaa aaccaacaga atttgttggc atgttctaca 120
cacagaccat ggcttttcag aagccaagct gaataaaaac agttttaaaa gaggcaacca 180
tttgtagagg agtccttgaa ggattcttca ttgttttctt ggacaaaaag agaccagtgg 240
atccaagtgc ttcaaatact tctctcttat tttcttaact 280

<210> 89
<211> 141
<212> ADN
<213> Homo sapiens

<400> 89
ctattgctct gcaatattta ctttaccctg ttaatgaaca ggacaaaatg gttaaaaaag 60
agataagcgt gcgtcaacaa attcaggctc ttctgtacaa gaattttctt aaaaaatgga 120
gaataaaaag agagtttatt g 141

<210> 90
<211> 205
<212> ADN
<213> Homo sapiens

<400> 90
gaatggacaa taacattgtt tctagggcta tatttgtgca tcttttcgga acacttcaga 60
gctacccgtt ttcctgaaca acctcctaaa gtcctgggaa gcgtggatca gtttaatgac 120
tctggcctgg tagtggcata tacaccagtc agtaacataa cacaaaggat aatgaataag 180
atggccttgg cttcctttat gaaag 205

<210> 91
<211> 165
<212> ADN

<213> Homo sapiens

<400> 91
gaagaacagt cattgggaca ccagatgaag agaccatgga tatagaactt ccaaaaaaat 60
accatgaaat ggtgggagtt atatttagtg atactttctc atatcgcctg aagtttaatt 120
ggggatatag aatcccagtt ataaaggagc actctgaata cacag 165

<210> 92
<211> 104
<212> ADN
<213> Homo sapiens

<400> 92
aacactgttg ggccatgcat ggtgaaattt tttgttactt ggcaaagtac tggctaaaag 60
ggtttgtagc ttttcaagct gcaattaatg ctgcaattat agaa 104

<210> 93
<211> 227
<212> ADN
<213> Homo sapiens

<400> 93
gtcacaacaa atcattctgt aatggaggag ttgacatcag ttattggaat aaatatgaag 60
ataccacctt tcatttctaa gggagaaatt atgaatgaat ggtttcattt tacttgctta 120
gtttctttct cttcttttat atactttgca tcattaaatg ttgcaaggga aagaggaaaa 180
tttaagaaac tgatgacagt aatgggtctc cgagagtcag cattctg 227

<210> 94
<211> 142
<212> ADN
<213> Homo sapiens

<400> 94
gctctcctgg ngattgacat acatttgctt catcttcatt atgtccattt ttatggctct 60
ggtcataaca tcaatctcaa ttgtatttca tactggcttc atggtgatat tcacactcta 120
tagcttatat ggcctttctt tg 142

<210> 95
<211> 186
<212> ADN
<213> Homo sapiens

<400> 95
atagcattgg ctttcctcat gagtgtttta ataaggaaac ctatgctcgc tggtttggct 60
ggatttctct tcactgtatt ttggggatgt ctgggattca ctgtgttata cagacaactt 120
cctttatctt tgggatgggt attaagtctt cttagccctt ttgccttcac tgctggaatg 180
gcccag 186

<210> 96
<211> 148
<212> ADN
<213> Homo sapiens

<400> 96
gttacacacc tggataatta cttaagtggt gttatttttc ctgatccctc tggggattca 60
tacaaaatga tagccacttt tttcattttg gcatttgata ctcttttcta tttgatattc 120
acattatatt ttgagcgagt tttacctg 148

<210> 97
<211> 169
<212> ADN
<213> Homo sapiens

<400> 97

ataaagatgg ccatggggat tctccattat ttttccttaa gtcctcattt tggtccaaac 60
atcaaaatac tcatcatgaa atctttgaga atgaaataaa tcctgagcat tcctctgatg 120
attcttttga accggtgtct ccagaattcc atggaaaaga agccataag 169

<210> 98
<211> 59
<212> ADN
<213> Homo sapiens

<400> 98
aatcagaaat gttataaaag aatataatgg aaagactgga aaagtagaag cattgcaag 59

<210> 99
<211> 111
<212> ADN
<213> Homo sapiens

<400> 99
gcatattttt tgacatatat gaaggacaga tcactgcaat acttgggcat aatggagctg 60
gtaaatcaac actgctaaac attcttagtg gattgtctgt ttctacagaa g 111

<210> 100
<211> 176
<212> ADN
<213> Homo sapiens

<400> 100
gatcagccac tatttataat actcaactct ctgaaataac tgacatggaa gaaattagaa 60
agaatattgg attttgtcca cagttcaatt ttcaatttga cttcctcact gtgagagaaa 120
acctcagggt atttgctaaa ataaaaggga ttcagccaaa ggaagtggaa caagag 176

<210> 101
<211> 120
<212> ADN
<213> Homo sapiens

<400> 101
gtaaaaagaa ttataatgga attagacatg caaagcattc aagacattat tgctaaaaaa 60
ttaagtggtg ggcagaagag aaaactaaca ctagggattg ccatcttagg agatcctcag 120

<210> 102
<211> 139
<212> ADN
<213> Homo sapiens

<400> 102
gttttgctgc tagatgaacc aactgctgga ttggatccct tttcaagaca ccgagtgtgg 60
agcctcctga aggagcataa agtagaccga cttatcctct tcagtaccca attcatggat 120
gaggctgaca tcttggctg 139

<210> 103
<211> 91
<212> ADN
<213> Homo sapiens

<400> 103
ataggaaagt atttctgtct aatgggaagt tgaaatgtgc aggatcatct ttgtttctga 60
agcgaaagtg gggtattgga tatcatttaa g 91

<210> 104
<211> 140
<212> ADN
<213> Homo sapiens

<400> 104
tttacacagg aatgaaatgt gtgacacaga aaaaatcaca tcccttatta agcagcacat 60
tcctgatgcc aagttaacaa cagaaagtga agaaaaactt gtatatagtt tgcctttgga 120
aaaaacgaac aaatttccag 140

<210> 105
<211> 120
<212> ADN
<213> Homo sapiens

<400> 105
atctttacag tgaccttgat aagtgttctg accagggcat aaggaattat gctgtttcag 60
tgacatctct gaatgaagta ttcttgaacc tagaaggaaa atcagcaatt gatgaaccag 120

<210> 106
<211> 199
<212> ADN
<213> Homo sapiens

<400> 106
attttgacat tgggaaacaa gagaaaatac atgtgacaag aaatactgga gatgagtctg 60
aaatggaaca ggttctttgt tctcttcctg aaacaagaaa ggctgtcagt agtgcagctc 120
tctggagacg acaaatctat gcagtggcaa cacttcgctt cttaaagtta aggcgtgaaa 180
ggagagctct tttgtgttt 199

<210> 107
<211> 167
<212> ADN
<213> Homo sapiens

<400> 107
gttactagta cttggaattg cttttatccc catcattcta gagaagataa tgtataaagt 60
aactcgtgaa actcattgtt gggagttttc acccagtatg tatttccttt ctctggaaca 120
aatcccgaag acgcctctta ccagcctgtt aatcgttaat aatacag 167

<210> 108
<211> 134
<212> ADN
<213> Homo sapiens

<400> 108
gatcaaatat tgaagacctc gtgcattcac tgaagtgtca ggatatagtt ttggaaatag 60
atgactttag aaacagaaat ggctcagatg atccctccta caatggagcc atcatagtgt 120
ctggtgacca gaag 134

<210> 109
<211> 138
<212> ADN
<213> Homo sapiens

<400> 109
gattacagat tttctgttgc gtgtaatacc aagaaattga attgttttcc tgttcttatg 60
ggaattgtta gcaatgccct tatgggaatt tttaacttca cggagcttat tcaaacggag 120
agcacttcat tttctcgt 138

<210> 110
<211> 108
<212> ADN
<213> Homo sapiens

<400> 110
gatgacatag tgctggatct tggttttata gatgggtcca tatttttgtt gttgatcaca 60
aactgcgttt ctccttttat cggcatgagc agcatcagcg attataaa 108

<210> 111
<211> 171
<212> ADN
<213> Homo sapiens

<400> 111
aaaaatgttc aatcccagtt atggatttca ggcctctggc cttcagcata ctggtgtgga 60
caggctctgg tggacattcc attatacttc ttgattctct tttcaataca tttaatttac 120
tacttcatat ttctgggatt ccagctttca tgggaactca tgtttgtttt g 171

<210> 112
<211> 114
<212> ADN
<213> Homo sapiens

<400> 112
gtggtatgca taattggttg tgcagtttct cttatattcc tcacatatgt gctttcattc 60
atctttcgca agtggagaaa aaataatggc ttttggtctt ttggcttttt tatt 114

<210> 113
<211> 120
<212> ADN
<213> Homo sapiens

<400> 113
atcttaatat gtgtatccac aattatggta tcaactcaat atgaaaaact caacttaatt 60
ttgtgcatga ttttcatacc ttccttcact ttgctggggt atgtcatgtt attgatccag 120

<210> 114
<211> 81
<212> ADN
<213> Homo sapiens

<400> 114
ctcgacttta tgagaaactt ggacagtctg gacaatagaa taaatgaagt caataaaacc 60
attcttttaa caaccttaat a 81

<210> 115
<211> 92
<212> ADN
<213> Homo sapiens

<400> 115
ccataccttc agagtgttat tttccttttt gtcataaggt gtctggaaat gaagtatgga 60
aatgaaataa tgaataaaga cccagttttc ag 92

<210> 116
<211> 121
<212> ADN
<213> Homo sapiens

<400> 116
aatctctcca cggagtagag aaactcatcc caatccggaa gagcccgaag aagaagatga 60
agatgttcaa gctgaaagag tccaagcagc aaatgcactc actgctccaa acttggagga 120
g 121

<210> 117
<211> 118
<212> ADN
<213> Homo sapiens

<400> 117
   gaaccagtca taactgcaag ctgtttacac aaggaatatt atgagacaaa gaaaagttgc 60
   ttttcaacaa gaaagaagaa aatagccatc agaaatgttt ccttttgtgt taaaaaag 118

<210> 118
<211> 92
<212> ADN
<213> Homo sapiens

<400> 118
gtgaagtttt gggattacta ggacacaatg gagctggtaa aagtacttcc attaaaatga 60
taactgggtg cacaaagcca actgcaggag tg 92

<210> 119
<211> 179
<212> ADN
<213> Homo sapiens

<400> 119
gtggtgttac aaggcagcag agcatcagta aggcaacagc atgacaacag cctcaagttc 60
ttggggtact gccctcagga gaactcactg tggcccaagc ttacaatgaa agagcacttg 120
gagttgtatg cagctgtgaa aggactgggc aaagaagatg ctgctctcag tatttcacg 179

<210> 120
<211> 76
<212> ADN
<213> Homo sapiens

<400> 120
attggtggaa gctcttaagc tccaggaaca acttaaggct cctgtgaaaa ctctatcaga 60
gggaataaag agaaag 76

<210> 121
<211> 95
<212> ADN
<213> Homo sapiens

<400> 121
ctgtgctttg tgctgagcat cctggggaac ccatcagtgg tgcttctaga tgagccgttc 60
accgggatgg accccgaggg gcagcagcaa atgtg 95

<210> 122
<211> 120
<212> ADN
<213> Homo sapiens

<400> 122
gcagatactt caggctaccg ttaaaaacaa ggagaggggc accctcttga ccacccatta 60
catgtcagag gctgaggctg tgtgtgaccg tatggccatg atggtgtcag gaacgctaag 120

<210> 123
<211> 141
<212> ADN
<213> Homo sapiens

<400> 123
gtgtattggt tccattcaac atctgaaaaa caagtttggt agagattatt tactagaaat 60
aaaaatgaaa gaacctaccc aggtggaagc tctccacaca gagattttga agcttttccc 120
acaggctgct tggcaggaaa g 141

<210> 124
<211> 80
<212> ADN
<213> Homo sapiens

<400> 124
atattcctct ttaatggcgt ataagttacc tgtggaggat gtccaccctc tatctcgggc 60
ctttttcaag ttagaggcga 80

<210> 125
<211> 56
<212> ADN
<213> Homo sapiens

<400> 125
tgaaacagac cttcaacctg gaggaataca gcctctctca ggctaccttg gagcag 56

<210> 126
<211> 769
<212> ADN
<213> Homo sapiens

<400> 126
gtattcttag aactctgtaa agagcaggag ctgggaaatg ttgatgataa aattgataca 60
acagttgaat ggaaacttct cccacaggaa gacccttaaa atgaagaacc tcctaacatt 120
caattttagg tcctactaca ttgttagttt ccataattct acaagaatgt ttccttttac 180
ttcagttaac aaaagaaaac atttaataaa cattcaataa tgattacagt tttcattttt 240
aaaaatttag gatgaaggaa acaaggaaat atagggaaaa gtagtagaca aaattaacaa 300
aatcagacat gttattcatc cccaacatgg gtctattttg tgcttaaaaa taatttaaaa 360
atcatacaat attaggttgg ttttcggtta ttatcaataa agctaacact gagaacattt 420
tacaaataaa aatatgagtt ttttagcctg aacttcaaat gtatcagcta tttttaaaca 480
ttatttactc ggattctaat ttaatgtgac attgactata agaaggtctg ataaactgat 540
gaaatggcac agcataacat ttaattataa tgacattctg attataaaat aaatgcatgt 600
gaattttagt acatattgaa gttatatgga agaagatagc cataatctgt aagaaagtac 660
cgcagttaat attttcttta gccaacttat attcaatgta ttttttatgg atcctttttc 720
aaaggtagta tcagtaggca tagtcatttt ctgtatcttt tcacctcac 769

<210> 127
<211> 19
<212> ADN
<213> Homo sapiens

<400> 127
cagtgactat gtatccgtg 19

<210> 128
<211> 19
<212> ADN
<213> Homo sapiens

<400> 128
gatggtttct cctcacaac 19

<210> 129
<211> 19
<212> ADN
<213> Homo sapiens

<400> 129
caccagacaa tgaggatga 19

<210> 130
<211> 19
<212> ADN
<213> Homo sapiens

<400> 130
gctatattct tcaatggca 19

<210> 131
<211> 19
<212> ADN
<213> Homo sapiens

<400> 131
cctagaagta gaccgcctt                                                    19

<210> 132
<211> 19
<212> ADN
<213> Homo sapiens

<400> 132
gttgtgagga gaaaccatc                                                    19

<210> 133
<211> 19
<212> ADN
<213> Homo sapiens

<400> 133
ctggatggtt tcagtcaca                                                    19

<210> 134
<211> 19
<212> ADN
<213> Homo sapiens

<400> 134
cagaaaagcc aatcgggtg                                                    19

<210> 135
<211> 23
<212> ADN
<213> Homo sapiens

<400> 135
ccaggtatat gttgtttaac cag                                               23

<210> 136
<211> 20
<212> ADN
<213> Homo sapiens

<400> 136
gggtcagatt actgccttac                                                   20

<210> 137
<211> 20
<212> ADN
<213> Homo sapiens

<400> 137
gaacattgaa gaaccaacac                                                   20

<210> 138
<211> 20
<212> ADN
<213> Homo sapiens

<400> 138
gtaaggcagt aatctgaccc                                                    20

<210> 139
<211> 18
<212> ADN
<213> Homo sapiens

<400> 139
ggaaactgga cagaatgc                                                      18

<210> 140
<211> 19
<212> ADN
<213> Homo sapiens

<400> 140
ctaccctatt tcacatgcc                                                     19

<210> 141
<211> 20
<212> ADN
<213> Homo sapiens

<400> 141
gtttctccca taataacagc                                                    20

<210> 142
<211> 20
<212> ADN
<213> Homo sapiens

<400> 142
gctgttatta tgggagaaac                                                    20

<210> 143
<211> 30
<212> ADN
<213> Homo sapiens

<400> 143
agactacagt aacaaaagcc tagtgcagcc                                         30

<210> 144
<211> 30
<212> ADN
<213> Homo sapiens

<400> 144
atccaatcct attagtgtga caaaggcttg                                         30

<210> 145
<211> 20
<212> ADN
<213> Homo sapiens

<400> 145
tcagcaaacc aaagcacttc                                                    20

<210> 146
<211> 27
<212> ADN

<213> Homo sapiens

<400> 146
caagtgctgt tttattcatt atctgct                                          27


<210> 147
<211> 28
<212> ADN
<213> Homo sapiens

<400> 147
gtacatgaaa actcaccata tccatccc                                         28


<210> 148
<211> 20
<212> ADN
<213> Homo sapiens

<400> 148
tcattgctgg gatggatatg                                                  20


<210> 149
<211> 19
<212> ADN
<213> Homo sapiens

<400> 149
ccctgtgatg gaggagttg                                                   19


<210> 150
<211> 21
<212> ADN
<213> Homo sapiens

<400> 150
tgacatcaac tcctccatca c                                                21


<210> 151
<211> 20
<212> ADN
<213> Homo sapiens

<400> 151
gaatgctgaa tcttggagac                                                  20


<210> 152
<211> 20
<212> ADN
<213> Homo sapiens

<400> 152
gattcagatt atcaaactgg                                                  20


<210> 153
<211> 21
<212> ADN
<213> Homo sapiens

<400> 153
tggtgtaatt tttcctgacc c                                                21


<210> 154

<211> 22
<212> ADN
<213> Homo sapiens

<400> 154
aagggtcagg aaaaattaca cc                                                22

<210> 155
<211> 19
<212> ADN
<213> Homo sapiens

<400> 155
ggaattcagg agctactgg                                                    19

<210> 156
<211> 22
<212> ADN
<213> Homo sapiens

<400> 156
gattgtctgt tccaacagaa gg                                                22

<210> 157
<211> 22
<212> ADN
<213> Homo sapiens

<400> 157
ccacttcctt tagatgaatc cc                                                22

<210> 158
<211> 22
<212> ADN
<213> Homo sapiens

<400> 158
aagtggaaca agaggtacaa cg                                                22

<210> 159
<211> 21
<212> ADN
<213> Homo sapiens

<400> 159
atggtaatcc caaaagtcag c                                                 21

<210> 160
<211> 22
<212> ADN
<213> Homo sapiens

<400> 160
ggggatgtga tgagtaatga ag                                                22

<210> 161
<211> 22
<212> ADN
<213> Homo sapiens

<400> 161
cttcattact catcacatcc cc                                                22

<210> 162
<211> 19
<212> ADN
<213> Homo sapiens

<400> 162
acaacttccc caggaaccc                                                    19

<210> 163
<211> 19
<212> ADN
<213> Homo sapiens

<400> 163
gatcaacagg ctggtacgg                                                    19

<210> 164
<211> 22
<212> ADN
<213> Homo sapiens

<400> 164
caagaaaaat gctaagtccc ag                                                22

<210> 165
<211> 19
<212> ADN
<213> Homo sapiens

<400> 165
tgcccacacc agtaagcag                                                    19

<210> 166
<211> 22
<212> ADN
<213> Homo sapiens

<400> 166
gaaaatcagt ggcactcaat tc                                                22

<210> 167
<211> 22
<212> ADN
<213> Homo sapiens

<400> 167
tgccactgat tttctagtct gc                                                22

<210> 168
<211> 19
<212> ADN
<213> Homo sapiens

<400> 168
ctgggatcac aaagccaac                                                    19

<210> 169
<211> 20
<212> ADN
<213> Homo sapiens

<400> 169

cctttcagtt ccacctctcc 20

<210> 170
<211> 21
<212> ADN
<213> Homo sapiens

<400> 170
tccacactga gattctgaag c 21

<210> 171
<211> 19
<212> ADN
<213> Homo sapiens

<400> 171
aatacctttc ctgccctgc 19

<210> 172
<211> 19
<212> ADN
<213> Homo sapiens

<400> 172
gcctgactct ttgggtgac 19

<210> 173
<211> 19
<212> ADN
<213> Homo sapiens

<400> 173
tgagcgtggg tcagcaaac 19

<210> 174
<211> 20
<212> ADN
<213> Homo sapiens

<400> 174
gcaactcctc cttgggcaac 20

<210> 175
<211> 19
<212> ADN
<213> Homo sapiens

<400> 175
tttgttgccc aaggaggag 19

<210> 176
<211> 22
<212> ADN
<213> Homo sapiens

<400> 176
ggaaaaacaa gggagaacat cg 22

<210> 177
<211> 19
<212> ADN
<213> Homo sapiens

<400> 177
gcccacttgg attcttcac                                                    19

<210> 178
<211> 22
<212> ADN
<213> Homo sapiens

<400> 178
ccacaccttt caaagcttct ac                                                22

<210> 179
<211> 22
<212> ADN
<213> Homo sapiens

<400> 179
atgtggtcct tgagaatgaa ac                                                22

<210> 180
<211> 22
<212> ADN
<213> Homo sapiens

<400> 180
actgtgaaag aaaacctcag gc                                                22

<210> 181
<211> 19
<212> ADN
<213> Homo sapiens

<400> 181
cttcatgtgg caaaatccc                                                    19

<210> 182
<211> 20
<212> ADN
<213> Homo sapiens

<400> 182
tgtgctgtca atttggcatc                                                   20

<210> 183
<211> 20
<212> ADN
<213> Homo sapiens

<400> 183
aagaagaaat ggggcatagg                                                   20

<210> 184
<211> 22
<212> ADN
<213> Homo sapiens

<400> 184
tgtatttgga gacagttccc ac                                                22

<210> 185
<211> 19

<212> ADN
<213> Homo sapiens

<400> 185
aacaatcagt ggcgtggcg                                                    19


<210> 186
<211> 22
<212> ADN
<213> Homo sapiens

<400> 186
gacatccagg aggacaggaa ag                                                22


<210> 187
<211> 21
<212> ADN
<213> Homo sapiens

<400> 187
gcagcctctt tcactccata c                                                 21


<210> 188
<211> 22
<212> ADN
<213> Homo sapiens

<400> 188
cattgtgtca ggtgatgaaa ag                                                22


<210> 189
<211> 20
<212> ADN
<213> Homo sapiens

<400> 189
ttcatttcta ggcatcgcag                                                   20


<210> 190
<211> 22
<212> ADN
<213> Homo sapiens

<400> 190
cattagcagg aggatcaaaa ag                                                22


<210> 191
<211> 21
<212> ADN
<213> Homo sapiens

<400> 191
   tctagggcta ttttttggca c                                                  21


<210> 192
<211> 19
<212> ADN
<213> Homo sapiens

<400> 192
cgctcccttt caaaatcac                                                    19

<210> 193
<211> 21
<212> ADN
<213> Homo sapiens

<400> 193
tgcgagactt tgatgagaca c                                                21

<210> 194
<211> 20
<212> ADN
<213> Homo sapiens

<400> 194
agaccatcag ggaggagaac                                                  20

<210> 195
<211> 18
<212> ADN
<213> Homo sapiens

<400> 195
tgtgccagca accaaatc                                                    18

<210> 196
<211> 22
<212> ADN
<213> Homo sapiens

<400> 196
gctggagatg aagctgaaga ac                                               22

<210> 197
<211> 18
<212> ADN
<213> Homo sapiens

<400> 197
tttccacttc accgaggg                                                    18

<210> 198
<211> 21
<212> ADN
<213> Homo sapiens

<400> 198
ccatgttttg tctgttgtgc c                                                21

<210> 199
<211> 22
<212> ADN
<213> Homo sapiens

<400> 199
cacccatcaa cccatcatct ac                                               22

<210> 200
<211> 22
<212> ADN
<213> Homo sapiens

<400> 200
aggcacaaca gacaaaacat gg                                               22

<210> 201
<211> 22
<212> ADN
<213> Homo sapiens

<400> 201
aagcatgatg tagtagtgac cc                                                22

<210> 202
<211> 25
<212> ADN
<213> Homo sapiens

<400> 202
cttgggtagt tttggattca ggtgc                                             25

<210> 203
<211> 27
<212> ADN
<213> Homo sapiens

<400> 203
agatccattg aagacatttg aggagtg                                           27

<210> 204
<211> 20
<212> ADN
<213> Homo sapiens

<400> 204
gattgacata catttgcttc                                                   20

<210> 205
<211> 20
<212> ADN
<213> Homo sapiens

<400> 205
tacagtgaag agaaatccag                                                   20

<210> 206
<211> 18
<212> ADN
<213> Homo sapiens

<400> 206
tggaattaga catgcaaa                                                     18

<210> 207
<211> 19
<212> ADN
<213> Homo sapiens

<400> 207
tgaagaggat aagtcggtc                                                    19

<210> 208
<211> 18
<212> ADN
<213> Homo sapiens

<400> 208
tataatcgct gatgctgc                                                    18

<210> 209
<211> 26
<212> ADN
<213> Homo sapiens

<400> 209
accaggccag agtcattaaa ctgatc                                           26

<210> 210
<211> 25
<212> ADN
<213> Homo sapiens

<400> 210
ccgaaaagat gcacaaatat agccc                                            25

<210> 211
<211> 26
<212> ADN
<213> Homo sapiens

<400> 211
ctcaaaactt cattctaatt gtgccc                                           26

<210> 212
<211> 18
<212> ADN
<213> Homo sapiens

<400> 212
agataagcgt gcgtcaac                                                    18

<210> 213
<211> 20
<212> ADN
<213> Homo sapiens

<400> 213
tcttatggga attgttagca                                                  20

<210> 214
<211> 19
<212> ADN
<213> Homo sapiens

<400> 214
ttatgactgg ttcctcctc                                                   19

<210> 215
<211> 18
<212> ADN
<213> Homo sapiens

<400> 215
tcatcaacat ttcccagc                                                    18

<210> 216
<211> 21
<212> ADN

<213> Homo sapiens

<400> 216
gaaatactgg agatgagtct g                                                21


<210> 217
<211> 19
<212> ADN
<213> Homo sapiens

<400> 217
gagcttaaga gcttccacc                                                   19